# EUROPEAN PATENT SPECIFICATION

(11) **EP 3 955 833 B1**
(45) Date of publication and mention of the grant of the patent: **27.03.2024**
(21) Application number: 20725266.9
(22) Date of filing: 15.04.2020
(51) Int. Cl.: A61B 17/221, A61F 2/01

(54) **CLOT RETRIEVAL SYSTEM**
GERINNSELBESEITIGUNGSSYSTEM
SYSTÈME DE RÉCUPÉRATION DE CAILLOT

(30) Priority: 16.04.2019 US 201916385862
(43) Date of publication of application: 23.02.2022
(73) Proprietor: Legacy Ventures LLC, Nashville, Tennessee 37203 (US)
(72) Inventor: ULM, III, Arthur John, Nashville, Tennessee 37215 (US)
(74) Representative: Dehns
(86) International application number: PCT/US2020/028246
(87) International publication number: WO 2020/214656

(56) References cited:
- WO-A1-2017/201299
- US-A1- 2018 271 547

## Description

### BACKGROUND

### TECHNICAL FIELD

The present invention relates to a deployable system for removing a blood clot or other object from a lumen of an animal.

### BACKGROUND OF THE INVENTION

Acute ischemic strokes develop when a blood clot (thrombus) blocks an artery supplying blood to the brain. Needless to say, when a blood clot creates such a blockage, time in removing the clot is critical.

The removal of intracranial obstructions is limited by several factors, such as the distance of the intracranial obstruction from the femoral access site, the tortuosity (twists and turns in the artery as it enters the base of the skull) of the cervical and proximal intracranial vasculature, the small size of the vessels and the extremely thin walls of intracranial vessels, which lack a significant muscular layer. These limitations require a device to be small and flexible enough to navigate through tortuous vessels within a guide catheter and microcatheter, expand after delivery at the site of occlusion and be retrievable into the microcatheter and yet be strong enough to dislodge strongly adherent thrombus from the vessel wall. In addition, the device should distally entrap or encase the thrombus to prevent embolization to other vessels and to completely remove the occlusion. The device should be retrievable without the need for proximal occlusion of the vessel, which carries risk of further ischemia and risk of vessel injury. The device should be simple to use and be capable of multi-use within the same patient treatment. The device should not be abrasive and should not have sharp corners exposed to the endothelial layer of the vessel wall.

Currently available intravascular thrombus and foreign body removal devices lack several of these features. Currently available devices include the MERCI^{™} RETRIEVER clot retriever device marketed by Concentric Medical, Inc. (Mountainview, CA), the PENUMBRA^{™} system marketed by Penumbra Inc. (Alameda, CA) to retrieve clots, and the newer stent retrieval devices TREVO^{™} (Stryker, Kalamazoo, MI) and SOLITAIRE^{™} (eV3 Endovascular Inc., Plymouth, MA, which is a subsidiary of Covidien). All the devices are ineffectual at removing organized hard thrombus that embolize to the brain from the heart and from atherosclerotic proximal vessels. These "hard" thrombi constitute the majority of strokes which are refractory to medical treatment and are therefore referred for removal by mechanical means through an endovascular approach. The MERCI retrieval system is comprised of coiled spring-like metal and associated suture material. The method of use is deployment distal to the thrombus and by withdrawing the device through the thrombus, the thrombus becomes entangled in the coil and mesh and then is retrieved. The MERCI system requires occlusion of the proximal vessel with a balloon catheter and simultaneous aspiration of blood while the thrombus is being removed. Most of the time, the device fails to dislodge the thrombus from the wall of the vessel and often, even when successfully dislodging the thrombus, the thrombus embolizes into another or the same vessel due to the open ended nature of the device.

The next attempt at a thrombus removal system was the PENUMBRA. The PENUMBRA is a suction catheter with a separator that macerates the thrombus which is then removed by suction. The device is ineffective at removing hard, organized thrombus which has embolized from the heart, cholesterol plaque from proximal feeding arteries and other foreign bodies.

The SOLITAIRE and TREVO systems are self-expanding non-detachable stents. The devices are delivered across the thrombus which is then supposed to become entwined in the mesh of the stent and which is then removed in a manner similar to the MERCI system. Again, these devices are ineffectual at treating hard thrombus. In fact, the thrombus is often compressed against the vessel wall by the stent which temporarily opens the vessel by outwardly pressing the clot against the vessel wall. Upon retrieval of the devices, the clot remains or is broken up into several pieces which embolize to vessels further along the vessel. US 2018/0271547 A1 and WO 2017/201299 A1 disclose basket thrombectomy devices, where the baskets comprise a number of cells.

Thus, there is a need for new, easy-to-use, easy-to-manufacture, safe surgical devices for removing obstructions, such as blood clots, from internal lumens of humans and other animals in a timely manner.

### BRIEF SUMMARY

The invention provides a system according to claim 1. Further embodiments of the invention are provided in the dependent claims. Methods for treatment of the human or animal body by surgery or therapy and diagnostic methods practised on the human or animal body do not form part of the present invention. (1 inch = 25.4 mm) The present disclosure provides several systems for removing obstructions and other objects within a blood vessel or other lumen of an animal. The system may be deployed in the lumen from a distal end of a catheter and, in some embodiments, includes a pull wire having a proximal end and a distal end; a distal body attached to the pull wire, the distal body comprising an interior, an exterior, a proximal end, a distal end, a plurality of proximal memory metal strips located at the proximal end, a proximal hub/junction located in the distal body interior, and a distal hub/junction located distal relative to the proximal hub/junction. The distal body has a relaxed state wherein the distal body has a first height and width and a collapsed state wherein the distal body has a second height and width, the second height less than said first height, the second width less than the first width. The system further includes a catheter having an interior, a proximal end leading to the interior and a distal end leading to the interior, the catheter comprised of a biocompatible material and configured to envelope the distal body when the distal body is in the collapsed state. Each of the proximal memory metal strips has a proximal end and a distal end and preferably, in the relaxed state, each of the proximal ends of the proximal memory metal strips is located proximal relative to the proximal hub/junction. Preferably, in the relaxed state, the proximal ends of the proximal memory metal strips are configured to move towards each other and towards the pull wire when an operator moves the proximal hub/junction distally and closer to the stationary distal hub/junction (i.e., when the operator decreases the distance between the hubs/junctions). Preferably, in the relaxed state, the proximal ends of the proximal memory metal strips are configured to move away from each other and away from the pull wire by moving the proximal hub/junction proximally away from the stationary distal hub/junction (i.e., when the operator increases the distance between the hubs/junctions).

Optionally, the system further includes a plurality of memory metal connector strips, the plurality of memory metal connector strips each having a proximal end attached to a proximal memory metal strip and a distal end attached to the proximal hub/junction. Optionally, the connector strips are integral with the proximal hub/junction (i.e., optionally, the connector strips and the proximal hub/junction are formed from the same piece of memory metal). Optionally, the proximal hub/junction is a tube having an aperture and the pull wire passes through the aperture. Optionally, in the relaxed state, the proximal hub/junction is slideable along the pull wire (i.e., at least a segment of the pull wire). Optionally, in the relaxed state, the proximal memory metal strips are distributed substantially evenly about a perimeter of the distal body. Optionally, the distal hub/junction is a tube having an aperture. Optionally, the distal hub/junction is attached to the pull wire such that the distal hub/junction is not slideable along the pull wire. Optionally, the distal body further comprises a lead wire extending distally from the distal hub/junction. Optionally, the distal body comprises a basket comprised of a plurality of memory metal strips distal relative to the proximal memory metal strips. Optionally, the distal hub/junction, the proximal hub/junction, and the distal basket are comprised of a nitinol having the same material composition. Optionally, the distal body further comprises an x-ray marker. Optionally, the proximal memory metal strips form a claw, the claw having a closeable proximal end formed by the proximal ends of the proximal memory metal strips. Optionally, between 2 and 4 proximal memory metal strips form the claw. Optionally, the distal body, in the relaxed state, has a tapered shape in which the distal body height and width decrease from the proximal end to the distal end. Optionally, the distal body, in the relaxed state, has a bullet shape. Optionally, the proximal hub/junction and the distal hub/junction are generally cylindrical in shape and each has an outer diameter and an inner diameter that forms the apertures of the proximal and distal hub/junctions, the outer diameters of the proximal and distal hub/junctions are substantially the same size, and the inner diameters of the proximal and distal hubs/junctions are substantially the same size. Optionally, the outer diameters of the proximal and distal hubs/junctions are from about 0.011 inches to about 0.054 inches, and the inner diameters of the proximal and distal hubs/junctions are from about 0.008 inches to about 0.051 inches. Optionally, the pull wire is generally cylindrical and the diameter of the pull wire is between about 0.008 inches and about 0.051 inches. Optionally, the proximal memory metal strips have a length of between about 10 and about 60 millimeters. Optionally, the first height and first width of the distal body are between about 2 millimeters (mm) and about 6 millimeters. Optionally, the proximal memory metal strips are configured to a separate a clot from a blood vessel wall.

The present disclosure also provides a method of removing an object from an interior lumen of an animal, the lumen having an interior wall forming the lumen. In some embodiments, the method includes:
a) providing a system comprising: i) a pull wire having a proximal end and a distal end; ii) a distal body attached to the pull wire, the distal body comprising a proximal end, a distal end, and a claw, the claw comprised of a plurality of memory metal strips, the distal body having a relaxed state wherein the distal body has a first height and width and a collapsed state wherein the distal body has a second height and width, the second height less than said first height, the second width less than said first width; and iii) a catheter having an interior, a proximal end leading to the interior and a distal end leading to the interior, the catheter comprised of a biocompatible material and configured to envelope the distal body when said distal body is in said collapsed state;
b) positioning the system in the lumen;
c) deploying the distal body from the distal end of the catheter;
d) allowing the height and width of said distal body to increase; and
e) moving the memory metal strips towards each other and the pull wire so as to capture the obstruction. Optionally, the claw and the memory metal strips are located at the proximal end of said distal body and the distal body is deployed distal to said object. Optionally, the proximal memory metal strips have a proximal end forming the proximal end of the claw and a distal end, and the method includes moving the proximal ends of the memory metal strips towards each other and the pull wire so as to capture the obstruction. Optionally, the distal body further comprises a proximal hub/junction located in the distal body interior, and a distal hub/junction located distal relative to the proximal hub/junction, each of the memory metal strips has a proximal end and a distal end, each of the proximal ends of the memory metal strips is located proximal relative to the proximal hub/junction, and the proximal ends of the memory metal strips are configured to move towards each other and towards the pull wire by moving the proximal hub/junction distally and closer to the distal hub/junction, and the proximal ends of the memory metal strips are configured to move away from each other and away from the pull wire by moving the proximal hub/junction proximally and away from the distal hub/junction, and the method further comprises moving the proximal hub/junction distally and closer to the distal hub/junction so as to capture the obstruction in the claw. Optionally, the interior lumen is an intracranial artery and the obstruction is a blood clot. Optionally, the method further comprises using the clot to move the proximal hub/junction toward the distal hub/junction and exert tension on the proximal memory metal strips. Optionally, the method further comprises using a tube to move the proximal hub/junction toward the distal hub/junction and exert tension on the proximal memory metal strips.

The present disclosure also provides a method of manufacturing a system for removing objects within an interior lumen of an animal. In some embodiments, the method includes:
a) providing a single tube comprised of a memory metal, the single tube having an exterior, a hollow interior, a wall separating the exterior from the hollow interior, a proximal portion comprising an aperture leading to the hollow interior, a distal portion comprising an aperture leading to the hollow interior, and a middle portion between the proximal portion and the distal portion;
b) cutting the wall of the middle portion with a laser;
c) removing the pieces of the middle portion cut by the laser to form a proximal tube, a middle portion comprising a plurality of memory metal strips attached to the proximal tube and a distal tube;
d) altering the shape of the middle portion;
e) allowing the middle portion to expand relative to the distal tube and the proximal tube;
f) cutting the memory metal strips to form a first segment comprising the proximal tube and a proximal segment of the memory metal strips, and a second segment comprising the distal tube and a distal segment of the memory metal strips; and
g) joining the proximal segments to the distal segments such that the distal segments form the proximal end of a distal body, such that the proximal tube is located inside an interior of said distal body, and such that the proximal tube is located distal relative to the proximal end.

Optionally, the method further includes placing a pull wire through the proximal tube such that the proximal tube is slideable along at least a segment of the pull wire. Optionally, the method further includes attaching the pull wire to the distal tube. Optionally, the step of joining the proximal segments to the distal segments comprises welding or soldering the proximal segments to the distal segments. Optionally, after the step of joining the proximal segments to the distal segments, the proximal end forms a claw comprised of between 2 and 4 memory metal strips, the claw memory metal strips configured to move towards each by moving said proximal tube distally and closer to the distal tube, and the claw memory metal strips configured to move away from each other by moving the proximal tube proximally and away from said distal tube. Optionally, the method further includes not altering the shape of the proximal and distal portions while altering the shape of the middle portion. Optionally, the method further includes cooling the proximal portion, the middle portion, and the distal portion after step D) and, after cooling, the proximal and distal portions have substantially the same size as the proximal and distal portions had prior to step A). Optionally, the method of allowing said middle portion to expand comprises heating the middle portion. Optionally, the method of altering the shape of the middle portion comprises using a mandrel. Optionally, the mandrel is tapered. Optionally, the proximal portion and the distal portion are not cut by the laser. Optionally, prior to cutting the memory metal tube, the memory metal tube has an outer diameter that is from about 0.011 inches to about 0.054 inches and an inner diameter that is from about 0.008 inches to about 0.051 inches.

In an alternate embodiment, the present disclosure provides a system for removing objects from an interior lumen of an animal that includes:
a pull wire having a proximal end and a distal end;
a distal body attached to the pull wire, the distal body comprising an interior, a proximal end, a distal end, a distal body length extending from the proximal end to the distal end, a proximal hub/junction (preferably in the form of a tube) forming the proximal end of the distal body, a basket comprised of a plurality of cells formed by a plurality of basket strips, a plurality of proximal strips, and, optionally a distal hub/junction (preferably in the form of a tube) forming a distal end of the basket, the basket comprising a basket interior, each proximal strip having a proximal end attached to the proximal hub/junction, and a distal end attached to a cell, the distal body having a relaxed state wherein the distal body has a first height and a first width, and a collapsed state wherein the distal body has a second height and a second width, the second height less than the first height, the second width less than the first width; and
a catheter having an interior, a proximal end leading to the interior and a distal end leading to the interior, the catheter comprised of a biocompatible material and configured to envelope the distal body when the distal body is in the collapsed state,
wherein, in the relaxed state, the basket comprises a first pair of distal crowns not attached to another cell of the basket and pointing generally in the distal direction, the first pair of distal crowns located approximately the same distance from the proximal hub/junction and approximately 180 degrees relative to each other (e.g., between about 150 degrees and about 180 degrees relative to each other), and further wherein the basket further comprises a second pair of distal crowns not attached to another cell of the basket and pointing generally in the distal direction, the second pair of distal crowns located distally relative to, and approximately 90 degrees relative to, the first pair of distal crowns (e.g., each distal crown of the second pair of distal crowns is located approximately 60 degrees to 90 degrees relative to a distal crown of the first pair of distal crowns), the distal crowns in the second pair of distal crowns located approximately the same distance from the proximal hub/junction and further wherein each of the distal crowns in the first and second pair of distal crowns comprises an x-ray marker, the x-ray maker more visible under x-ray as compared to the basket strips when the distal body is located in a cranial blood vessel inside the body of a human and the x-ray is taken from outside the human's body. Optionally, instead of a distal hub/junction, the basket includes an open distal end.

Optionally, the x-ray markers are comprised of a material different than the material forming the basket strips. Optionally, in the relaxed state, the basket interior is substantially hollow. Optionally, in the relaxed state, the distal body does not have another x-ray marker that is located approximately the same distance from the proximal hub/junction as the first pair of x-ray markers and the distal body does not have another x-ray marker that is located approximately the same distance from the proximal hub/junction as the second pair of x-ray markers. In other words, the first and second pair of x-ray markers are the only markers their respective distances from the proximal hub/junction. Optionally, each distal crown in the first and second pair of distal crowns forms part of an enlarged cell and further wherein the surface area of each enlarged cell in the relaxed state is greater than the surface area of each of the other individual cells of the basket and further wherein the enlarged cells are configured to allow a thrombus to pass therethrough and into the basket interior. Optionally, in the relaxed state, the distal body does not have another free distal-pointing crown that is located approximately the same distance from the proximal hub/junction as the first pair of distal crowns and the distal body does not have another free distal-pointing crown that is located approximately the same distance from the proximal hub/junction as the second pair of distal crowns. Optionally, the basket strips are comprised of a memory metal. Optionally, each of the distal crowns in the first pair and second pair of distal crowns curve radially inward toward the basket interior in the relaxed state, wherein the distal crowns of the first pair of distal crowns are configured to contact each other when an exterior, external compressive force (such as a thrombus) is exerted on a distal crown of the first pair of distal crowns when the distal body is in the relaxed state, and further wherein the distal crowns of the second pair of distal crowns are configured to contact each other when an exterior, external compressive force (such as a thrombus) is exerted on a distal crown of the second pair of distal crowns when the distal body is in the relaxed state. Optionally, the proximal hub/junction is located approximately in the center of the first height and first width in the relaxed state. For example, preferably the proximal hub/junction is located within 0.5 mm of the center of first width and the first height. Optionally, the catheter is comprised of a polymeric material (i.e., one or more polymeric materials such as silicone, PVC, latex rubber or braided nylon). Optionally, the pull wire is comprised of a biocompatible metallic material (e.g., a biocompatible metal or a biocompatible metal alloy). Optionally, the proximal end of a first proximal strip is located at least about 65 degrees (e.g., between about 65 and about 180 degrees) relative to the distal end of the first proximal strip, wherein the proximal end of a second proximal strip is located at least about 65 degrees (e.g., between about 65 and about 180 degrees) relative to the distal end of the second proximal strip, and further wherein the first and second proximal strips intersect adjacent and distal to the proximal hub/junction (e.g., within about 0 and about 4 mm of the proximal hub/junction). Optionally, each distal crown forms part of a cell that further comprises a proximal crown pointing generally in the proximal direction and connected to a memory metal strip (e.g., a proximal strip comprised of a memory metal or a basket strip comprised of a memory metal). In other words, the proximal crowns are not free. Optionally, the basket, the proximal hub/junction and the proximal strips are comprised of a memory metal, wherein the proximal hub/junction comprises a proximal end and a distal end, and further wherein the proximal strips are integral with the distal end of the proximal hub/junction. Optionally, the length of the distal body from the proximal hub/junction to the distal hub/junction (not including any lead wire) is from about 20 mm to about 65 mm. Optionally, the system is used in a method of removing a blood clot from a blood vessel of an animal the method comprising the steps of:
a) providing the system;
b) positioning the system in the lumen;
c) deploying the distal body from the distal end of the catheter;
d) allowing the height and width of the distal body to increase;
e) irradiating the distal body with x-rays;
f) moving the clot into the distal basket interior; and
g) moving the distal body proximally out of the blood vessel.

Optionally, the method further comprises irradiating the distal body with x-rays at at least two different angles. Optionally, at least one x-ray marker attached to the distal crowns is distal to the clot when the distal body is deployed from the distal end of the catheter. Optionally, the method further comprises applying contrast dye proximally and distally to the clot. Optionally, the method further comprises providing a suction catheter having a proximal end and a distal end, and attaching the distal end of the suction catheter to the clot by applying suction to the suction catheter. Optionally, the method further comprises aspirating by hand a pre-determined volume of fluid from the suction catheter using a syringe and then locking the syringe at the pre-determined volume. Optionally, the method further comprises delivering the suction catheter adjacent to the clot by advancing the catheter over the pull wire.

In yet another embodiment, the system includes:
a pull wire having a proximal end and a distal end;
a distal body attached to the pull wire, the distal body comprising an interior, a proximal end, a distal end, a distal body length extending from the proximal end to the distal end, a proximal hub/junction (preferably in the form of a tube) forming the proximal end of the distal body, a basket comprised of a plurality of cells formed by a plurality of basket strips, a plurality of proximal strips, and optionally a distal hub/junction (preferably in the form of a tube) forming a distal end of the basket, the basket comprising a basket interior, each proximal strip having a proximal end attached to the proximal hub/junction, and a distal end attached to a cell, the distal body having a relaxed state wherein the distal body has a first height and a first width, and a collapsed state wherein the distal body has a second height and a second width, the second height less than the first height, the second width less than the first width; and
a catheter having an interior, a proximal end leading to the interior and a distal end leading to the interior, the catheter comprised of a biocompatible material and configured to envelope the distal body when the distal body is in the collapsed state,
wherein, in the relaxed state, the basket comprises a first pair of distal crowns not attached to another cell of the basket and pointing generally in the distal direction, the first pair of distal crowns located approximately the same distance from the proximal hub/junction and approximately 180 degrees relative to each other (e.g., between about 150 degrees and about 180 degrees relative to each other), and further wherein the basket further comprises a second pair of distal crowns not attached to another cell of the basket and pointing generally in the distal direction, the second pair of distal crowns located distally relative to, and approximately 90 degrees relative to, the first pair of distal crowns (e.g., each distal crown of the second pair of distal crowns is located approximately 60 degrees to 90 degrees relative to a distal crown of the first pair of distal crowns), the distal crowns in the second pair of distal crowns located approximately the same distance from the proximal hub/junction, wherein each distal crown of the first and second pair of distal crowns form a cell, each cell further comprising a proximal crown pointing generally in the proximal direction and connected to a memory metal strip, wherein each of the distal crowns in the first pair and second pair of distal crowns curve radially inward toward the basket interior in the relaxed state, wherein the distal crowns of the first pair of distal crowns are configured to contact each other when an exterior, external compressive force (e.g., a thrombus) is exerted on a distal crown of the first pair of distal crowns when the distal body is in the relaxed state, and further wherein the distal crowns of the second pair of distal crowns are configured to contact each other when an exterior, external compressive force (e.g., a thrombus) is exerted on a distal crown of the second pair of distal crowns when the distal body is in the relaxed state. When it is said that a proximal crown pointing generally in the proximal direction and is connected to a memory metal strip, it is meant that the proximal crown is either connected to a basket strip or a proximal strip comprised of a memory metal (e.g., nitinol). Optionally, instead of a distal hub/junction, the basket includes an open distal end.

Optionally, the proximal hub/junction is located approximately in the center of the first height and first width in the relaxed state. For example, preferably the proximal hub/junction is located within 0.5 mm of the center of first width and the first height. Optionally, the catheter is comprised of a polymeric material (i.e., one or more polymeric materials such as silicone, PVC, latex rubber or braided nylon). Optionally, the pull wire is comprised of a biocompatible metallic material (e.g., a biocompatible metal or a biocompatible metal alloy). Optionally, in the relaxed state, the basket interior is substantially hollow. Optionally, the proximal end of a first proximal strip is located at least about 65 degrees (e.g., between about 65 and about 180 degrees) relative to the distal end of the first proximal strip, wherein the proximal end of a second proximal strip is located at least about 65 degrees (e.g., between about 65 and about 180 degrees) relative to the distal end of the second proximal strip, and further wherein the first and second proximal strips intersect adjacent and distal to the proximal hub/junction (e.g., within about 0 mm and about 4 mm of the proximal hub/junction). Optionally, each distal crown in the first and second pair of distal crowns forms part of an enlarged cell and further wherein the surface area of each enlarged cell in the relaxed state is at least twice as large as the surface area of each other individual cell of the basket and further wherein the enlarged cells are configured to allow a thrombus to pass therethrough and into the basket interior. Optionally, the pull wire is attached to the proximal hub/junction. Optionally, the basket, the proximal hub/junction and the proximal strips are comprised of a memory metal, wherein the proximal hub/junction comprises a proximal end and a distal end, and further wherein the proximal strips are integral with the distal end of the proximal hub/junction. Optionally, the distal body further comprises a lead wire extending distally from the distal hub/junction, the lead wire having a length of from about 3 mm to about 10 mm. Optionally, the distal hub/junction, the proximal hub/junction, and the basket are comprised of a nitinol having the same material composition and further wherein the proximal and the distal hubs/junctions are tubular and generally cylindrical in shape and each has an outer diameter and an inner diameter, the inner diameter forming apertures of the proximal and distal hubs/junctions and further wherein the outer diameters of the proximal and distal hubs/junctions are substantially the same size and further wherein the inner diameters of the proximal and distal hubs/junctions are substantially the same size. Optionally, the length of the distal body from the proximal hub/junction to the distal hub/junction (not including any lead wire) is from about 20 mm to about 65 mm.

Optionally, the system is used in a method of removing a blood clot from a blood vessel of an animal the method comprising the steps of:
a) providing the system;
b) positioning the system in the lumen;
c) deploying the distal body from the distal end of the catheter;
d) allowing the height and width of the distal body to increase;
e) irradiating the distal body with x-rays;
f) moving the clot into the distal basket interior; and
g) moving the distal body proximally out of the blood vessel.

Optionally, the method further comprises irradiating the distal body with x-rays at at least two different angles.

In still further embodiments, the present disclosure provides yet another embodiment of a system for removing objects from an interior lumen of an animal. The system may include a pull wire having a proximal end and a distal end. The system may also include a distal body attached to the pull wire (e.g., the distal end of the pull wire). The distal body may include an interior, a perimeter, a proximal end, a distal end, a distal body length extending from the proximal end to the distal end, a proximal junction forming the proximal end of the distal body, a plurality of proximal strips, a basket comprised of a plurality of cells formed by a plurality of basket strips, and a distal junction forming a distal end of the basket. The basket may include a basket interior. Each proximal strip may have a distal end attached to a cell and a proximal end, and the proximal ends of the proximal strips may converge at the proximal junction. The distal body may have a relaxed state wherein the distal body has a first height and a first width, and a collapsed state wherein the distal body has a second height and a second width, the second height less than the first height, the second width less than the first width. Optionally, in the relaxed state, the basket comprises a series of at least three pair of cells located on the distal body perimeter having a proximal crown pointing generally in the proximal direction and attached to a memory metal strip and a free distal crown pointing generally in the distal direction. Optionally, in the series, the proximal-most free distal crowns are located at the 12 and 6 o'clock positions and located about the same distance (i.e., the same distance +/- 5 millimeters (mm)) from the proximal junction, the next proximal-most free distal crowns are located at the 3 and 9 o'clock positions and located about the same distance (i.e., the same distance +/- 5 mm) from the proximal junction, and the succeeding proximal-most free distal crowns are located at the 12 and 6 o'clock positions and located about the same distance (i.e., the same distance +/- 5 mm) from the proximal junction. Each free distal crown may form part of a different enlarged cell that is configured to allow a thrombus to enter the basket interior. Optionally, in the relaxed state, the basket comprises a plurality of distal cells distal to the distal-most free distal crowns of the series. The plurality of distal cells may have a proximal crown attached to another cell of the basket and pointing generally in the proximal direction and a distal crown pointing generally in the distal direction and attached to the distal junction. Optionally, each enlarged cell has a proximal end, a distal end comprising a distal crown pointing generally in the distal direction, and a length extending from the proximal end to the distal end of the respective enlarged cell. Optionally, in the relaxed state, each distal cell has a length extending from the proximal crown to the distal crown of the respective distal cell. Optionally, in the relaxed state, each of the enlarged cells is longer than each of the distal cells. Optionally, for some, most or each of the enlarged cells, the distance from the proximal end of the enlarged cell to the free distal crown of the enlarged cell is less than the distance from the free distal crown of the enlarged cell to the distal end of the enlarged cell. Optionally, in the relaxed state, the basket does not have any free crowns that point generally in the proximal direction. Optionally, the distal body, in the relaxed state, comprises a distal tapered region in which the distal body height and width decrease as the basket approaches the distal junction. Optionally, each of the enlarged cells is longer than each of the pair of cells in the series. Optionally, in the relaxed state, each of the enlarged cells extends from the 6 o'clock position to the 12 o'clock position or the 3 o'clock position to the 9 o'clock position. Optionally, in the relaxed state, the basket further comprises a plurality of proximal cells proximal to the proximal-most free distal crowns of the series. Optionally, the plurality of proximal cells have a proximal crown attached to the proximal junction and pointing generally in the proximal direction and a distal crown pointing generally in the distal direction and attached to another cell of the basket. Optionally, the proximal crowns of the cells comprising the proximal-most free distal crowns are attached to the distal ends of the proximal strips. Optionally, in the relaxed state, for each of the enlarged cells, two basket strips meet to form the distal crown located at the distal end of the enlarged cell. Optionally, each basket strip has a basket strip proximal end, a basket strip distal end, and a basket strip length extending from the proximal end to the distal end. Optionally, for at least some, most, or each of the enlarged cells, the maximum angle between the two basket strips at the same location along the distal body length is at least 90 degrees. Optionally, in the relaxed state, for said at least some, most or each of the enlarged cells, two basket strips meet to form the free distal crowns of the enlarged cell, wherein each basket strip has a basket strip proximal end, a basket strip distal end, and a basket strip length extending from the proximal end to the distal end, and further wherein the maximum angle between the two basket strips at the same location along the distal body length is no more than 40 degrees. Optionally, for at least some, most or each of the enlarged cells, the average angle between the two basket strips is at least 90 degrees. Optionally, for at least some, most or each of the enlarged cells, each of the basket strip lengths are approximately equal to ½ of the distal body height and width. Optionally, from at least the proximal crowns of the cells comprising the next proximal-most free distal crowns to the proximal ends of the enlarged cells formed by the succeeding free distal crowns, the basket has no cells other than the enlarged cells and the cells comprising the free distal crowns. Optionally, from at least the proximal-most free distal crowns to the proximal ends of the enlarged cells formed by the succeeding free distal crowns, the basket has no cells other than the enlarged cells and the cells comprising the free distal crowns. Optionally, the distal crowns of the enlarged cells are attached to another cell of the basket. Optionally, in the relaxed state, the basket further comprises an additional pair of cells located about the same distance (i.e., the same distance +/- 5 mm) from the proximal junction as the cells comprising the proximal-most pair of free distal crowns and located at the 9 and 3 o'clock positions. Optionally, each cell of the additional pair of cells having a proximal crown attached to a memory metal strip and a distal crown attached to another cell of the basket. Optionally, the additional pair of cells adjoin the enlarged cells formed by the proximal-most free distal crowns. Optionally, from at least the distal crowns of the additional pair of cells to the proximal ends of the enlarged cells formed by the succeeding free distal crowns, the basket has no cells other than the enlarged cells, the cells comprising the free distal crowns and the additional pair of cells. Optionally, in the relaxed state, the basket comprises a series of bridge memory metal strips having a proximal end attached to a distal crown of a cell and a distal end attached to a proximal crown of a distally-located cell. Optionally, a proximal pair of bridge memory metal strips are located at the 3 and 9 o'clock positions. Optionally, a more distally-located pair of bridge memory metal strips are located at the 12 and 6 o'clock positions. Optionally, each of the pair of bridge memory metal strips forms part of at least one enlarged cell. Optionally, the bridge memory metal strips are the sole distally-extending basket strips attached to the respective proximal crowns and the sole proximally-extending basket strips attached to the respective distal crowns. Optionally, in the relaxed state, each of the pair of bridge memory metal strips forms part of two enlarged cells. Optionally, the bridge memory metal strips are substantially parallel to the distal body length. Optionally, the plurality of distal cells is comprised of four cells located about the same distance (i.e., the same distance +/- 5 mm) from the proximal junction. Optionally, each cell has a center, and the centers of the cells are spaced at approximately 90 degree intervals about the distal body perimeter. Optionally, each of said four cells adjoins two of the other of said four cells. Optionally, each of said four cells comprises two lateral crowns pointing generally in a direction perpendicular to the distal body length and further wherein each lateral crown of one of said four cells adjoins a lateral crown of an adjacent one of said four cells. Optionally, the distal body has no more than four cells at any location along the distal body length. Optionally, each of the enlarged cells are approximately the same size. Optionally, in the relaxed state, the basket interior is substantially hollow. Optionally, the basket comprises a series of at least five pairs of cells located on the distal body perimeter having a proximal crown pointing generally in the proximal direction and attached to a memory metal strip and a free distal crown pointing generally in the distal direction, wherein in the series, the next proximal-most free distal crowns after the succeeding free distal crowns are located at the 3 and 9 o'clock positions and located about the same distance (the same distance +/- 5 mm) from the proximal junction, and the distal-most free distal crowns are located at the 12 and 6 o'clock positions and located about the same distance (the same distance +/- 5 mm) from the proximal junction. Optionally, the enlarged cells formed by the proximal-most free distal crowns are adjoining, the enlarged cells formed by the next proximal-most free distal crowns are adjoining and the enlarged cells formed by the succeeding free distal crowns are adjoining. Optionally, each of the enlarged cells formed by the next proximal-most free distal crowns adjoins an enlarged cell formed by a proximal-most free distal crown and an enlarged cell formed by a succeeding free distal crown. Optionally, the distal body further comprises a lead wire extending distally from the distal junction. Optionally, the system further comprises a catheter having an interior, a proximal end leading to the interior and a distal end leading to the interior, the catheter comprised of a biocompatible material and configured to envelop the distal body when the distal body is in the collapsed state. Optionally, for each enlarged cell, the free distal crown is aligned with the distal crown located at the distal end of the enlarged cell. Optionally, the distal body, in the relaxed state comprises a proximal tapered region in which the distal body height and width decrease as the proximal strips approach the proximal junction. Optionally, the system is used in a method of removing a blood clot from a blood vessel of an animal that includes: a) providing the system; b) positioning the system in the blood vessel; c) allowing the height and width of the distal body to increase; d) moving the blood clot into the basket interior; and e) moving the distal body proximally out of the blood vessel.

Optionally, instead of three pair of cells in the series, the series may include only two pair of cells located on the distal body perimeter having a proximal crown pointing generally in the proximal direction and attached to a memory metal strip and a free distal crown pointing generally in the distal direction, and, in the series, the proximal-most free distal crowns may be located at the 12 and 6 o'clock positions and located about the same distance (the same distance +/- 5 mm) from the proximal junction, and the next proximal-most free distal crowns may be located at the 3 and 9 o'clock positions and located about the same distance (the same distance +/- 5 mm) from the proximal junction. In such embodiment, the distal body and system may have any feature mentioned above in connection with the distal body having at least three pairs of cells with free distal crowns.

### BRIEF DESCRIPTION OF THE DRAWINGS

FIG. 1A illustrates a side, elevation view of a memory metal tube prior to being cut by a laser.
FIG. 1B illustrates a side, elevation view of the memory metal tube of FIG. 1A being cut by a laser.
FIG. 2A illustrates a side, elevation view of the memory metal tube of FIG. 1B after being cut by a laser; in FIG. 2A, the tube is shown as though it were flat for purposes of illustrating the cut pattern only.
FIG. 2B illustrates a side, perspective view of the memory metal tube of FIG. 1B after being cut by a laser.
FIG. 2C illustrates another side, perspective view of the memory metal tube of FIG. 1B after being cut by a laser; in FIG. 2C, the tube is rotated as compared to FIG. 2B.
FIGs. 3A-3H illustrate a method of manufacturing a distal body, shown for reference purposes only using the laser cut memory metal tube of FIGs. 1 and 2; in FIGs. 3A-3H, the basket portion of the distal body is not shown for simplicity of illustration.
FIGs. 4A-4D illustrate the welding steps of the method of manufacturing shown in FIG. 3; in FIGs. 4A-4D, the basket portion of the distal body is not shown for simplicity of illustration.
FIGs. 5 and 6 illustrate different locations that connector strips may be welded to the proximal memory metal strips.
FIG. 7 illustrates a side, elevation view of a catheter and the distal body of FIG. 6.
FIG. 8 illustrates a side, elevation view of a deployable system, shown for reference purposes only being used to capture a blood clot; in FIG. 8, the basket portion of the distal body is not shown for simplicity of illustration.
FIG. 9 illustrates a side, elevation view of a claw of one embodiment, shown for reference purposes onlybeing closed by a claw actuator tube; in FIG. 9, the basket portion of the distal body is not shown for simplicity of illustration.
FIG. 10 illustrates a side, elevation view of a deployable system, shown for reference purposes onlybeing used to capture a blood clot; in FIG. 10, the basket portion of the distal body is not shown for simplicity of illustration.
FIG. 11 illustrates a first, perspective view of a distal body, shown for reference purposes only; the distal body is in what is referred to herein as "Orientation 1".
FIG. 12A illustrates a second, perspective view of the distal body of FIG. 11; the distal body is in what is referred to herein as "Orientation 2".
FIG. 12B illustrates a proximal, elevation view of the proximal strips of the distal body of FIG. 11.
FIG. 13 illustrates a close-up, perspective view of two unattached distal-pointing crowns of the distal body of FIG. 11.
FIG. 14A illustrates a native memory metal tube used to manufacture the distal body of FIG. 11; the native tube has been rolled out flat and the lines in the tube indicate where the tube has been cut by a laser.
FIG. 14B illustrates a first, perspective view of the distal body manufactured from the native tube of FIG. 14A; the distal body is in Orientation 1.
FIG. 14C illustrates a second, perspective view of the distal body manufactured from the native tube of FIG. 14A; the distal body is in Orientation 2.
FIGs. 15A-G illustrate stepwise use of the distal body of FIG. 11 in retrieving a soft clot; the distal body is in Orientation 1.
FIGs. 16A-H illustrate stepwise use of the distal body of FIG. 11 in retrieving a hard clot; the distal body is in Orientation 1.
FIGs. 17A-G illustrate stepwise use of the distal body of FIG. 11 in retrieving a soft clot; the distal body is in Orientation 2.
FIGs. 18A-G illustrate stepwise use of the distal body of FIG. 11 in retrieving a hard clot; the distal body is in Orientation 2.
FIGs. 19A-N illustrate stepwise use of the distal body of FIG. 11 in retrieving a deformable, cohesive adherent clot; the distal body is in Orientation 2.
FIG. 20A illustrates a view of a native memory metal tube used to manufacture a distal body, shown for reference purposes only; the native tube has been rolled out flat, the lines in the tube indicate where the tube has been cut by a laser, and the distal body of FIGs. 20A-20C is slightly shorter than the distal body of FIGs. 11-19 and is meant for use in tortuous blood vessels.
FIG. 20B illustrates a first, perspective view of the distal body manufactured from the native tube of FIG. 20A; the distal body is in Orientation 1.
FIG. 20C illustrates a second, perspective view of the distal body manufactured from the native tube of FIG. 20A; the distal body is in Orientation 2.
FIG. 21 shows a perspective view of a clot retrieval system that includes the distal body of FIGs. 20B-C being delivered in a blood vessel using a delivery catheter.
FIG. 22 shows a perspective view of the distal body of FIG. 21, after deployment of the distal body and retraction of the delivery catheter, in a blood vessel.
FIG. 23 shows a perspective view of the distal body of FIG. 21; as compared to FIG. 22, the distal body has been moved proximally and tension has been exerted on the pull wire.
FIG. 24 shows a perspective view of a suction catheter that is being delivered over the pull wire of the system of FIG. 21.
FIG. 25 shows a perspective view of the distal end of the suction catheter of FIG. 24 being pushed into a clot; a syringe is sucking the clot to the suction catheter because the user has pulled back on the lever of the syringe.
FIG. 26 shows a perspective view of the distal end of the suction catheter of FIG. 24 being pushed into a clot; in FIG. 26, the user has locked the syringe lever at the desired volume.
FIG. 27 shows a perspective view of the system of FIG. 24; in FIG. 27, the suction catheter has partially sucked the distal body and clot into the suction catheter.
FIG. 28 shows a perspective view of the system of FIG. 24; in FIG. 28, the suction catheter has completely sucked the distal body and clot into the suction catheter.
FIG. 29 shows a perspective view of the system of FIG. 24; the system, and captured clot, is being removed proximally from the vessel.
FIG. 30 illustrates a right side perspective view of a mandrel used to prepare unattached distal-pointing crowns that curve radially toward the basket interior.
FIG. 31 illustrates a right side elevation view of the mandrel of FIG. 30.
FIG. 32 illustrates an alternate embodiment of a distal body; in the distal body of FIG. 32, the proximal strips converge and are soldered or welded at the proximal hub/junction and the basket strips located at the distal end of the basket converge and are soldered or welded at the distal hub/junction.
FIG. 33A illustrates a native memory metal tube used to manufacture a distal body, shown for reference purposes only; the native tube has been rolled out flat and the lines in the tube indicate where the tube has been cut by a laser.
FIG. 33B illustrates a first, perspective view of the distal body manufactured from the native tube of FIG. 33A; in FIG. 33B, the distal body is in Orientation 1.
FIG. 33C illustrates a second, perspective view of the distal body manufactured from the native tube of FIG. 33A; in FIG. 33C, the distal body is in Orientation 2.
FIG. 34A illustrates a first, perspective view of a distal body, shown for reference purposes only; in FIG. 34A, the distal body is in Orientation 1.
FIG. 34B illustrates a second, perspective view of the distal body of FIG. 34A; in FIG. 34B, the distal body is in Orientation 2.
FIG. 35A illustrates a first, perspective view of a proximal portion of a distal body , shown for reference purposes only; in FIG. 35A, the distal body is in Orientation 1.
FIG. 35B illustrates a second, perspective view of the proximal portion of the distal body of FIG. 35A; in FIG. 35B, the distal body is in Orientation 2.
FIG. 36A illustrates a front perspective view of a distal body, shown for reference purposes only; in FIG. 36A, the distal body is in the relaxed state.
FIG. 36B illustrates another front perspective view of the distal body of FIG. 36A without the proximal and distal hubs/junctions and pull wire.
FIG. 36C illustrates an enlarged front perspective view of the distal body of FIG. 36A.
FIG. 36D illustrates an enlarged front perspective view of the area labelled 36D in FIG. 36C.
FIG. 36E illustrates an enlarged front perspective view of the area labelled 36E in FIG. 36C.
FIG. 36F illustrates an enlarged front perspective view of the area labelled 36F in FIG. 36C.
FIG. 36G illustrates an enlarged front perspective view of the area labelled 36G in FIG. 36E.
FIG. 36H illustrates an enlarged front perspective view of the area labelled 36H in FIG. 36F.
FIG. 36I illustrates a top plan view of the distal body of FIG. 36A.
FIG. 36J illustrates a front elevation view of the distal body of FIG. 36A.
FIG. 36K illustrates a bottom plan view of the distal body of FIG. 36A.
FIG. 36L illustrates a rear elevation view of the distal body of FIG. 36A.
FIG. 36M illustrates a proximal elevation view of the distal body of FIG. 36A; in FIG. 36M, the miniature clock faces illustrate the 12, 3, 6 and 9 o'clock positions.
FIG. 36N illustrates a distal elevation view of the distal body of FIG. 36A; in FIG. 36N, the miniature clock faces illustrate the 12, 3, 6 and 9 o'clock positions.
FIG. 37A illustrates a front perspective view of a distal body of an embodiment of the present invention; in FIG. 37A, the distal body is in the relaxed state.
FIG. 37B illustrates another front perspective view of the distal body of FIG. 37A without the proximal and distal hubs/junctions and pull wire.
FIG. 37C illustrates a top plan view of the distal body of FIG. 37A.
FIG. 37D illustrates a front elevation view of the distal body of FIG. 37A.
FIG. 37E illustrates a bottom plan view of the distal body of FIG. 37A.
FIG. 37F illustrates a rear elevation view of the distal body of FIG. 37A.
FIG. 37G illustrates an enlarged front elevation view of the area labelled 37G in FIG. 37A.
FIG. 37H illustrates a proximal elevation view of the distal body of FIG. 37A; in FIG. 37H, the miniature clock faces illustrate the 12, 3, 6 and 9 o'clock positions.
FIG. 37I illustrates a distal elevation view of the distal body of FIG. 37A; in FIG. 37I, the miniature clock faces illustrate the 12, 3, 6 and 9 o'clock positions.
FIG. 37J illustrates an enlarged front perspective view of the area labelled 37J in FIG. 37A.
FIG. 37K illustrates an enlarged rear elevation view of the area labelled 37K in FIG. 37F.

### DETAILED DESCRIPTION

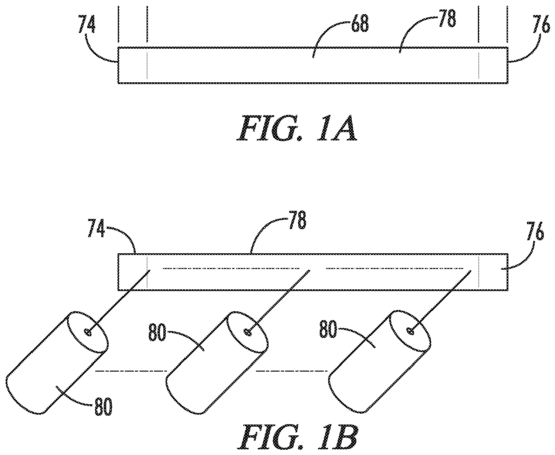

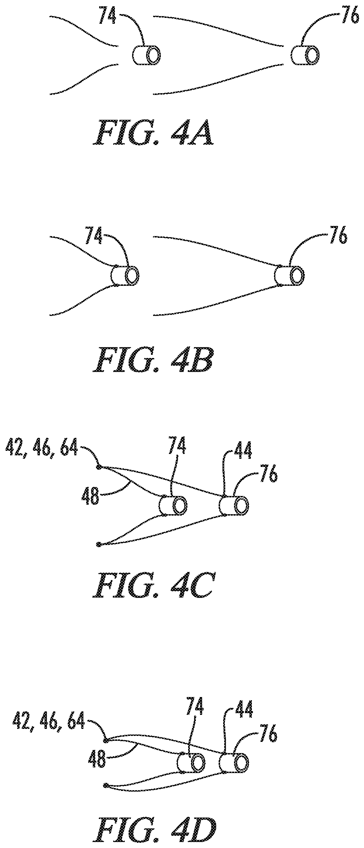

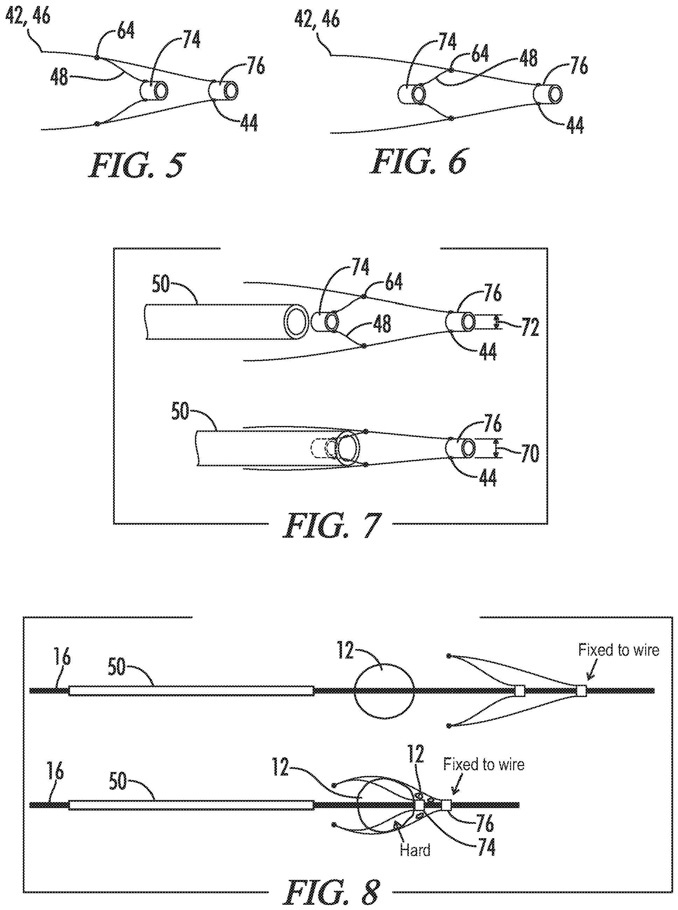

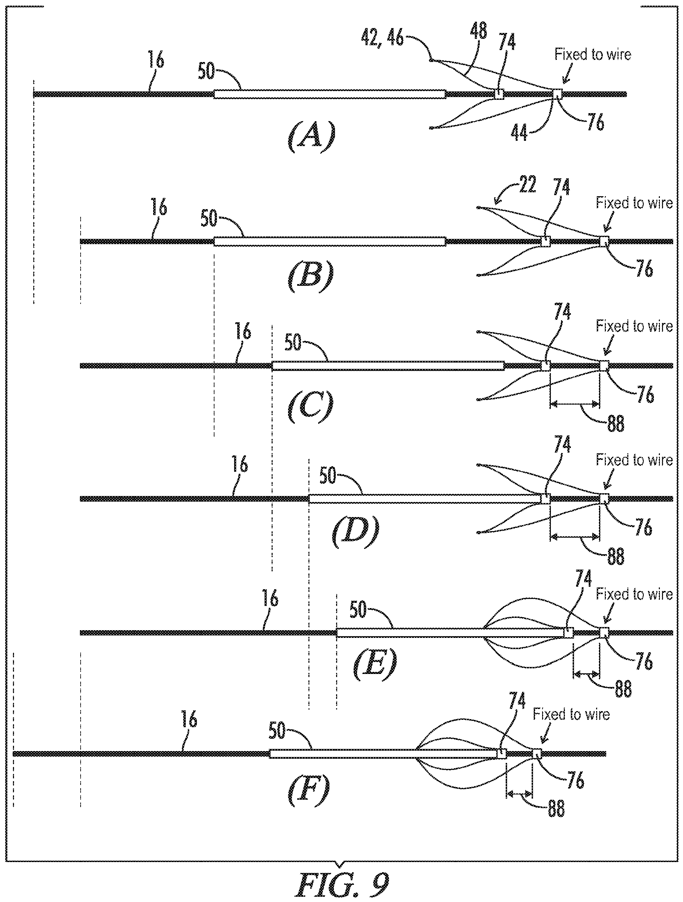

With reference to FIGs. 1-10, the present disclosure provides a deployable system, generally designated by the numeral **10,** for removing an obstruction such as a blood clot 12 or other object from a blood vessel **14** or other interior lumen of an animal. In addition to a blood clot **12,** the obstruction may be, for example, extruded coils during aneurysm treatment, intravascular embolic material such as onyx or other obstructions requiring mechanical intravascular removal from small distal vessels. In the drawings, not all reference numbers are included in each drawing for the sake of clarity.

Referring further to FIGs. 1-10, the deployable system **10** includes a pull wire **16** that has a proximal end (not shown) and a distal end **20.** Optionally, the diameter of the pull wire is between about 0.008 inches and about 0.051 inches. Preferably, the pull wire **16** is comprised of a biocompatible metallic material.

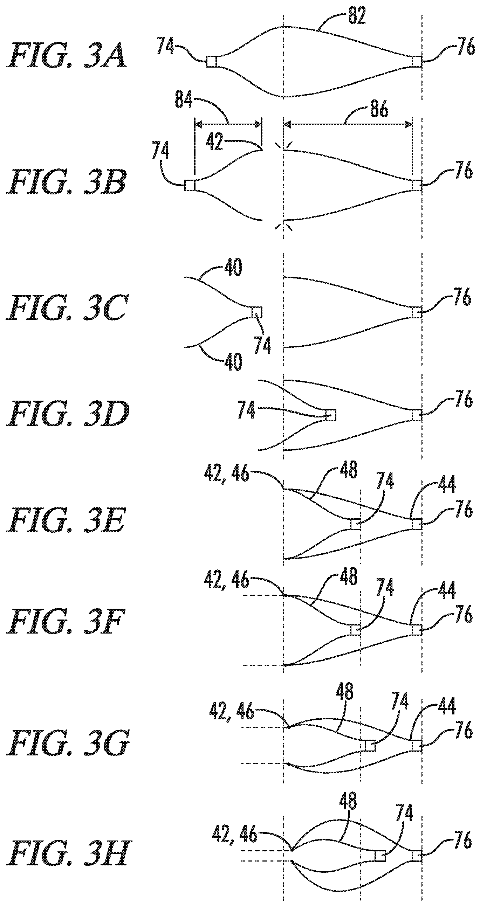

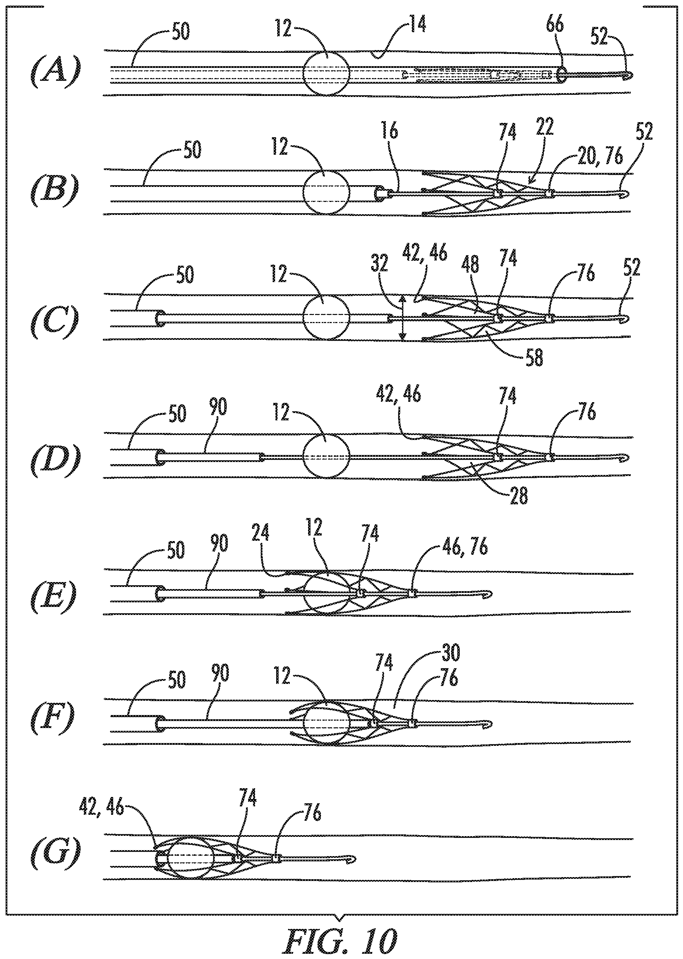

The system **10** further includes a distal body **22,** which is attached to the pull wire **16.** The distal body **22** has a proximal end **24,** a distal end **26,** an interior **28,** and an exterior **30.** The distal body **22** has a collapsed state, wherein the distal body **22** has a first height and width and is configured to fit into a catheter **50** (see FIG. 10A), and a relaxed state wherein the distal body **22** has a different height **32** and width and is configured to expand to about the height and width of a human blood vessel **14** when the distal body **22** is deployed from the catheter **50** (see FIGS. 10B-G). The distal body **22** further includes a proximal hub/junction **74** and a distal hub/junction **76** that is located distal relative to the proximal hub/junction **74.** In some examples, the distal body **22** includes a plurality of strips **40** comprised of a memory metal (e.g., a memory metal alloy such as nitinol) that form the proximal end **24** of the distal body **22.** Optionally, the proximal memory metal strips **40** each have a distal end **44** and a proximal end **42** that forms an openable and closeable claw **46.** Optionally, the proximal memory metal strips **40** are attached to the proximal hub/junction **74** through connector memory metal strips **48.** In such examples, the proximal hub/junction **74** may be slideable along at least a segment of the pull wire **16,** in contrast to the distal hub/junction **76,** which is optionally fixed to the pull wire **16** and not slideable along the pull wire **16.** Moving the proximal hub/junction **74** distally and closer to the distal hub/junction **76** (i.e., shortening the distance **88** between the proximal hub/junction **74** and distal hub/junction **76** by moving the proximal hub/junction **74** distally while keeping the distal hub/junction **76** stationary) exerts tension on the connector memory metal strips **48** and, in turn, the proximal memory metal strips **40.** This tension, in turn, causes the proximal ends **42** of the proximal memory metal strips **40** to move radially toward each other and the pull wire **16.** As the proximal ends **42** of the proximal memory metal strips **40** move radially toward each other and the pull wire **16,** the claw **46** (formed by the proximal memory metal strips **40**) is brought from the open position to at least a partially closed position, which in turn, separates the obstruction **12** from the wall of the human lumen **14** and captures the obstruction **12.** See FIG. 3H, FIG. 8, FIG. 9F, and FIG. 10F and 10G. Conversely, preferably, movement of the proximal hub/junction **74** proximally and away from the distal hub/junction **76** (i.e., increasing the distance **88** between the hubs/junctions **74** and **76**) releases the tension in the proximal memory metal strips **40,** which in turn, causes the proximal ends **42** of the proximal memory metal strips **40** to move away from each other and the pull wire **16,** opening the claw **46.** The claw **46** and proximal hub/junction **74** form several functions. First, as described, closing of the claw **46** captures the obstruction **12.** Second, closing the claw **46** retracts the claw **46** from the wall of the lumen **14** so that the claw **46** does not scrape against (and damage) the lumen wall while capturing the obstruction **12.** Third, closing the claw **46** reduces the height and width of the distal body **22,** which allows the distal body **22** to be re-sheathed in the catheter **50,** which may be desired, for example, if the operator seeks to re-deploy the distal body **22** in another location in the body (which may be the case if the operator originally deploys the distal body **22** in the wrong location in the lumen **14**). For purposes of the present disclosure, "closing the claw" embraces both partially closing the claw **46** (where the proximal ends **42** of the proximal memory metal strips **40** do not contact the pull wire **16**) and fully closing the claw **46** (where the proximal ends **42** contact the pull wire **16**).

The claw **46** may be comprised of any number of proximal memory metal strips 40. Preferably, however, between 2 and 4 proximal memory metal strips **40** comprise the claw **46** (it being understood that the connector strips **48,** if present, merely serve to tether the claw **46** to the proximal hub/junction **74**). Preferably, the proximal memory metal strips **40** have a length of between about 10 and about 60 millimeters. The proximal memory metal strips **40** can be thought of as arms of the claw **46**.

In some examples, the connector strips **48** are integral with the proximal hub/junction **74** (i.e., formed from the same piece of memory metal). In other examples, the proximal hub/junction **74** may be welded or soldered to the connector strips **48.** Optionally, in the relaxed state, the proximal memory metal strips **42** are distributed substantially evenly about a perimeter of the distal body **22.**

Optionally, the distal body **22** includes a lead wire **52** extending distally from the distal body **22.** Optionally, the lead wire **52** extends distally from the distal hub/junction **76.** If present, the lead wire **52** may be used to facilitate movement of the system **10** in the lumen **14.**

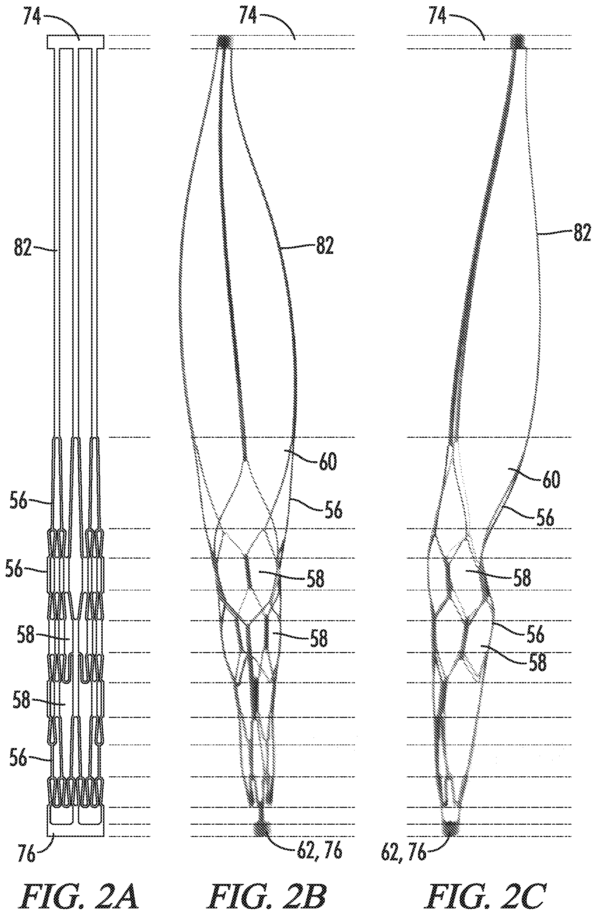

Optionally, the distal body **22** includes a basket **54** distal to the proximal memory metal strips **40,** the basket **54** comprised of a plurality of memory metal strips **56** distal relative to the proximal memory metal strips **40.** The distal memory metal strips **56** may, for example, form a basket **54** with a plurality of mesh openings **58.** Optionally, the size of the mesh openings **58** in the basket **54** when the distal body **22** is in its relaxed state is less (preferably significantly less) than the diameter of an average-sized ischemic blood clot **12** so that the blood clot **12** does not escape from the distal basket **54** after being captured by the distal body **22.** Optionally, the basket **54** has an open proximal end **60** and a substantially closed distal end **62,** which is formed by distal tube **76.** Optionally, the distal and proximal hubs/junctions **74** and **76** and the distal basket **54** are comprised of a nitinol having the same material composition. Optionally, the size of the mesh openings **58** decreases from the proximal end **60** of the basket **54** to the distal end **62.** The distal basket **54** is best seen in FIG. 2 and can be comprised of a different number of cell patterns. The distal basket **54** is not shown in FIGs. 3-10 for ease of illustrating the other components in the system **10.**

Optionally, the proximal hub/junction **74** and the distal hub/junction **76** are cylindrical tubes comprising substantially circular apertures that span the length of the hubs/junctions **74** and **76** and the hubs/junctions **74** and **76** have approximately the same inner diameter **72** and the same outer diameter **70.** Preferably, the inner diameter **72** is at least slightly larger than the diameter of the pull wire **16** so that the pull wire **16** can slide through the proximal hub/junction **74.** In some examples, the outer diameters **70** of the proximal and distal hubs/junctions **74** and **76** may be from about 0.011 inches to about 0.054 inches and the inner diameters **72** of the proximal and distal hubs/junctions **74** and **76** may be from about 0.008 inches to about 0.051 inches.

Optionally, the distal body **22** further comprises an x-ray marker **64** that is more visible under x-ray as compared to the proximal memory metal strips **40** when the distal body **22** is located in a cranial blood vessel inside the body of a human and the x-ray is taken from outside the human's body. If the connector strips **48** are welded or soldered to the proximal memory metal strips **40,** the x-ray markers **64** may be, for example, located at the welding or soldering site. In some cases, the increased thickness at the welding or soldering site may in of itself comprise the x-ray marker **64.** Preferably, the x-ray marker **64** is comprised of a radiopaque material. Some examples of radiopaque materials can include, but are not limited to, gold, platinum, palladium, tantalum, tungsten alloy, polymer material loaded with radiopaque filler, and the like. Preferably, the proximal memory metal strips **40** are comprised of nitinol and the x-ray marker **64** is comprised of a material having a density greater than the nitinol.

A catheter **50** with an open proximal end (not shown) and an open distal end 66 initially envelopes the system **10.** As used herein, the term "catheter" generally refers to any suitable tube through which the system **10** can be deployed. Preferably, the catheter **50** is sterile and comprised of a biocompatible material (i.e., a material that does not irritate the human body during the course of a 45 minute operation that involves using the system **10** to remove a clot **12** from an intracranial blood vessel **14**). The catheter **50** can be any suitable shape, including but not limited to generally cylindrical. Preferably, the catheter **50** is a microcatheter. For purposes of the present disclosure, when it is said that the catheter **50** envelopes the system **10,** it will be understood that the catheter **50** envelopes at least one component of the system **10** (preferably, the distal body **22,** the lead wire **52,** and the pull wire **16**). In some examples, the catheter **50** is about 2.5 French in diameter. Optionally, the catheter **50** is delivered to the region of the lumen **14** that has the obstruction **12** as follows: a guide wire is delivered to the obstruction region past the obstruction **12;** the catheter **50** is delivered over the guide wire; the guide wire is removed; and the system **10** is delivered with its pull wire **16** and lead wire **52** through the catheter **50.** Optionally, the pull wire **16** is used to push the system **10** through the catheter **50** as well as to retrieve the distal body **22** after capturing the obstruction **14** as described below. The system **10** may utilize a plurality of catheters **50,** such as, for example, a wider catheter that travels to the brain and a very flexible, smaller diameter microcatheter that is delivered from the first catheter and travels through the small arteries of the brain. Preferably, the catheter **50** is comprised of a biocompatible, polymeric material (i.e., one or more polymeric materials such as silicone, PVC, latex rubber or braided nylon).

Optionally, in the relaxed, opened-claw state, the distal body **22 or** optionally just the distal basket **54** has a tapered shape (e.g., substantially conical or bullet in shape) so that the distal body **22** or just the distal basket **54** tapers from the distal body **22** or the distal basket's **54** proximal end to the distal end.

The proximal end of the system **10** is shown at the left end of FIGs. 1 and 3-10 and the distal end of the system **10** is shown at the right end of FIGs. 1 and 3-10 because a principal use of the system **10** is to remove a blood clot **12** from a human intracranial artery **14,** in which case the system **10** generally will enter the artery **14** at its proximal end by the surgeon entering the patient's body near the groin and pushing the catheter **50** towards the brain. The diameter of human arteries **14** generally decrease from their proximal end to their distal end. However, when used in other types of lumens, the distal body **22** may be located proximally relative to the catheter **50** as the term proximally and distally are used in that lumen.

The surgeon may deploy the distal body **22** by, for example, moving the catheter **50** proximally so as to unsheathe the distal body **22** or by pushing the distal body **22** out of the catheter **50.**

Use of the system **10** will now be described to remove a blood clot **12 from** an intracranial artery **14** of a human ischemic stroke patient, however, it will be appreciated that the system **10** may be used to remove other objects from other interior lumens.

A catheter **50,** which contains the collapsed distal body **22** is positioned in the lumen **14** distal to the clot **12.** See FIG. 10A.

The distal body **22** is deployed from the catheter **50** and the height and width of the distal body **22** expand to about the height and width of the blood vessel **14.** See FIG. 10B.

The catheter **50 is** pulled proximally and a claw-actuator tube **90** is deployed into the blood vessel **14.** See FIG. 10C.

The distal body **22** is moved proximally so that the clot **12** is located in the interior **28** of the distal body **22.** See FIGs. 10D and 10E.

The claw-actuator tube **90** is moved distally, which pushes the proximal hub/junction **74** distally so that the distance **88** between the proximal hub/junction **74** and the distal hub/junction **76** (which is fixed to the pull wire **16** and kept stationary) decreases. Distal movement of the proximal hub/junction **74** exerts tension on the connector and proximal memory metal strips **40** and **48,** which in turn, closes the claw **46.** See FIG. 10F. (The claw actuator tube **90** should float on the pull wire **16** - i.e., have an aperture extending the tube's length that has a diameter larger than the diameter of the pull wire **16** - and the aperture of the claw actuator tube **90** should be smaller than the diameter of the proximal hub/junction **74** so that the claw actuator tube **90** pushes the proximal hub/junction **74**).

The system **10** is withdrawn proximally and removed from the body. See FIG. 10G.

To test the efficacy of the system **10,** a distal body **22** with a distal basket **54,** proximal and distal hubs/junctions **74** and **76,** and a claw **46** comprised of three proximal memory metal strips **42** was tested in a flow model that included a tube and a moist cotton ball located in the tube. The cotton ball was used to simulate a blood clot. The system **10** was deployed distal to the cotton ball. The claw **46** was closed by moving the proximal hub/junction **74** distally to capture the cotton ball. The system **10** and cotton ball were withdrawn proximally in the tube.

In some examples, the distal body **22** is prepared by a process that includes one or more of the following steps, as illustrated in FIGs. 1-4
a) providing a single tube **68** comprised of a memory metal such as nitinol, the single tube **68** having an exterior, a substantially hollow interior, a wall separating the exterior from the substantially hollow interior, an open proximal end **74,** an open distal end **76,** a middle portion **78** between the open proximal end **74** and the open distal end **76** (see FIG. 1A);
b) cutting the wall of the middle portion **78** with a laser **80** (see FIG. 1B);
c) removing the pieces of the middle portion **78** cut by the laser **80** to form a proximal tube **74,** a distal tube **76** and a middle portion **78** comprising a plurality of memory metal strips **82** attached to the proximal tube **74;**
d) altering the shape of the middle portion **78** using a mandrel and allowing the middle portion **78** to expand relative to the distal tube **76** and proximal tube **74** to form the distal basket **54;**
e) quenching the middle portion **78** at room temperature;
f) removing the mandrel from the middle portion **78** (see FIGs. 2 and 3A);
g) mechanically or chemically electropolishing the middle portion **78** to remove oxides;
h) cutting the memory metal strips **82** to form a first segment **84** comprising the proximal tube **74** and a proximal segment of the memory metal strips **82** and a second segment **86** comprising the distal tube **76** and a distal segment of the memory metal strips **82** (see FIG. 3B); and
i) joining the proximal segments to the distal segments such that the distal segments form the proximal end **24** of the distal body **22,** such that the proximal tube **74** is located inside the interior **28** of the distal body **22,** and such the proximal tube **74** is located distal relative to the distal body proximal end **24** (see FIGs. 3C-3E).

In some examples, the method further includes placing the pull wire **16** through the proximal tube **74** so that the proximal tube **74** is slideable along at least a segment of the pull wire **16.**

In some examples, the method further includes attaching the pull wire **16** to the distal tube **76** so that the distal tube **76** is not slideable along the pull wire **16** but instead the distal tube **76** moves with the pull wire **16.**

In some examples, after step i, the proximal end **24** of the distal body **22** forms a claw **46** comprised of between 2 to 4 proximal memory metal strips **40,** the claw proximal memory metal strips **40** configured to move towards each other and the pull wire **16** by moving the proximal tube **74** distally and toward the distal tube **76** (i.e., decreasing the distance **88** between the tubes **74** and **76**) and the claw memory metal strips **40** configured to move away from each other and away from the pull wire (i.e., increasing the distance **88** between the tubes **74** and **76**) by moving the proximal tube **76** proximally and away from the distal tube **76** (as described previously).

In some examples, the middle portion **78** is expanded by heating the mandrel and the middle portion **78** by, for example, placing the mandrel and the middle portion **78** in a fluidized sand bath at about 500°C for about 3 to about 7 minutes. As the middle portion **78** is heated, the heating causes the crystalline structure of the memory metal tube **68** to realign. Preferably, the mandrel is tapered (e.g., substantially conical or bullet in shape) so that the distal basket **54** formed from the middle portion **78** tapers from the proximal end **60** to the distal end **62.** Preferably, the proximal and distal ends of the tube **74** and **76** are not shape set by the mandrel and are not cut by the laser **80** so that the proximal and distal ends **74** and **76** do not change in shape and only slightly expand in size under heating and return to the size of the native tube **68** after the heat is removed. Preferably, the laser cuts are programmed via a computer. To ensure that the laser cuts only one surface of the tube wall at the time (and not the surface directly opposite the desired cutting surface), the laser **80** is preferably focused between the inner and outer diameter of the desired cutting surface and a coolant is passed through the memory metal tube **68** so that the laser **80** cools before reaching the surface directly opposite the desired cutting surface.

The portions of the wall not cut by the laser **80** create the distal basket **53,** proximal and distal tubes **74** and **76,** and memory metal strips **40, 48** and **56,** as described.

Preferably, the memory metal selected for the native tube **68** has a heat of transformation below average human body temperature (37°C) so that the distal body **22** has sufficient spring and flexibility after deployment from the catheter **50** in the human blood vessel **14.**

In some examples, the native tube **68** (and hence the distal and proximal tubes **74** and 76) have an outer diameter of less than about 4 French, e.g., a diameter of about 1 to about 4 French. In some examples, the diameter of the pull wire **16** is between about 0.008 inches and about 0.051, as noted above, and in such examples, the diameter of the pull wire **16** may be approximately equal to the inner diameter **72** of the native nitinol tube **68.**

Without being bound by any particular theory, it is believed that manufacturing the distal body **22** from a single memory metal tube **68** provides ease of manufacturing and safety from mechanical failure and provides tensile strength necessary for the system **10** to remove hard thrombus **12** and other obstructions.

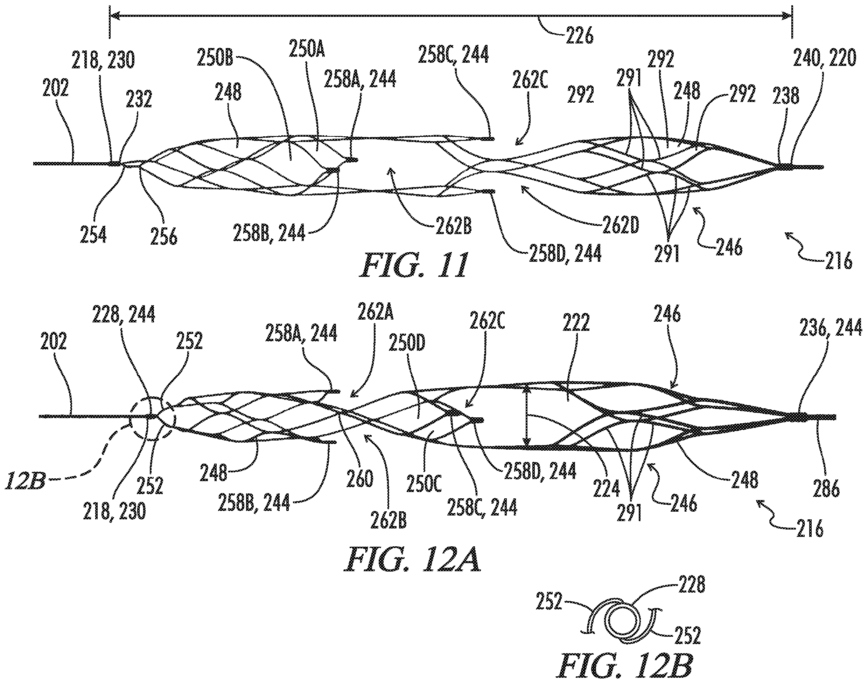

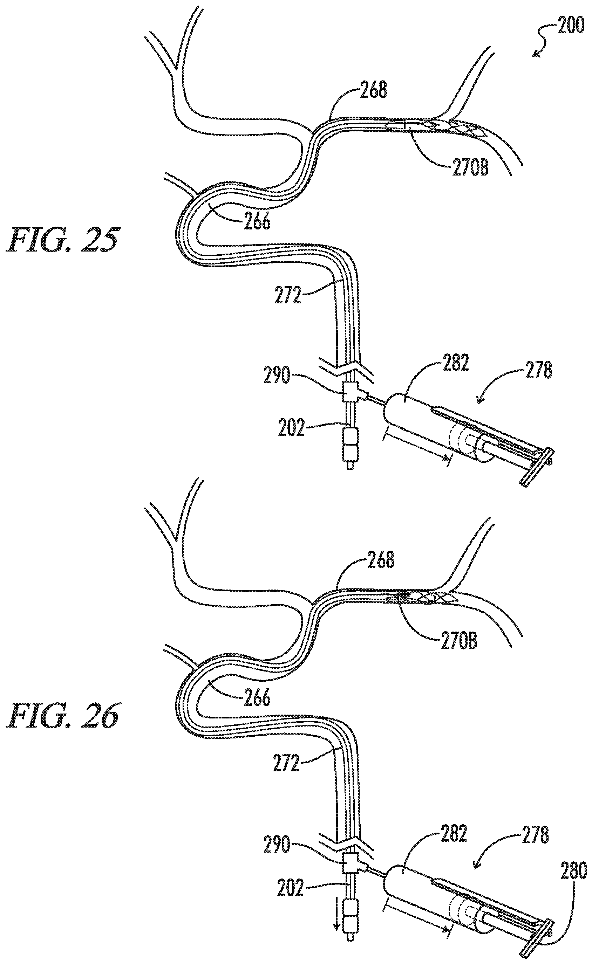

### The examples of Figures 11-29

Figures 11-29 illustrate an alternate examples **200** that includes one or more of the following additional features, as described below: twisting proximal strips/tethers **252,** unattached/free distal-pointing crowns **258** that optionally curve inward and have x-ray markers **244,** and enlarged openings/drop zones **262** in the basket **246** immediately distal to the unattached, distal-pointing crowns **258** that allow the obstruction or other object **270** to enter the distal basket interior **222.**

More specifically, as shown in FIGs. 11-29, the system **200** may include a pull wire **202** having a proximal end **204** and a distal end **206,** as described above, a distal body **216** attached to the pull wire **202,** the distal body **216** comprising an interior **222,** a proximal end **218,** a distal end **220,** a distal body length **226** extending from the proximal end **218** to the distal end **220,** a distal body height **224,** a proximal hub/junction **228** (preferably in the form of a tube and which has a proximal end **230** and a distal end **232**) forming the proximal end **218** of the distal body **216,** a basket **246** comprised of a plurality of cells/openings **248** formed by a plurality of basket strips **291** that preferably are comprised of a memory metal, optionally a distal hub/junction **236** that forms the distal end of the basket **246** (preferably in the form of a tube that has a proximal end **238** and a distal end **240**), and a plurality of proximal strips **252** (preferably the proximal strips **252** are comprised of a memory metal), each proximal strip **252** having a proximal end **254** attached to the proximal hub/junction/tube **228,** and a distal end **256** attached to a cell **248** (more specifically a proximal-pointing crown of a cell **248** located at the proximal end of the basket **246**), the basket comprising a basket interior **292,** the distal body **216** having a relaxed state wherein the distal body **216** has a first height and width, a collapsed state wherein the distal body **216** has a second height and width, the second height less than the first height, the second width less than the first width; and a delivery catheter **208** for delivering the distal body **216,** as described above, having an interior **210,** a proximal end **212** leading to the interior **210** and a distal end **214** leading to the interior **210,** the delivery catheter **208** comprised of a biocompatible (preferably polymeric) material and configured to envelope the distal body **216** when the distal body **216** is in the collapsed state. Optionally, the basket interior **292** is substantially hollow - i.e., unlike U.S. Patent Publication No. 2013/0345739, the basket interior **292** does not contain an inner elongate body. Optionally, instead of a distal hub/junction **236,** the basket **246** includes an open distal end. Optionally, at least two cells **250** of the basket **246** comprise a proximal crown **260** pointing generally in the proximal direction and a distal crown **258** pointing generally in the distal direction, and the distal crowns **258** of the at least two cells **250** are not attached to another cell **248** of the basket **246.** In other words, the distal crowns **258** of at least two cells **250** are free floating and are not attached to any strip except for the strips forming part of the at least two cells **250;** such distal crowns **258** are referred to below as unattached, distal-pointing crowns **258.** Preferably, the distal tips of the unattached, distal-pointing crowns **258** terminate at an x-ray marker **244.** (Cells labeled with the numerals **250, 250A, 250B, 250C,** and **250D** refer to the at least two cells that include a proximal crown **260** pointing generally in the proximal direction and an unattached, distal-pointing crown **258,** cells labeled with the numerals **262, 262A, 262B, 262C,** and **262D** refer to the enlarged cells/drop zones adjacent to (preferably immediately distal to) an unattached, distal-pointing crown **258,** and cells designated with numeral **248** refer to generally the cells of the basket **246**). (When it is said that the enlarged cells/drop zones **262** are preferably immediately distal to an unattached, distal-pointing crown **258,** it will be understood that at least a portion of an enlarged cell/drop zone **262** is immediately distal to an unattached, distal-pointing crown **258,** and that a portion of the enlarged cell/drop zone **262** may be proximal to an unattached, distal-pointing crown **258,** as shown in FIGs. 11-12 due to the shape of the enlarged cells/drop zones **262**). It will be understood that part number **250** refers generally to one or more of the at least two cells, whereas part numbers **250A, 250B, 250C,** and **250D** refer to a specific one of the at least two cells. Similarly, it will be understood that part number **262** refers generally to one or more of the enlarged cells/drop zones, whereas part numbers **262A, 262B, 262C,** and **262D** refer to a specific one of the enlarged cells/drop zones. Similarly, it will be understood that part number **258** refers generally to one or more of the unattached, distal-pointing crowns, whereas part numbers **258A, 258B, 258C,** and **258D** refer to a specific one of the unattached, distal-pointing crowns.

Optionally, at least two of the unattached, distal-pointing crowns **258** are located approximately 180 degrees (e.g., about 150 to about 180 degrees) relative to each other and approximately the same distance from the proximal hub/junction/tube **228,** as best seen in FIG. 12A. Optionally, the basket **246** comprises a first pair of unattached, distal-pointing crowns **258A** and **258B,** each of the first pair of unattached, distal-pointing crowns **258A** and **258B** is located approximately the same distance from the proximal hub/junction/tube **228** and approximately 180 degrees relative to each other, and the basket **246** further comprises a second pair of unattached, distal-pointing crowns **258C** and **258D** located distally relative to, and approximately 90 degrees (e.g., between about 60 and about 90 degrees) relative to, the first pair of unattached, distal-pointing crowns **258A** and **258B.** Optionally, the second pair of unattached, distal-pointing crowns **258C** and **258D** form cells **250C** and **250D** that are adjacent to, but offset from, the cells **250A** and **250B** formed by the first pair of unattached, distal-pointing crowns **258A** and **258B.** (In other words, optionally, the center of cell **250A** is about 90 degrees relative to the centers of cells **250C** and **250D** and optionally the center of cell **250B** is also about 90 degrees relative to the centers of cells **250C** and **250D**). Optionally, at least one of (and preferably all) the unattached, distal-pointing crowns **258A, 258B, 258C or 258D** comprise an x-ray marker **244** that is more visible under x-ray as compared to the basket strips **291** when the distal body **216** is located in a cranial blood vessel **266** inside the body of a human and the x-ray is taken from outside the human's body. Preferably, the x-ray marker **244** is a radiopaque material. Some examples of radiopaque materials can include, but are not limited to, gold, platinum, palladium, tantalum, tungsten alloy, polymer material loaded with radiopaque filler, and the like. Preferably, the basket strips **291** are comprised of nitinol and the x-ray marker **244** is comprised of a material having a density greater than the nitinol. In some examples, the x-ray markers **244** comprise a heavy metal welded or soldered to the unattached, distal-pointing crowns **258.** Optionally, the unattached, distal-pointing crowns **258** curve subtly towards the interior **222** of the distal basket **246,** which decreases the likelihood that the unattached, distal-pointing crowns **258** will rub against and damage the vessel wall **268.** Optionally, the basket **246** comprises at least two cells proximal to the at least two cells **250** that include the unattached, distal-pointing crowns **258.** Optionally, the unattached, distal-pointing distal crowns **258** are located about at least 5 mm (e.g., about 5 to about 30 mm) from the proximal hub/junction/tube **228.** Optionally, the unattached, distal-pointing crowns **258** are located at least about 5 mm from the distal hub/junction/tube **236.** Optionally, the unattached, distal-pointing crowns **258** of the at least two cells **250** also each form part (namely a portion of the proximal boundary) of an enlarged cell **262** (which is the entry point of hard thrombus **270B** into the basket interior **222**) and further wherein the surface area of the enlarged cells **262** in the relaxed state is greater than the surface area of the other cells of the basket **246** in the relaxed state. Optionally, the unattached, distal-pointing crowns **258** serve several functions: 1) they form flex points of the basket **246,** which makes it easier for the system **200** to navigate the curves of the blood vessels **266** of the brains; 2) through the use of x-ray markers **244** on the unattached, distal-pointing crowns **258,** they allow the operator to locate the enlarged cells **262** of the basket **246** that form the point at which hard thrombuses **270B** enter the basket **246;** and 3) they allow the operator to ratchet or force the object **270** into the basket **246** by moving the unattached, distal-pointing crowns **258** proximally and distally relative to the object **270.** (As explained below, the numeral **270** refers to clots/thrombuses and other objects generally, and **270A** refers to a soft clot, **270B** refers to a hard clot and **270C** refers to a deformable, cohesive, adherent clot). Optionally, the proximal end **254** of a proximal strip **252** is located about 65-180 degrees (preferably approximately 180 degrees) relative to the distal end **256** of the same proximal strip **252,** as best seen in FIG. 12B. In other words, preferably the proximal end **254** of a first proximal strip **252** is attached to the 12 o'clock position on the proximal tube **228** and the distal end **256** of the first proximal strip **252** (which terminates at a proximal cell **248** of the basket **246)** is located at the 6 o'clock position (i.e., 180 degrees from the start position), and the proximal end **254** of a second proximal strip **252** is attached to the 6 o'clock position on the proximal tube **228** and the distal end **254** (which terminates at a cell **248** of the basket **246)** of the second proximal strip **252** is located at the 12 o'clock position (i.e., 180 degrees from the start position). This twisting feature serves two functions: 1) it allows the proximal strips **252** to surround the object **270;** and 2) it allows the manufacturer to insert a mandrel into the basket **246** during the shape-setting procedure. Optionally, the pull wire **202** is attached to the proximal tube **228** (e.g., by gluing, welding, soldering or the like). Preferably, the pull wire **202** does not extend through the distal basket interior **222.** Optionally, the proximal strips **252** are integral with the distal end **232** of the proximal tube **228** and the entire distal body **216** is created from a single tube **264** of a memory metal. Optionally, the proximal crowns **260** of the at least two cells **250** that include the unattached, distal pointing-crowns **258** are each attached to another cell **248** of the basket **246.** In other words, preferably the basket **246** does not have any free-floating proximal-pointing crowns, as free-floating proximal-pointing crowns could damage the vessel **266** when the distal body **216** is pulled proximally. Optionally, the system **200** further comprises a lead wire **286** extending distally from the distal tube **236,** the lead wire **286** having a length of from about 3 mm to about 10 mm. Optionally, the distal hub/junction/tube **236,** the proximal hub/junction/tube **228,** and the basket **246** are comprised of a nitinol having the same material composition. In other words, as with the prior example of FIGs. 1-10, optionally the entire distal body **216** is manufactured from a single tube of nitinol **264.** Optionally, the proximal and distal hubs/junctions/tubes **228** and **236** comprise an x-ray marker **244** that is more visible under x-ray as compared to the basket strips **291** when the distal body **216** is located in a cranial blood vessel **266** inside the body of a human and the x-ray is taken from outside the human's body. Preferably, the x-ray marker **244** is a radiopaque material. Some examples of radiopaque materials can include, but are not limited to, gold, platinum, palladium, tantalum, tungsten alloy, polymer material loaded with radiopaque filler, and the like. Preferably, the basket strips **291** are comprised of nitinol and the x-ray marker **244** is comprised of a material having a density greater than the nitinol. In some examples, the proximal and distal hubs/junctions/tube interiors **234** and **242** may comprise tantalum welded or otherwise attached to the interior **234** and **242** of the proximal and distal hubs/junctions/tubes **228** and **236.** Optionally, the proximal and the distal tubes **228** and **236** are generally cylindrical in shape and each has an outer diameter and an inner diameter, the inner diameter forming apertures of the proximal and distal tubes **228** and **236** and further wherein the outer diameters of the proximal and distal tubes **228** and **236** are substantially the same size and further wherein the inner diameters of the proximal and distal tubes **228** and **236** are substantially the same size. Optionally, the outer diameters of the proximal and distal tubes **228** and **236** are from about 0.011 inches to about 0.054 inches, and further wherein the inner diameters of the proximal and distal tubes **228** and **236** are from about 0.008 inches to about 0.051 inches. Optionally, the pull wire **202** is generally cylindrical and further wherein the diameter of the pull wire **202** is between about 0.008 inches and about 0.051 inches. Optionally, the distal body **216** has a length of between about 10 and about 60 millimeters. Optionally, the first height **224** and first width **226** of the distal body **216** are between about 2 millimeters and about 6 millimeters.

The present disclosure also provides a method of removing a clot **or** other object **270** from an interior lumen **266** of an animal, the method comprising the steps of:
a) providing the system **200** of Figures 11-29, wherein at least two cells **250** of the basket **246** comprise a proximal crown **260** pointing generally in the proximal direction and a distal crown **258** pointing generally in the distal direction, and the distal crowns **258** of the at least two cells **250** are not attached to another cell **248** of the basket **246** (i.e., free-floating), and further wherein at least one of the unattached, distal-pointing crowns **258** comprises an x-ray marker **244;**
b) positioning the system **200** in the lumen **266;**
c) deploying the distal body **216** from the distal end **214** of the delivery catheter **208;**
d) allowing the height and width **224** and **226** of the distal body **216** to increase;
e) irradiating the x-ray marker **244** with x-ray radiation and
f) moving the object **270** into the distal basket interior **222.**

Optionally, the object **270** enters the distal basket interior **222** adjacent to (preferably adjacent and immediately distal to) at least one of the unattached, distal-pointing crowns **258** - i.e., in the enlarged cells/drop zones **262.** In some examples, the distal body **216** is deployed so that at least one (e.g., preferably the two proximal **258A** and **258B**) of the unattached, distal-pointing crowns **258** is distal to the object **270.** As explained below, the x-ray markers **244** of the unattached, distal-pointing crowns **258** are used to locate the distal body **216** relative to the clot or other object **270.** It will be appreciated that clots **270** can generally be located in blood vessels **266** by injecting a contrast dye, for example, into the blood vessel **266** proximal and distal to the believed area of obstruction and viewing on an x-ray where the fluid stops moving in the blood vessel **266.** It will also be appreciated that if the object **270** is not a blood clot but is a radio-opaque object, the object **270** may be viewed on an x-ray.

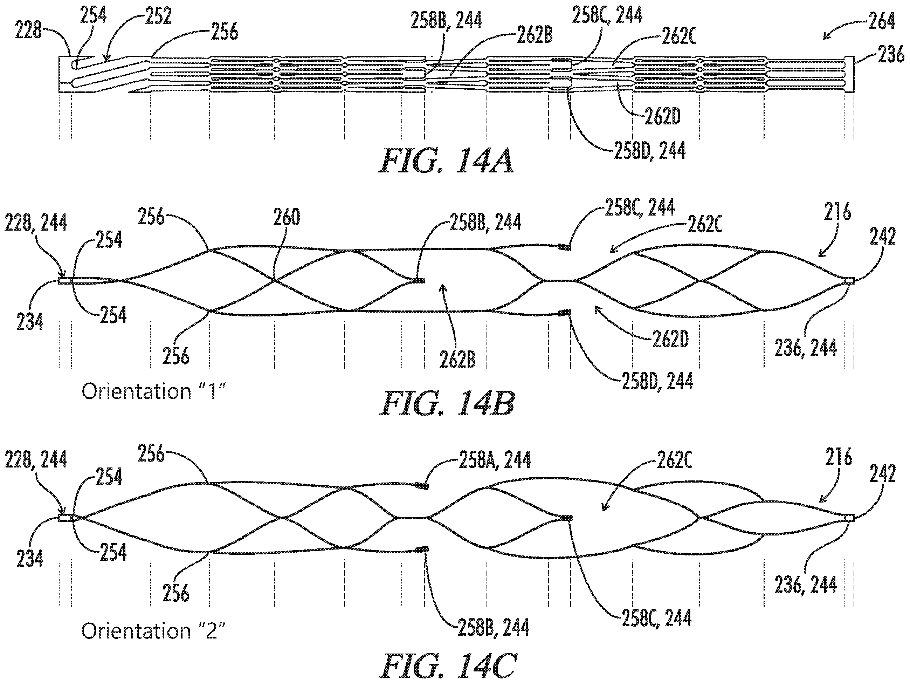

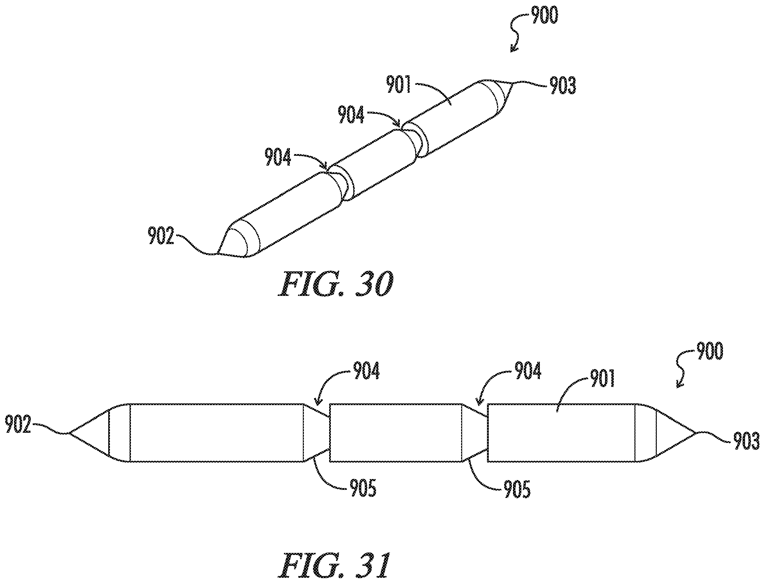

FIGs. 11 and 14B illustrate a first, perspective view of one exampleof a distal body 216 with twisting proximal strips **252,** unattached distal-pointing crowns **258** that subtly curve inward and have x-ray markers **244,** and enlarged openings/drop zones **262** in the basket **246** that allow the obstruction or other object **270** to enter. In FIGs. 11 and 14B, the distal body **216** is in Orientation 1. (To prepare a basket **246** with unattached distal-pointing crowns **258** that curve inward toward the basket interior **292,** a mandrel **900** such as that illustrated in FIGs. 63 and 64 may be used. The mandrel **900** includes a generally cylindrical body 901 with tapered proximal and distal ends **902** and **903** that slope like the ends of a pencil. The cylindrical body **901** includes two grooves **904** that extend around the circumference of the cylindrical body **901.** The grooves **904** include tapered portions **905** that slope towards the distal end **903,** which are designed to shape the unattached distal-pointing crowns **258.** The grooves **904** are generally in the shape of a truncated cone, as shown in FIGs. 30-31). The two proximal, unattached distal-pointing crowns **258A** and **258B** are located approximately the same distance from the proximal hub/junction/tube **228** and are oriented approximately 180 degrees relative to each other. The two distal, unattached distal-pointing crowns **258C** and **258D** are located approximately the same distance from the proximal hub/junction/tube **228** as each other (and distal to the two proximal, unattached distal-pointing crowns **258A** and **258B**) and are oriented approximately 180 degrees relative to each other and approximately 90 degrees to the proximal, unattached distal-pointing crowns **258A** and **258B.** For example, for purposes of FIGs. 11-29, "approximately the same distance from the proximal hub/junction/tube **228"** means that if one free distal crown **258A** of the first pair of distal crowns **258A/258B** is located X distance from the proximal hub/junction/tube **228,** the other distal crown **258B** of the first pair of distal crowns **258A/258B** is located X distance plus or minus (+/-) 3 millimeters (mm) from the proximal hub/junction/tube **228.** Similarly, if one free distal crown **258C** of the second pair of distal crowns **258C/258D** is located Y distance from the proximal hub/junction/tube **228,** the other distal crown **258D** of the second pair of distal crowns **258C/258D** is located Y distance plus or minus (+/-) 3 mm from the proximal hub/junction/tube **228.** In a preferred example, the first free distal crowns **258A** and **258B** are located the same distance +/- 0.5 mm from the proximal hub/junction/tube **228.** Similarly, in a preferred example, the second free distal crowns **258C** and **258D** are located the same distance +/- 0.5 mm from the proximal hub/junction/tube **228.**

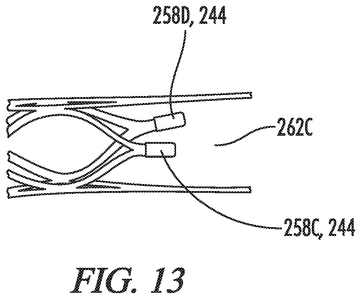

The two proximal enlarged openings/drop zones **262A** and **262B** distal to the proximal, unattached distal pointing crowns **258A** and **258B** are located approximately the same distance from the proximal hub/junction/tube **228** and the centers of the two proximal enlarged openings/drop zones **262A** and **262B** are oriented approximately 180 degrees relative to each other. (As noted above, preferably, the proximal, unattached distal-pointing crowns **258A** and **258B** form part of the proximal boundary of the proximal, enlarged cells/drop zones **262A** and **262B,** and the distal, unattached distal-pointing crowns **258C** and **258C** form part of the proximal boundary of the distal, enlarged cells/drop zones **262C** and **262D**). The two distal, enlarged openings/drop zones **262C** and **262D** distal to the distal, unattached distal pointing crowns **258C** and **258D** are located approximately the same distance from the proximal hub/junction/tube **228** and the centers of the distal, enlarged openings/drop zones **262C** and **262D** are oriented approximately 180 degrees relative to each other and approximately 90 degrees relative to the proximal enlarged openings/drop zones **262A** and **262B.** FIGs. 12A and 14C illustrate a second view of the distal body **216** of FIG. 11 (Orientation 2). FIG. 13 is a close-up view of two unattached, distal-pointing crowns **262.** The lines in FIG. 14 show how a nitinol tube **264** is cut with a laser to create the distal body **216** shown in FIG. 14B and FIG. 14C. It will be appreciated that FIG. 14B is a simplified view of the distal body 216 and orientation shown in FIG. 11 and FIG. 14C is a simplified view of the distal body **216** and orientation shown in FIG. 12A.

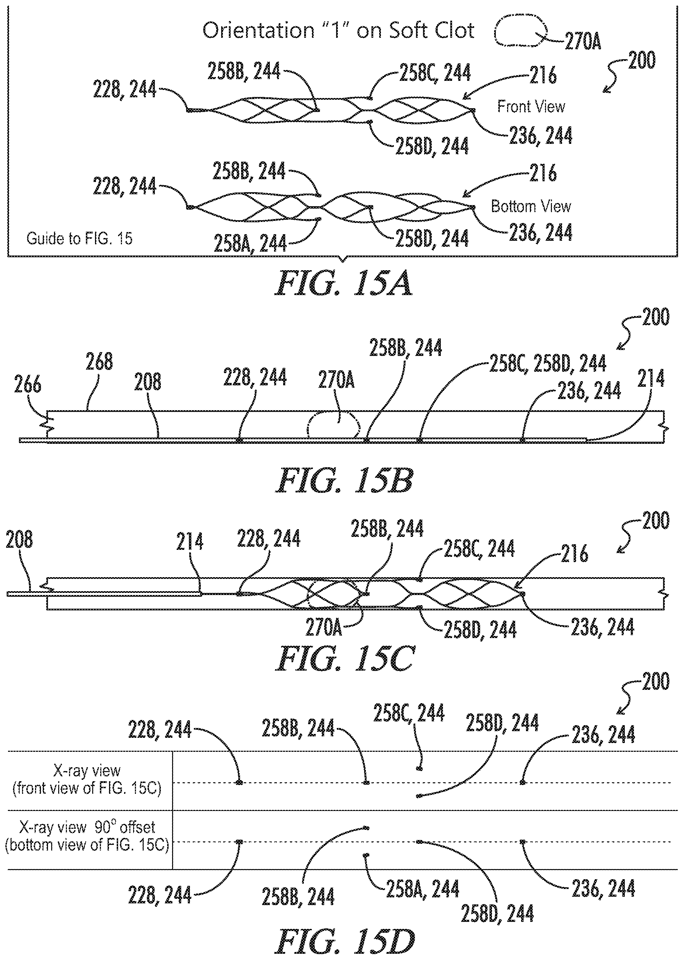

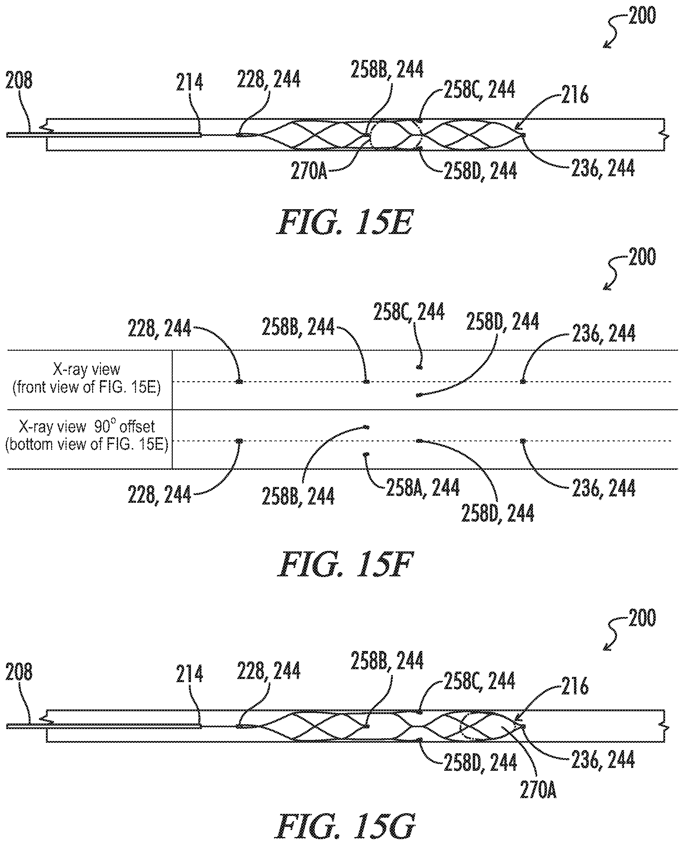

As described below, FIGs. 15-19 describe how the distal body **216** is used to retrieve, soft clots **270A,** hard clots **270B,** and deformable, cohesive adhesive clots **270C** in a human intracranial artery **266.** (In FIGs. 15-19, the center of the artery **266** is denominated by the dashed line). As explained below, the distal body **216** has four rows of x-ray markers namely, 1) a first row of one x-ray marker, which is located inside the proximal tube denominated by the numeral **228, 244;** 2) a second row of two x-ray markers, which are located at the two proximal, unattached distal-pointing crowns (the two markers are oriented 180 degrees relative to each other) denominated by the numerals **258A, 244** and **258B, 244;** 3) a third row of two x-ray markers, which are located at the two distal, unattached distal-pointing crowns (these two markers are oriented 180 degrees relative to each other and 90 degrees relative to the two proximal, unattached distal-pointing crowns) denominated by the numerals **258C, 244** and **258D, 244;** and 4) a fourth row of one x-ray marker, which is located inside the distal tube denominated by the numeral **236, 244.** (It will be appreciated that the first number in the sequence describes the position of the x-ray marker and the second number, **244,** represents the fact that the item is an x-ray marker). As explained below, upon deploying the distal body **216** so that the two proximal, unattached distal-pointing crowns **258A, 244** and **258B, 244** are immediately distal to the clot **270,** the surgeon interventionalist (i.e., operator of the distal body **216**) detects the four rows of x-ray markers using x-ray radiation from a first vantage point and from a second vantage point that is offset from the first vantage point (e.g. 90 degrees). Next, the surgeon moves the distal body **216** proximally relative to the clot **270** and takes additional x-rays from the first and second vantage points. As explained in greater detail below, the surgeon uses the x-ray markers of the proximal and distal, unattached distal-pointing crowns, namely **258A, 244; 258B, 244; 258C, 244;** and **258D, 244** (more specifically, the convergence or lack thereof of the proximal and distal, unattached distal-pointing crowns **258A, 244; 258B, 244; 258C, 244;** and **258D, 244** as shown on the x-ray) to determine whether the clot **270** is located inside the distal body interior **222** or whether the clot **270** is collapsing the distal body **216.**

More specifically, FIGs. 15A-G illustrate stepwise use of the distal body **216** in retrieving a soft clot **270A** in a human intracranial artery **266.** (The distal body **216** in FIGS. 15A-15G is in Orientation 1). First, as always, the surgeon determines the location of the clot **270A** in the vessel **266** using, for example, a contrast dye injected proximal and distal to the clot **270A.** Next, the delivery catheter **208,** which is enveloping the distal body **216,** is positioned in the blood vessel **266** so that the two proximal, unattached distal-pointing crowns **258A** and **258B** are immediately distal to the clot **270A.** See FIG. 15B. The distal body **216** is then deployed from the delivery catheter **208** by moving the catheter **208** proximally. The soft clot **270A,** which is unable to collapse the distal body **216,** then enters the distal body interior **222.** See FIG. 15C. However, at this time, the surgeon is unaware that the clot **270A** has entered into the distal body interior **222.** Thus, without moving the distal body **216,** the surgeon irradiates the four rows of x-ray markers at a first vantage point (i.e., from the front of the distal body **216** in the orientation shown in FIGs. 15A-G; i.e., into the page). As shown in FIG. 15D, the first vantage point shows four rows of x-ray markers. The first row is a single point, which represents the x-ray marker located in the proximal tube **228, 244;** the proximal tube x-ray marker **228, 244** always appears as a single point. The second row is a single point, which represents the x-ray marker located at the front, proximal, unattached distal-pointing crown **258B, 244;** the reason that this second row of markers is a single point is that the rear x-ray marker of the second row **258A, 244** is hidden from view because it is directly behind the front x-ray marker of the second row **258B, 244.** The third row has two points, which represents the two x-ray markers located at the distal, unattached distal-pointing crowns **258C, 244** and **258D, 244;** the reason that this third row of markers has two points is that neither marker in the third row **258C, 244** and **258D, 244** is hidden from view on the x-ray at this angle - rather, one marker **258C, 244** is located above the other marker **258D, 244** - and as shown in FIG. 15C, the distal body **216** is not collapsed at the distal, unattached distal-pointing crowns **258C, 244** and **258D, 244.** The fourth row is a single point, which represents the x-ray marker located in the distal tube **236, 244;** the distal tube x-ray marker **236, 244** always appears as a single point. Without moving the distal body **216, the** surgeon then irradiates the four rows of x-ray markers from a second vantage point 90 degrees offset from the first vantage point (i.e., from the bottom of the distal body **216** in the orientation shown in FIG. 15A). As shown, the first row is, as always, a single point, which represents the x-ray marker located in the proximal tube **228, 244.** The second row has two points, which represents the two x-ray markers located at the proximal, unattached distal-pointing crown **258A, 244** and **258B, 244;** the reason that this second row of markers shows up as two points is that neither marker **258A, 244** and **258B, 244** in the second row is hidden from view on the x-ray at this offset angle - rather, one marker **258B, 244** is located above the other marker **258A, 244** - and the distal body **216** is not collapsed at the proximal, unattached distal-pointing crowns **258A, 244** and **258B, 244.** The third row is a single point, which represents the x-ray marker located at the bottom, distal, unattached distal-pointing crown **258D, 244;** the reason that this third row of markers is a single point is that the top x-ray marker of the third row **258C, 244** is directly behind the bottom x-ray marker of the third row **258D, 244,** and thus, hidden from view. The fourth row is, as always, a single point, which represents the x-ray marker located in the distal tube **236, 244.** The surgeon, thus, concludes that neither the x-ray markers at the second row **258A, 244** and **258B, 244** nor the x-ray markers at the third row **258C, 244** and **258D, 244** (i.e., the x-ray markers at both the proximal and distal unattached distal pointing-crowns) have converged. As shown in FIG. 15E, the surgeon then moves the distal body **216** proximally relative to the soft clot **270A** so that the distal, unattached distal-pointing crowns **258C, 244** and **258D, 244** are immediately distal to the clot **270A** and then the surgeon irradiates the four rows of x-ray markers again from the first vantage point and the second vantage point. As shown in FIG. 15F, the results are the same as FIG. 15D. With the results from FIGs. 15D and 15F, the surgeon concludes that neither x-ray markers at the second row **258A, 244** and **258B, 244** nor the x-ray markers at the third row **258C, 244** and **258D, 244** (i.e., the x-ray markers at both the proximal and distal unattached distal pointing-crowns) converged at either the original position of the distal body **216** (FIGs. 15C and 15D) or the position after moving the distal body **216** proximally (FIGs. 15E and 15F), and, thus, the distal body **216** was expanded in the vessel **266** in both positions. Thus, the surgeon concludes that the clot is a soft clot **270A** that has entered into the distal body interior **222** and the surgeon removes the distal body **216** and the soft clot **270A,** captured by the distal body **216,** by moving the distal body **216** proximally out of the vessel **266,** as shown in FIG. 15G.

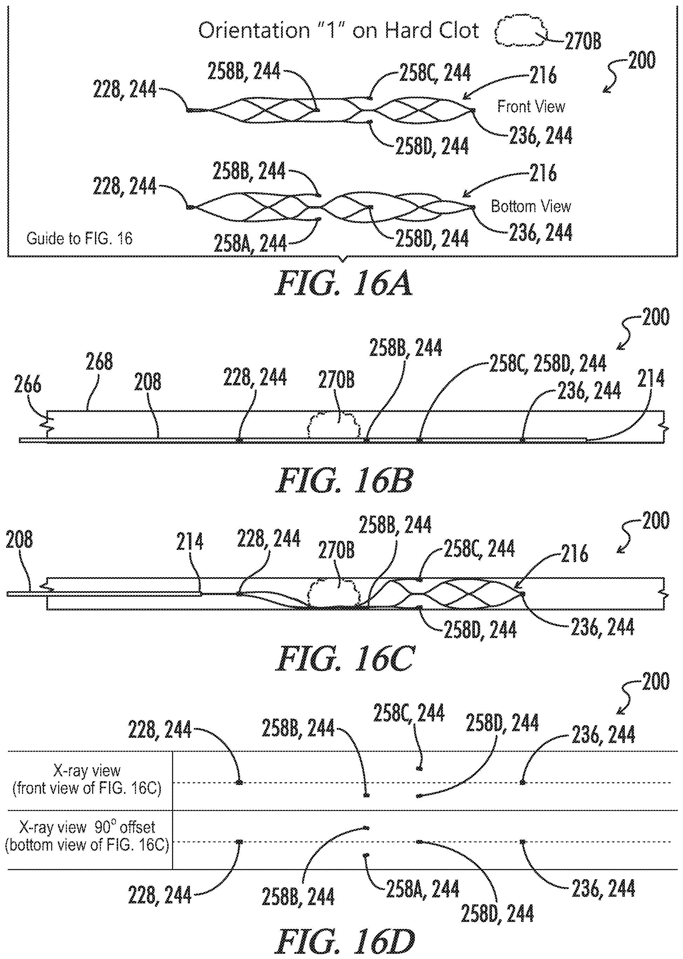

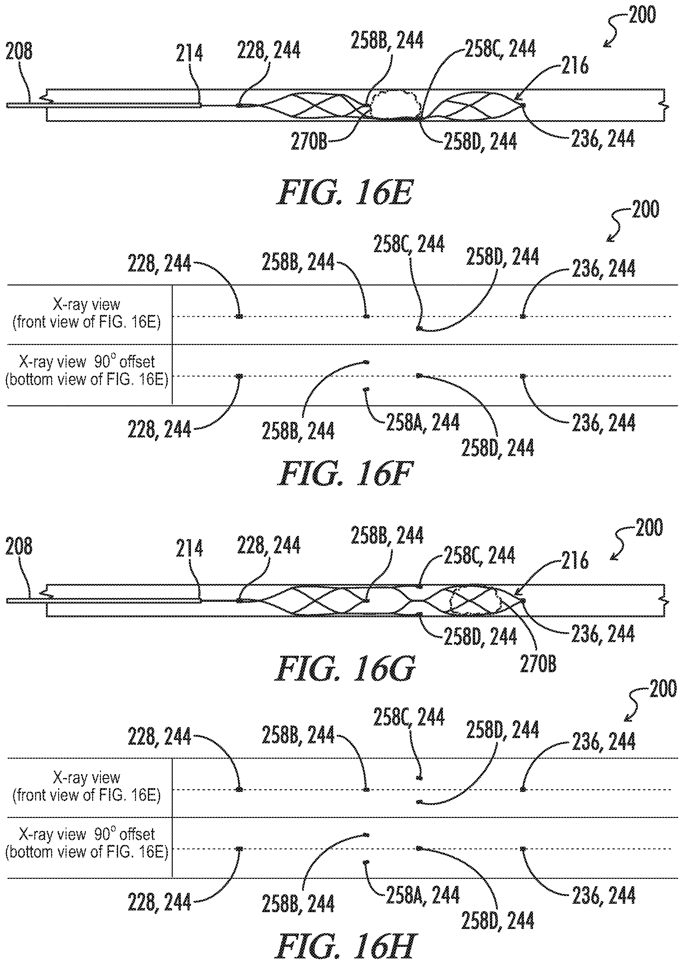

FIGs. 16A-H illustrate stepwise use of the distal body **216** in retrieving a hard clot **270B** in a human intracranial artery **266.** (In FIGs. 16A-H, the distal body **216** is in Orientation 1). First, as always, the surgeon determines the location of the clot **270B** in the vessel **266** using, for example, a contrast dye injected proximal and distal to the clot **270B.** Next, the delivery catheter **208,** which is enveloping the distal body **216,** is positioned in the blood vessel **266** so that the two proximal, unattached distal-pointing crowns **258A** and **258B** are immediately distal to the clot **270B.** See FIG. 16B. The distal body **216** is then deployed from the delivery catheter **208** by moving the catheter **208** proximally. The hard clot **270B,** which is located above the distal body **216,** collapses the distal body **216,** as shown in FIG. 16C. However, at this time, the surgeon is unaware that the clot **270B** has collapsed the distal body **216.** Thus, without moving the distal body **216,** the surgeon irradiates the x-ray markers at a first vantage point (i.e., from the front of the distal body **216;** i.e., into the page). As shown in FIG. 16D, the first vantage point shows four rows of x-ray markers. The first row is, as always, a single point, representing the x-ray marker located in the proximal tube - i.e., **228, 244.** The second row is a single point, which represents the x-ray marker located at the front, proximal, unattached distal-pointing crown **258B, 244;** the reason that this second row of markers is a single point is that the rear x-ray marker of the second row **258A, 244** is hidden from view because it is directly behind the front x-ray marker of the second row **258B, 244.** The third row has two points, which represents the two x-ray markers located at the distal, unattached distal-pointing crowns **258C, 244** and **258D, 244;** the reason that this third row of markers has two points is that neither marker in the third row is hidden from view on the x-ray at this angle - rather, one marker **258C, 244** is located above the other marker **258D, 244** - and as shown in FIG. 16C, the distal body **216** is not collapsed at the distal, unattached distal-pointing crowns **258C, 244** and **258D, 244.** The fourth row is, as always, a single point, representing the x-ray marker located in the distal tube **236, 244.** Without moving the distal body **216,** the surgeon then irradiates the markers from a second vantage point 90 degrees offset from the first vantage point (i.e., from the bottom of the distal body **216**). As shown, the first row is, as always, a single point, which represents the x-ray marker located in the proximal tube **228, 244.** The second row has two points, which represents the two x-ray markers located at the proximal, unattached distal-pointing crowns **258A, 244** and **258B, 244;** the reason that this second row of markers shows up as two points is that neither marker in the second row is hidden from view on the x-ray at this offset angle - rather, one marker **258B, 244** is located above the other marker **258A, 244** - and although the distal body **216** is collapsed at the proximal, unattached distal-pointing crowns as shown in FIG. 16C, the second row of x-ray markers have not converged because the clot **270B** is on top of the second row of x-ray markers. The third row is a single point, which represents the x-ray marker located at the bottom, distal, unattached distal-pointing crown **258D, 244;** the reason that this third row of markers is a single point is that the top x-ray marker of the third row **258C, 244** is directly behind the bottom x-ray marker of the third row **258D, 244,** and thus, hidden from view. The fourth row is, as always, a single point, which represents the x-ray marker located in the distal tube **236, 244.** The surgeon, thus, concludes that neither the second row **258A, 244** and **258B, 244** nor the third row **258C, 244** and **258D, 244** of x-ray markers (i.e., the x-ray markers at both the proximal and distal unattached distal pointing-crowns) has converged. As shown in FIG. 16E, the surgeon then moves the distal body 216 proximally so that the distal, unattached distal-pointing crowns **258C, 244** and **258D, 244** are immediately distal to the clot **270B** and the surgeon then irradiates the x-markers again from the first vantage point. As shown in FIG. 16F, the first row is, as always, a single point, representing the x-ray marker located in the proximal tube **228, 244.** The second row is a single point, which represents the x-ray marker located at the front, proximal, unattached distal-pointing crown **258B, 244;** the reason that this second row of markers is a single point is that the rear x-ray marker of the second row **258A, 244** is hidden from view because it is directly behind the front x-ray marker of the second row **258B, 244.** The third row has only one point because the clot **270B,** which is on top of the third row of x-ray markers **258C, 244** and **258D, 244** (i.e., the markers at the distal, unattached distal-pointing crowns), has pushed the third row of x-ray markers **258C, 244** and **258D, 244** together. The fourth row is, as always, a single point, representing the x-ray marker located in the distal tube **236, 244.** Without moving the distal body **216,** the surgeon then irradiates the markers from a second vantage point 90 degrees offset from the first vantage point (i.e., from the bottom of the distal body). As shown, the first row is, as always, a single point, which represents the x-ray marker located in the proximal tube **228, 244.** The second row has two points, which represents the two x-ray markers located at the proximal, unattached distal-pointing crown **258A, 244** and **258B, 244;** the reason that this second row of markers shows up as two points is that neither marker in the second row is hidden from view on the x-ray at this offset angle and the distal body **216** is not collapsed at the proximal, unattached distal-pointing crowns **258A, 244** and **258B, 244.** The third row is a single point, which represents the x-ray marker located at the bottom, distal, unattached distal-pointing crown **258D, 244;** the reason that this third row of markers is a single point is that the bottom x-ray marker of the third row **258D, 244** is directly in front of the top x-ray marker of the third row **258C, 244,** and thus, the top x-ray marker of the third row **258C, 244** is hidden from view. The fourth row is, as always, a single point, which represents the x-ray marker located in the distal tube **236, 244.** Knowing that the distal, unattached distal-pointing crowns **258C, 244** and **258D, 244** have converged as shown in FIG. 16F, the surgeon moves the distal body **216** proximally and the hard clot **270B** falls into the distal body interior **222** in the enlarged cell/drop zone **262C** immediately distal to the top, distal, unattached distal-pointing crown **258C.** See FIG. 16G. To confirm that the hard clot **270B** has entered the distal body interior **222,** the surgeon takes x-rays from the first and second vantage points. The results are shown in FIG. 16H. As compared to 16F, the front x-ray view of FIG. 16H shows that the distal, unattached distal-pointing crowns **258C, 244** and **258D, 244** are not converged, and, thus, the surgeon concludes that the hard clot **270B** has entered the distal body interior **222.** The surgeon then removes the distal body **216** and the hard clot **270B,** captured by the distal body **216,** by moving the distal body **216** proximally out of the vessel **266.**

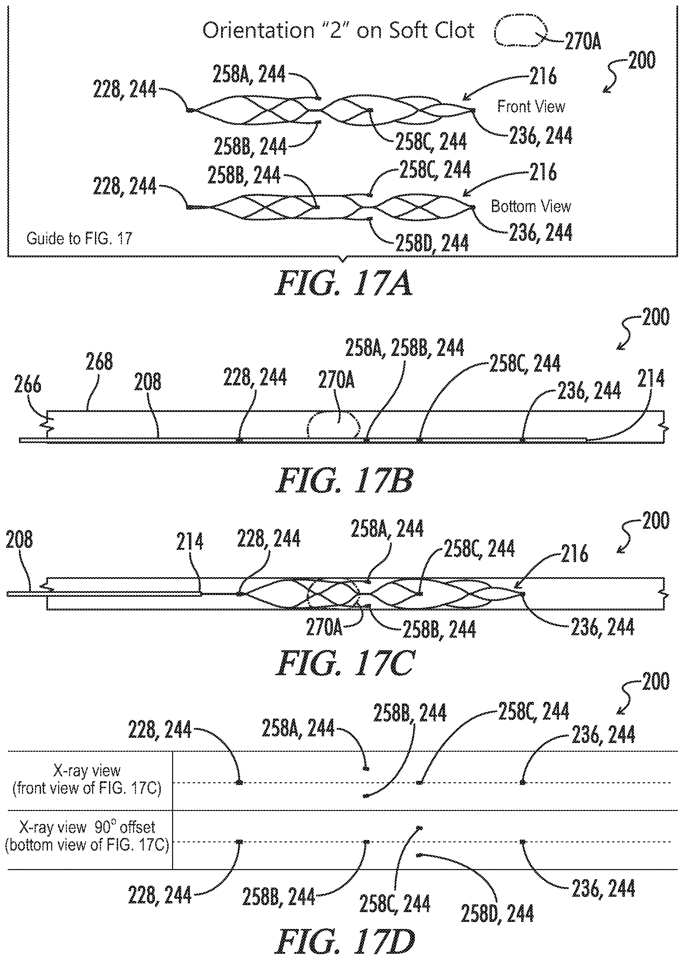

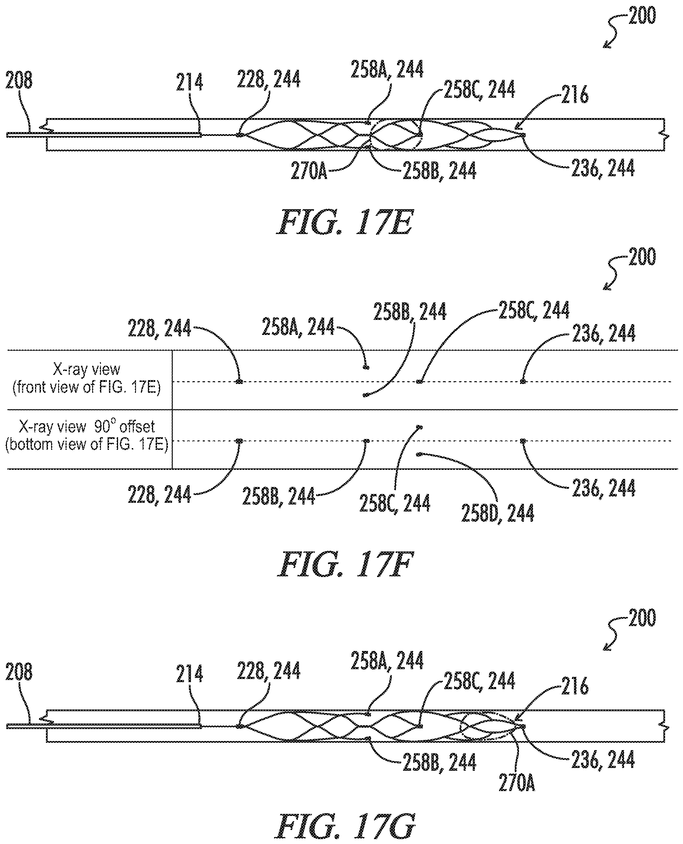

FIGs. 17A-G illustrate stepwise use of the distal body **216** in retrieving a soft clot **270A** in a human intracranial artery **266.** (In FIGs. 17A-G, the distal body **216** is in Orientation 2). First, as always, the surgeon determines the location of the clot **270A** in the vessel **266** using, for example, a contrast dye injected proximal and distal to the clot **270A.** Next, the delivery catheter **208,** which is enveloping the distal body **216,** is positioned in the blood vessel **266** so that the two proximal, unattached distal-pointing crowns **258A** and **258B** are immediately distal to the clot **270A.** See FIG. 17B. The distal body **216** is then deployed from the catheter **208** by moving the catheter **208** proximally. The soft clot **270A,** which is unable to collapse the distal body **216,** then enters the distal body interior **222.** See FIG. 17C. However, at this time, the surgeon is unaware that the clot **270A** has entered into the distal body interior 222. Thus, without moving the distal body **216,** the surgeon irradiates the x-ray markers at a first vantage point (i.e., from the front of the distal body; into the page). As shown in FIG. 17D, the first vantage point shows four rows of x-ray markers. The first row is, as always, a single point, representing the x-ray marker located in the proximal tube **228, 244.** The second row has two points, which represents the two x-ray markers located at the proximal, unattached distal-pointing crowns **258A, 244** and **258B, 244;** the reason that this second row of markers has two points is that neither marker in the second row is hidden from view on the x-ray at this angle - rather, one marker **258A, 244** is located above the other marker **258B, 244 -** and as shown in FIG. 17C, the distal body **216** is not collapsed at the proximal, unattached distal-pointing crowns **258A, 244** and **258B, 244.** The third row has a single point, which represents the x-ray marker located at the front (in Orientation 2), distal, unattached distal-pointing crown **258C, 244;** the reason that this third row of markers is a single point is that the rear (in Orientation 2) x-ray marker **258D, 244** of the third row is hidden from view because it is directly behind the front x-ray marker **258C, 244** of the third row. The fourth row is, as always, a single point, representing the x-ray marker located in the distal tube **236, 244.** Without moving the distal body, the surgeon then irradiates the markers from a second vantage point 90 degrees offset from the first vantage point (i.e., from the bottom of the distal body, as shown in this view). As shown, the first row is, as always, a single point, which represents the x-ray marker located in the proximal tube **228, 244.** The second row is a single point, which represents the x-ray marker located at the bottom (in Orientation 2), proximal, unattached distal-pointing crown **258B, 244;** the reason that this second row of markers is a single point is that the top (in Orientation 2) x-ray marker of the second row **258A, 244** is directly behind the bottom x-ray marker of the second row **258B, 244,** and thus, hidden from view. The third row has two points, which represents the two x-ray markers located at the distal, unattached distal-pointing crowns **258C, 244** and **258D, 244;** the reason that this third row of markers shows up as two points is that neither marker in the third row is hidden from view on the x-ray at this offset angle and the distal body **216** is not collapsed at the distal, unattached distal-pointing crowns **258C, 244** and **258D, 244.** The fourth row is, as always, a single point, which represents the x-ray marker located in the distal tube **236, 244.** The surgeon, thus, concludes that neither the second row **258A, 244** and **258B, 244** nor the third row of x-ray markers **258C, 244** and **258D, 244** (i.e., the x-ray markers at both the proximal and distal unattached distal pointing-crowns) has converged. As shown in FIG. 17E, the surgeon then moves the distal body **216** proximally relative to the clot **270A** so that the distal, unattached distal-pointing crowns **258C, 244** and **258D, 244** are immediately distal to the clot **270A** and then the surgeon irradiates the x-markers again from the first vantage point and the second vantage point. As shown in FIG. 17F, the results are the same as FIG. 17D. With the results from FIGs. 17D and 17F, the surgeon concludes that neither the second row **258A, 244** and **258B, 244** nor the third row of x-ray markers **258C, 244** and **258D, 244** (i.e., the x-ray markers at both the proximal and distal unattached distal pointing-crowns) were converged at either the original position of the distal body **216** (FIG. 17C and 17D) or the position after moving the distal body **216** proximally (FIG. 17E and 17F), and, thus, the distal body **216** was expanded in the vessel **266** in both positions. Thus, the surgeon concludes that the clot **270A** is a soft clot **270A** that has entered into the distal body interior **222** and the surgeon removes the distal body **216** and the soft clot **270A,** captured by the distal body **216,** by moving the distal body **216** proximally out of the vessel **266,** as shown in FIG. 17G.

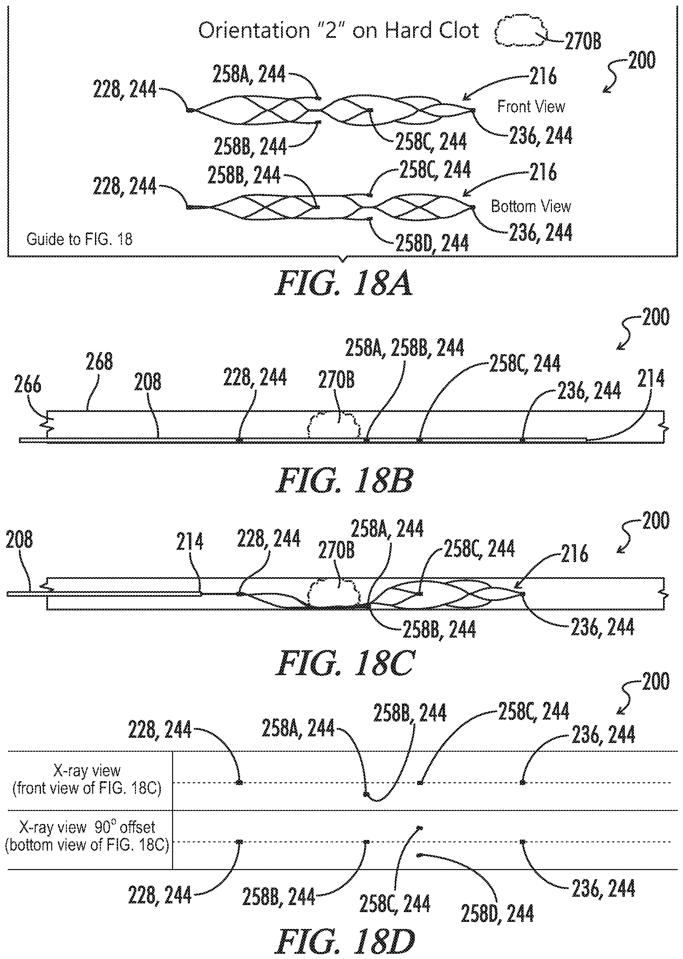

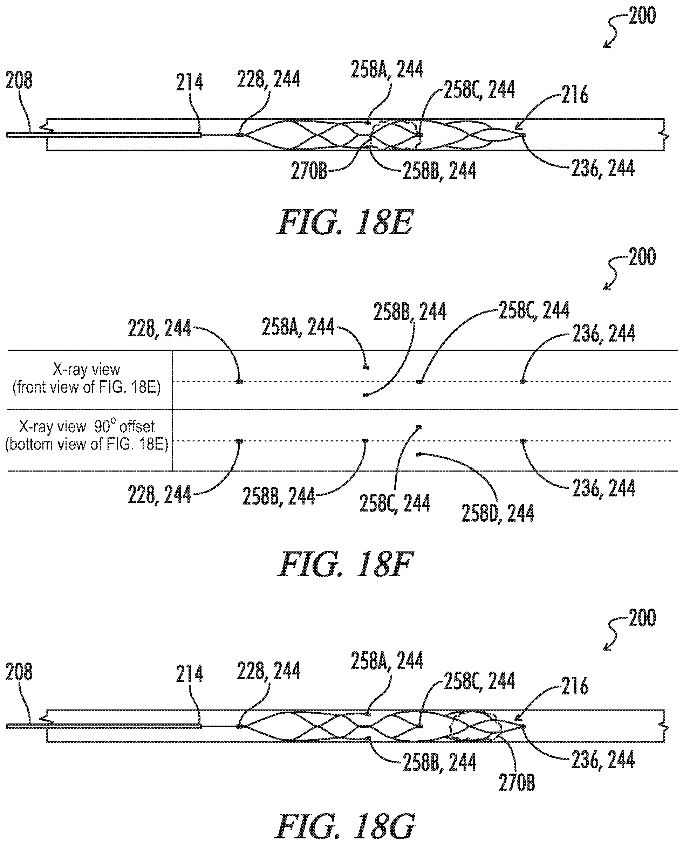

FIGs. 18A-G illustrate stepwise use of the distal body **216** in retrieving a hard clot **270B** in a human intracranial artery **266.** (In FIGS. 18A-G, the distal body **216** is in Orientation 2). (As described below, the primary differences between FIGs 18A-G and FIGs. 16A-G is that the clot **270B** enters the distal body interior **222** in an enlarged cell/drop zone **262A** immediately distal to one of the proximal, unattached distal-pointing crowns **258A** in FIGs. 18A-G, as compared to FIGs. 16A-G where the clot 270B enters the distal body interior **222** in an enlarged cell/drop zone **262C** immediately distal to one of the distal, unattached distal-pointing crowns **258C**). First, as always, the surgeon determines the location of the clot **270B** in the vessel 266 using, for example, a contrast dye injected proximal and distal to the clot **270B.** Next, the delivery catheter **208,** which is enveloping the distal body **216,** is positioned in the blood vessel **266** so that the two proximal, unattached distal-pointing crowns **258A** and **258B** are immediately distal to the clot **270B.** See FIG. 18B. The distal body **216** is then deployed from the catheter **208** by moving the catheter **208** proximally. The hard clot **270B,** which is located above the distal body **216,** collapses the distal body **216,** as shown in FIG. 18C. However, at this time, the surgeon is unaware that the clot **270B** has collapsed the distal body **216.** Thus, without moving the distal body **216,** the surgeon irradiates the x-ray markers at a first vantage point (i.e., from the front of the distal body in Orientation 2; into the page). As shown in FIG. 18D, the first vantage point shows four rows of x-ray markers. The first row is, as always, a single point, representing the x-ray marker located in the proximal tube **228, 244.** The second row has only one point because the clot **270B,** which is on top of the second row of x-ray markers **258A, 244** and **258B, 244** (i.e., the markers at the proximal, unattached distal-pointing crowns), has pushed them together. The third row has only one point, which represents the x-ray marker located at the front (in Orientation 2), proximal, unattached distal-pointing crown **258C, 244;** the reason that this third row of markers is a single point is that the rear (in this view) x-ray marker of the third row **258D, 244** is hidden from view because it is directly behind the front x-ray marker of the third row **258C, 244.** The fourth row is, as always, a single point, representing the x-ray marker located in the distal tube **236, 244.** Without moving the distal body, the surgeon then irradiates the markers from a second vantage point 90 degrees offset from the first vantage point (i.e., from the bottom of the distal body **216**)**.** As shown, the first row is, as always, a single point, which represents the x-ray marker located in the proximal tube **228, 244.** The second row has a single point because the top (in Orientation 2) x-ray marker of the second row **258A, 244** is located behind the bottom (in Orientation 2) x-ray marker **258B, 244** and thus, the top x-ray marker of the second row **258A, 244** is hidden from view. The third row has two points, which represents the x-ray markers located at the distal, unattached distal-pointing crowns **258C, 244** and **258D, 244;** in this x-ray view neither of the x-ray markers of the third row is hidden from view. The fourth row is, as always, a single point, which represents the x-ray marker located in the distal tube **236, 244.** The surgeon, thus, concludes that the second row of x-ray markers **258A, 244** and **258B, 244** (i.e., the x-ray markers at the proximal, unattached distal pointing-crowns) has converged. As shown in FIG. 18E, the surgeon then moves the distal body **216** proximally so that the distal, unattached distal-pointing crowns **258C, 244** and **258D, 244** are immediately distal to the clot **270B.** Unbeknownst to the surgeon, the clot **270B** enters the distal body interior **222** immediately distal to the top (in Orientation 2), proximal unattached distal-pointing crown **258A** and the distal body **216** is no longer collapsed. The surgeon then irradiates the x-markers again from the first vantage point. As shown in FIG. 18F, the first row is, as always, a single point, representing the x-ray marker located in the proximal tube **228, 244.** The second row has two x-ray markers because the distal body **216** is not collapsed and neither the top (in Orientation 2) **258A, 244** nor the bottom **258B, 244** (in Orientation 2) x-ray marker of the second row (i.e., the marker at the proximal, unattached distal-pointing crowns) is hidden from view. The third row has only one point because the rear (in Orientation 2), distal unattached distal-pointing crown **258D, 244** is hidden behind the front (in Orientation 2), distal, unattached distal pointing-crown **258C, 244.** The fourth row is, as always, a single point, representing the x-ray marker located in the distal tube **236, 244.** Without moving the distal body **216,** the surgeon then irradiates the markers from a second vantage point 90 degrees offset from the first vantage point (i.e., from the bottom of the distal body **216**). As shown, the first row is, as always, a single point, which represents the x-ray marker located in the proximal tube **228, 244.** The second row has a single point because the x-ray marker at the top (in Orientation 2), proximal, unattached distal-pointing crown **258A, 244** is hidden behind the bottom (in Orientation 2), proximal, unattached-distal pointing crown **258B, 244.** The third row has two points because neither the front nor the rear x-ray markers at the distal, unattached, distal-pointing crowns **258C, 244** and **258D, 244** is hidden from view. The fourth row is, as always, a single point, which represents the x-ray marker located in the distal tube **236, 244.** Based on the information from FIGs. 18D and 18F, the surgeon concludes that the clot **270B** has entered into the distal body interior **222.** The surgeon then removes the distal body **216** and the hard clot **270B,** captured by the distal body **216,** by moving the distal body **216** proximally out of the vessel **266,** as shown in FIG. 18G. Upon comparing FIGs. 16A-G and FIGs. 18A-G it will be appreciated that the orientation of the enlarged cells/drop zone **262A-D** relative to the orientation of a hard clot **270B** determine which enlarged cell/drop zone **262A, 262B, 262C,** or **262D,** the hard clot **270** enters the distal body interior **222** through. For example, in FIG. 16C, the hard clot **270B** is located above the distal body **216,** and thus, the hard clot **270B** must enter through the enlarged cell/drop zone located at the top of the distal body, which in the orientation of the distal body shown in FIGs. 16A-G, is the enlarged cell/drop zone **262C** immediately distal to the top, distal, unattached, distal-pointing crown **258C.** In FIG. 18C, the hard clot **270B** is again located above the distal body and, thus, the hard clot **270B** must enter through the enlarged cell/drop zone located at the top of the distal body. However, in FIG. 18C, the enlarged cell/drop zone located at the top of the distal body **216,** in the orientation of the distal body **216** shown in FIGs. 18A-G, is the enlarged cell/drop zone **262A** immediately distal to the top, proximal, unattached, distal-pointing crown **258A.**

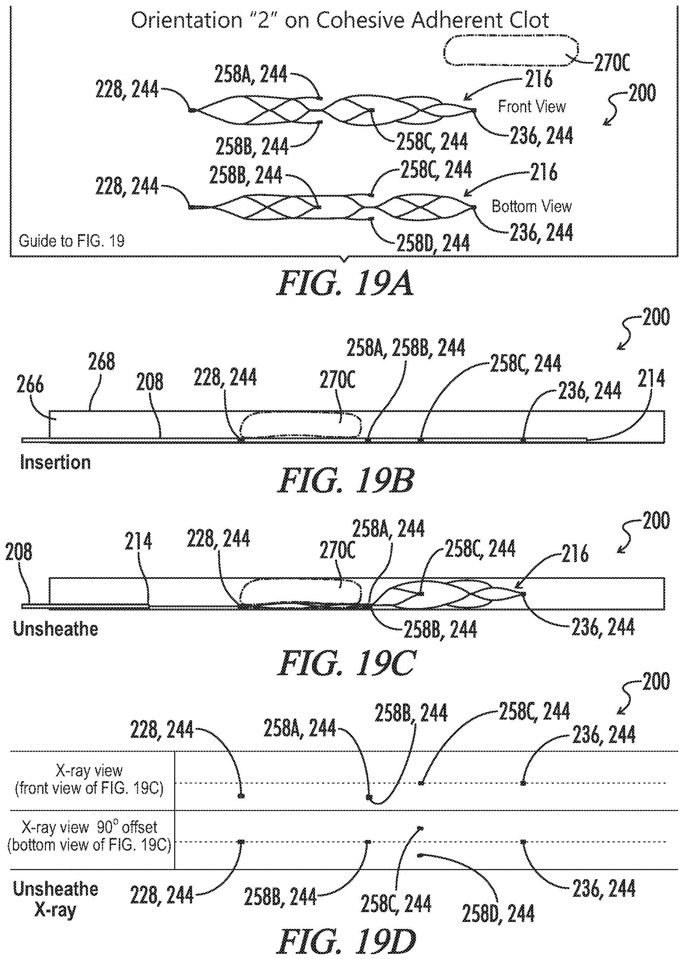

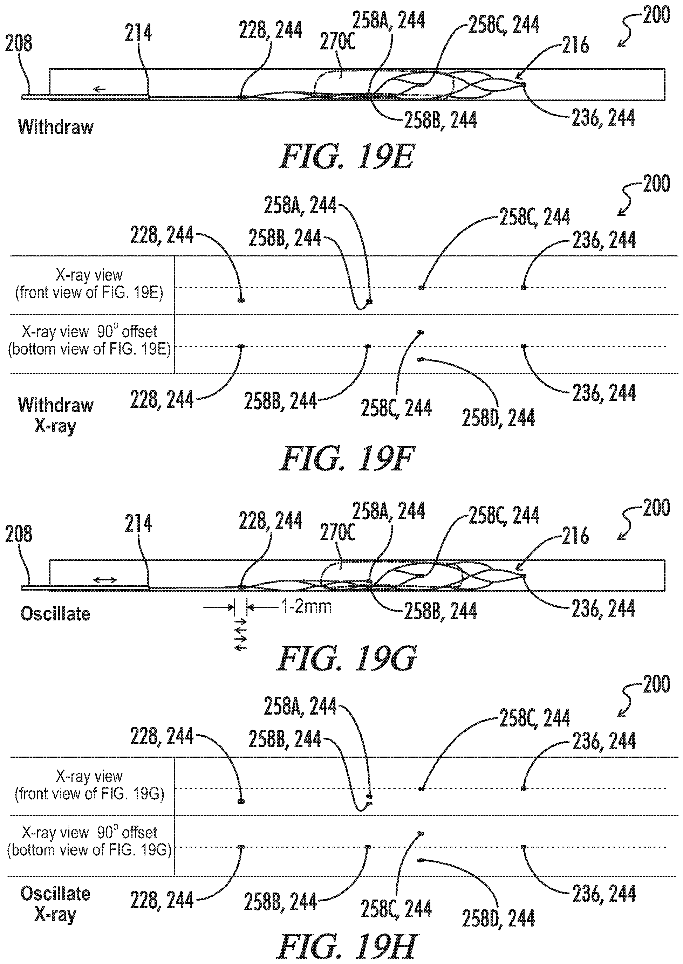

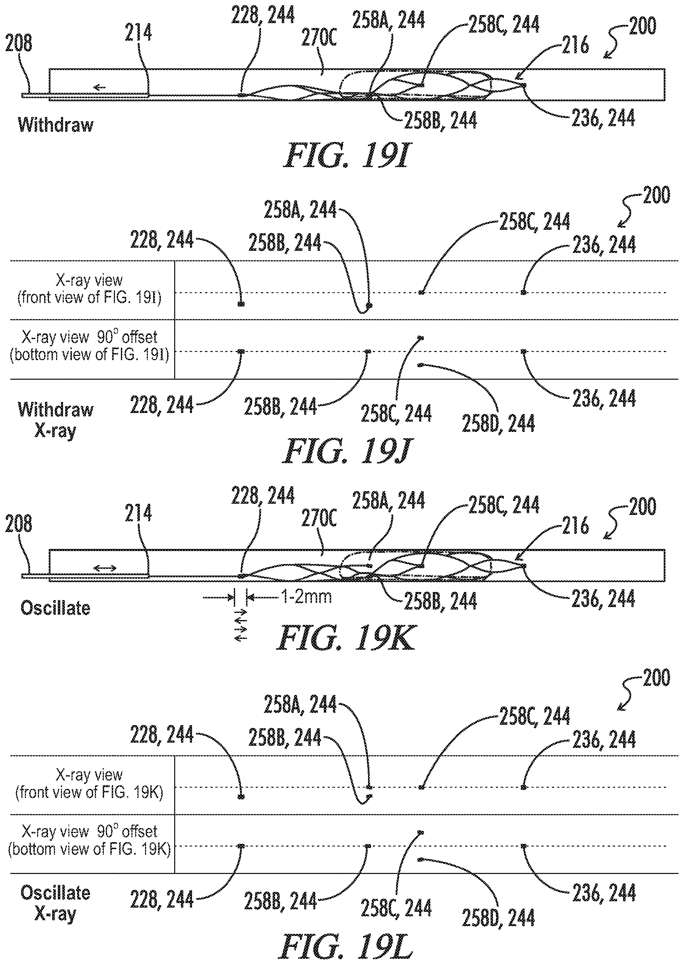

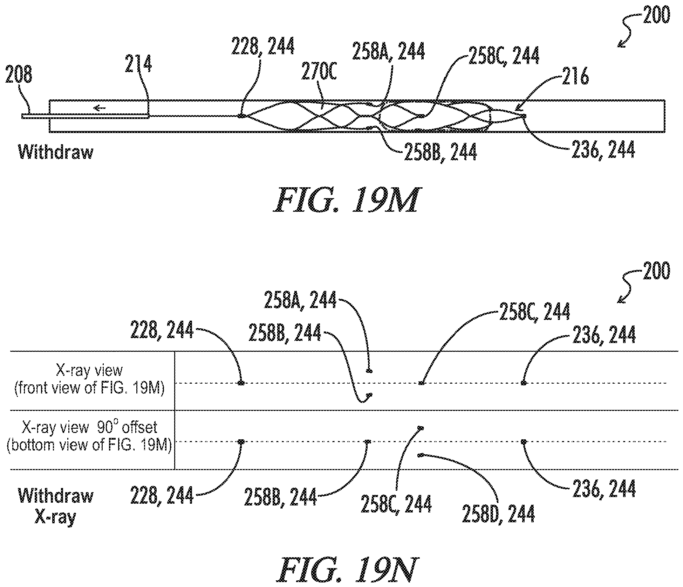

FIGs. 19A-N illustrate stepwise use of the distal body **216** in retrieving a deformable cohesive, adherent clot **270C-** i.e., a clot that is difficult to break up and is tightly adhered to the vessel wall **268** - in a human intracranial artery 266. (In FIGS. 19A-N, the distal body **216** is in Orientation 2). First, as always, the surgeon determines the location of the clot **270C** in the vessel **266** using, for example, a contrast dye injected proximal and distal to the clot **270C.** Next, the delivery catheter **208,** which is enveloping the distal body **216,** is positioned in the blood vessel **266** so that the two proximal, unattached distal-pointing crowns **258A** and **258B** are immediately distal to the clot **270C.** See FIG. 19B. The distal body **216** is then deployed from the catheter **208** by moving the catheter **208** proximally. The deformable, cohesive adherent clot **270C,** which is located above the distal body **216,** collapses the distal body **216,** as shown in FIG. 19C. However, at this time, the surgeon is unaware that the clot **270C** has collapsed the distal body **216.** Thus, without moving the distal body **216,** the surgeon irradiates the x-ray markers at a first vantage point (i.e., from the front of the distal body; i.e., into the page). As shown in FIG. 19D, the first vantage point shows four rows of x-ray markers. The first row is, as always, a single point, representing the x-ray marker located in the proximal tube **228, 244.** The second row has a single point, corresponding to the top (in Orientation 2) and bottom (in Orientation 2), proximal, unattached distal-pointing crowns **258A, 244** and **258B, 244,** which have converged because the clot **270C** is collapsing the distal body **216.** The third row has a single point, which represents the x-ray marker located at the front (in Orientation 2), distal, unattached distal-pointing crown **258C, 244;** the x-ray marker located at the rear, distal, unattached distal-pointing crown **258D, 244** is hidden from view. The fourth row is, as always, a single point, representing the x-ray marker located in the distal tube **236, 244.** Without moving the distal body **216,** the surgeon then irradiates the markers from a second vantage point 90 degrees offset from the first vantage point (i.e., from the bottom of the distal body). As shown, the first row is, as always, a single point, which represents the x-ray marker located in the proximal tube **228, 244.** The second row has a single point, which corresponds to the bottom (in Orientation 2), proximal, unattached distal-pointing crown **258B, 244;** the top (in Orientation 2), proximal, unattached distal-pointing crown **258A, 244** is located behind the bottom, proximal, unattached distal-pointing crown **258B, 244** and hidden from view. The third row has two points, which correspond to the front (in Orientation 2) **258C, 244** and rear **258D, 244** (in Orientation 2), distal, unattached distal-pointing crowns, neither of which is blocked in this view. The fourth row is, as always, a single point, which represents the x-ray marker located in the distal tube **236, 244.** As shown in FIG. 19E, the surgeon then moves the distal body **216** proximally (i.e., slightly withdraws the distal body **216**). The surgeon then irradiates the x-markers again from the first and second vantage points. As shown in FIG. 19F, the results are exactly the same as in FIG. 19D. Based on the observation that the proximal, unattached distal-pointing crowns **258A, 244** and **258B, 244** have converged at both the original position (FIGs. 19C and 19D in which the proximal, unattached distal-pointing crowns **258A, 244** and **258B, 244** are immediately distal to the clot **270C)** and the second position (FIGs. 19E and 19F), the surgeon concludes that the clot **270C** is a deformable cohesive, adherent clot **270C.** The surgeon then oscillates the distal body **216** proximally and distally a small distance (e.g., about 1mm to about 2 mm) in the vessel **266,** and the clot **270C** begins to enter the distal body 216, as shown in FIG. 19G. The surgeon then irradiates the x-markers again from the first and second vantage points. As shown in FIG. 19H, the results are exactly the same as in FIG. 19D and FIG. 19F except that the second row of markers **258A, 244** and **258B, 244** (at the proximal, unattached distal-pointing crowns) are beginning to move apart. The surgeon then moves the distal body **216** proximally again, as shown in FIG. 19I. The surgeon then irradiates the x-markers again from the first and second vantage points. As shown in FIG. 19J, the results are exactly the same as in FIGs. 19D and 19F, as the clot **270C** has caused the second row of markers **258A, 244** and **258B, 244** to re-converge. The surgeon then oscillates the distal body **216** proximally and distally a small distance (e.g., about 1mm to about 2 mm) in the vessel **266,** and the clot **270C** begins to further enter the distal body interior **222,** as shown in FIG. 19K. The surgeon then irradiates the x-markers again from the first and second vantage points. As shown in FIG. 19L, the results are the same as in FIG. 19H. The surgeon then moves the distal body **216** again proximally, and, instead of collapsing the second row of markers **258A, 244** and **258B, 244,** the clot **270C** fully enters the distal body interior **222,** as shown in FIG. 19M. The surgeon then irradiates the x-markers again from the first and second vantage points. As shown in FIG. 19N, the results show that the second row of markers **258A, 244** and **258B, 244** (at the proximal, unattached distal-pointing crowns) have moved apart. Satisfied that the x-ray markers in the second row **258A, 244** and **258B, 244** (at the proximal, unattached distal-pointing crowns) are sufficiently far apart and that the x-ray markers in the third row (at the distal, unattached distal-pointing crowns) **258C, 244** and **258D, 244** have stayed far apart, the surgeon concludes that the deformable cohesive, adherent clot **270C** has been sufficiently captured by the distal body **216** and the surgeon then removes the distal body **216** and the clot **270C,** captured by the distal body **216,** by moving the distal body **216** proximally out of the vessel **266.**

Several observations can be made from FIGs. 15-19, as indicated above. For example, the x-ray markers at the proximal and distal, unattached distal-pointing crowns **258A-D, 244** provide the surgeon feedback concerning the interaction between the distal body **216** and the clot **270** in the blood vessel **266.** In addition, the guiding principle of a soft clot **270A** is that the soft clot **270A** does not collapse the distal body **216,** and thus, x-ray markers at the proximal and distal, unattached distal-pointing crowns **258A-D, 244** always appear as two points except when a marker is hidden behind another marker (due to the view). When it comes to a hard clot **270B,** the hard clot **270B** is generally able to enter the distal body interior **222** without needing to oscillate the distal body **216** proximally and distally (unlike a deformable cohesive, adherent clot **270C).** However, to capture the hard clot **270B,** the hard clot **270B** must be oriented properly relative to the enlarged cell/drop zones **262A, 262B, 262C,** or **262D.** (This is the reason that the distal body **216** has four enlarged cells/drop zones: one enlarged cells/drop zone at 0 degrees **262B,** one enlarged cells/drop zone at 90 degrees **262C,** one enlarged cells/drop zone at 180 degrees **262A** and one enlarged cells/drop zone at 270 degrees **262D).** As a guiding principle, an enlarged cell/drop zone **262A, 262B, 262C,** or **262D** is properly oriented to the clot **270B** when the x-ray markers at the proximal, unattached distal-pointing crowns **258A, 244** and **258B, 244** or the distal, unattached distal pointing crowns **258C, 244** and **258D, 244** are together at both a first x-ray view and a second x-ray view 90 degrees relative to the first x-ray view, and the hard clot **270B** can enter the enlarged cell/drop zone **262A, 262B, 262C,** or **262D** by moving the distal body **216** proximally. See FIG. 16F and 18D. Finally, the guiding principal of retrieval of deformable cohesive, adherent clots **270C** is that oscillation of the distal body **216** causes the deformable cohesive, adherent clots **270C** to gradually enter the distal basket interior **222** over time.

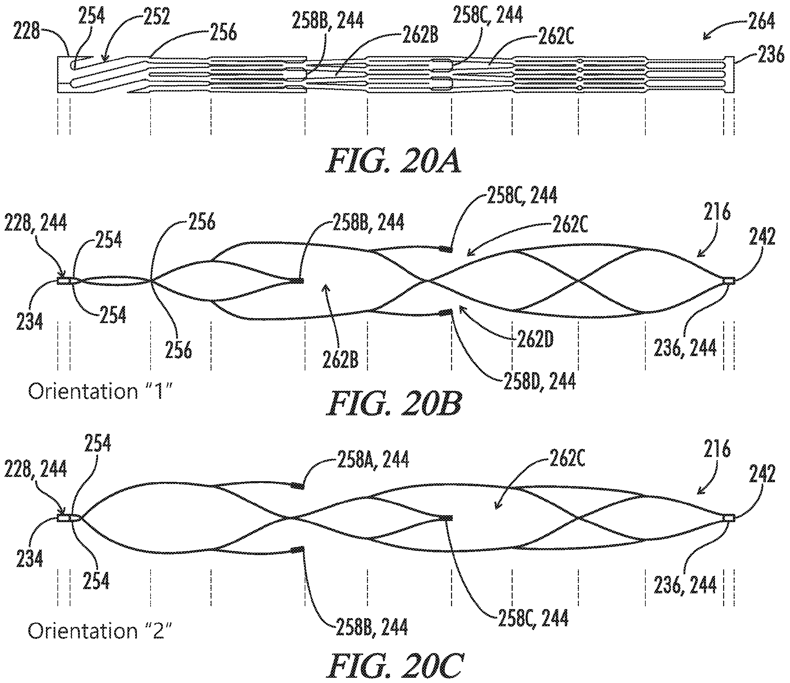

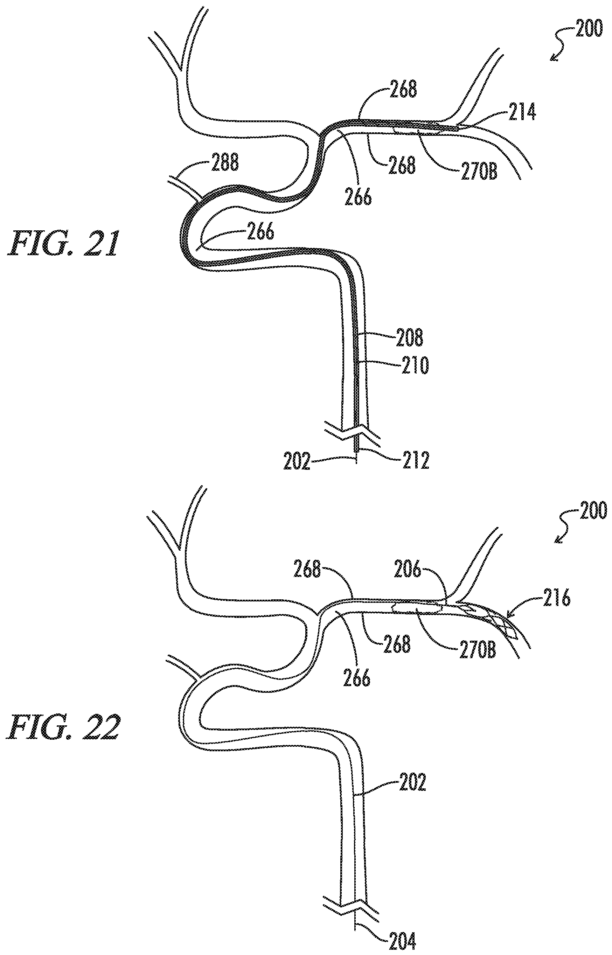

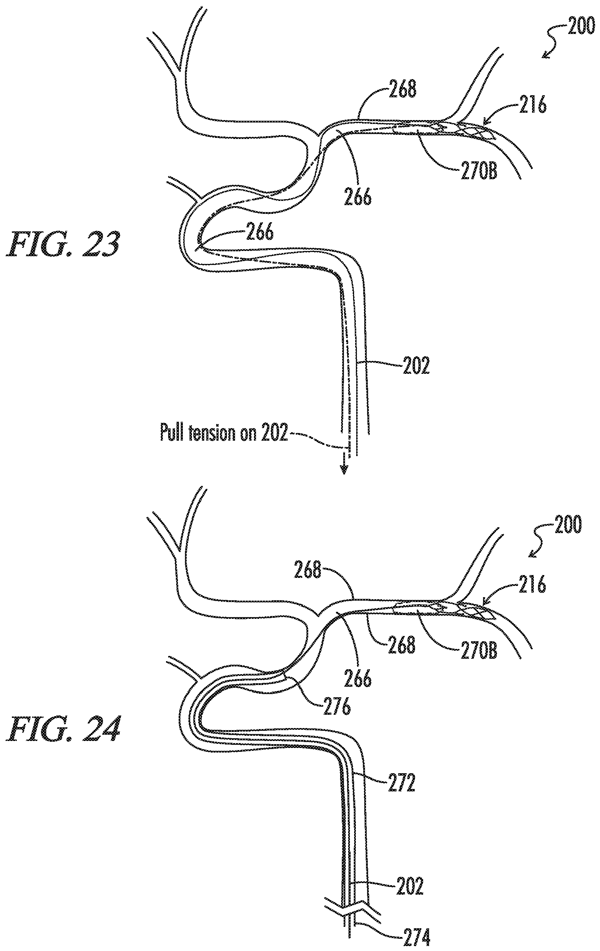

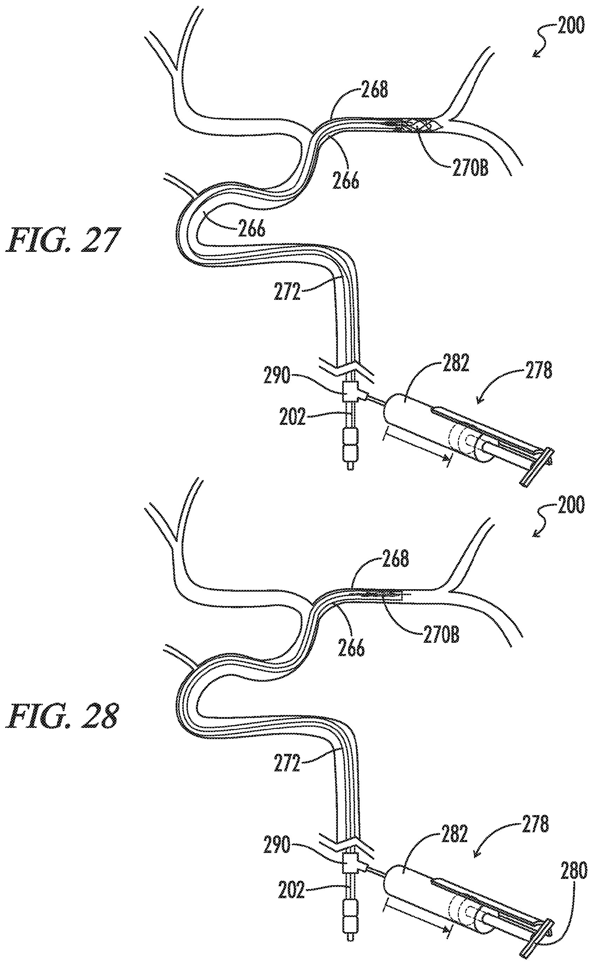

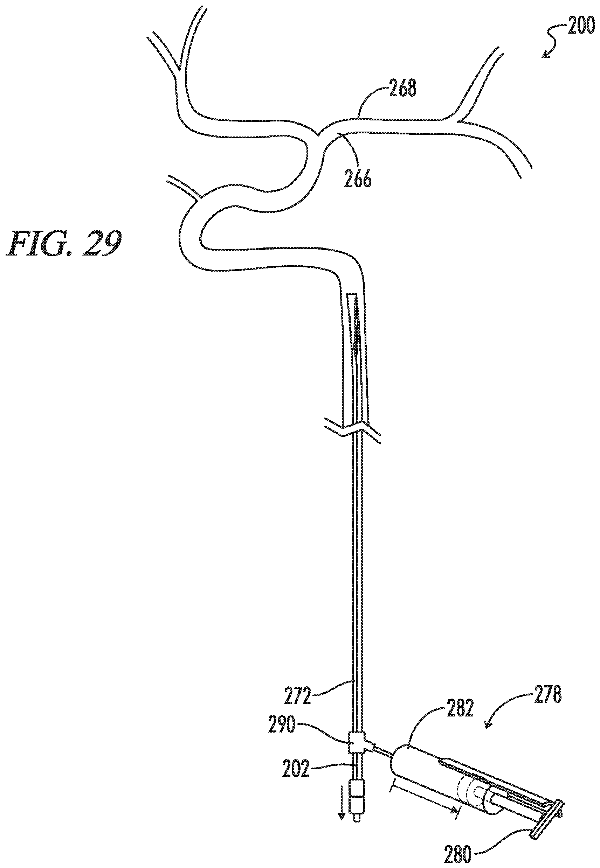

FIGs. 20A, 20B and 20C show a distal body **216** that is similar to the distal body **216** of FIGs. 14A, 14B and 14C except that the distal body **216** of FIGs. 20A, 20B and 20C is slightly shorter and its unattached, distal-pointing crowns **258A, 258B, 258C,** and **258D** are closer to the proximal tube **228.** The shortened distal body **216** of FIGs. 20A, 20B and 20C is particularly adapted for tortuous blood vessels **266.** FIG. 21-29 show stepwise deployment of the distal body **216** of FIGs. 20A, 20B and 20C in use with a manual (i.e., hand-operated), volume-dependent (i.e. volume locked) suction catheter **272** that is locked at between about 10 to about 60 cubic centimeters (cc). Optionally, the suction catheter **272** has an outer diameter of between about 0.05 inches and about 0.09 inches and its outer diameter is substantially larger than the outer diameter of the delivery catheter **208.** The clot **270** is located in the vessel **266** through the use of, for example, contrast dye injected proximal and distal to the clot **270.** As shown in FIG. 21, a delivery catheter **208** containing the distal body **216** of FIGs. 20A, 20B and 20C is positioned in the tortuous vessel **266** distal to the clot **270.** The delivery catheter **208** is withdrawn, deploying the distal body **216.** See FIG. 22. The distal body **216** is moved proximally relative to the clot **270** and tension is exerted on pull wire **202.** See FIG. 23. While maintaining tension on the pull wire **202,** a suction catheter **272** having a proximal end **274** and a distal end **276** is delivered over the pull wire **202** that is attached to the distal body **216.** See FIG. 24. (The reason for exerting tension on the pull wire **202** is that the pull wire **202** serves as the guide/track for the movement of the suction catheter **272** and without tension, the suction catheter **272** and pull wire **202** could end up in the ophthalmic artery **288**). The distal end **276** of the suction catheter **272** is positioned against the clot **270.** A syringe **278** is attached to the suction catheter **272** using a rotating hemostatic valve **290,** which allows the surgeon to aspirate while a pull wire **202** is in the system. The surgeon aspirates the syringe **278** by pulling back on the lever **280** to a mark on the base **282** corresponding to between about 10 and about 60 cubic centimeters of fluid. The surgeon then locks the lever **280** (and attached plunger) into place, leaving the suction catheter **272** under suction. The surgeon captures the clot **270** in the distal body **216** using the techniques described in FIGs. 15-19. The distal body **216** and clot **270** become captured by the suction catheter **272.** See FIGs. 27 and 28. The surgeon then removes the suction catheter **272** and the distal body **216** and the clot **270,** captured by the suction catheter **272,** by moving the suction catheter **272** proximally out of the vessel **266.** See FIG. 29. It is believed that the suction catheter **272** would be helpful in the event that a small portion of the clot **270** breaks off when retrieving the clot **270** using the distal body **216.**

To examine effectiveness of the systems **200,** the systems **200** of FIGs. 11-20, without the use of a suction catheter **272,** were used to retrieve soft and hard clots **270A** and **270B** induced in a pig weighing between 30 to 50 kg. The weight of the pig was chosen so that the size of its vessels **266** would be approximate to the size of a human vessel. The pig was anesthetized. Several hard clots **270B** were prepared by mixing pig blood and barium and incubating the mixture for 2 hours. Several soft clots **270A** were prepared by mixing pig blood, thrombin and barium and incubating the mixture for 1 hour. The clots **270A** and **270B,** each of which had a width of 4 to 6 mm and a length of 10 to 40 mm, were then inserted into a vessel **266** having a diameter of 2 to 4 mm. (Only one clot **270A** and **270B** was located in the vessel **266** at a time). Angiograms were then performed to confirm occlusion. After waiting ten minutes after confirming occlusion, the distal bodies **216** of FIGs. 11-20 were then delivered distal to the clots **270A** and **270B** as described above and were used to retrieve the clots 270A and **270B** as described in FIGs. 11-19. In each case, the distal bodies **216** were successful in retrieving the clots **270A** and **270B.** As shown, the distal body height in the relaxed state tapers/decreases as the proximal strips **252** approach the proximal hub/junction/tube **228** and also tapers/decreases as the basket strips **291** located at the distal end **220** of the basket **246** converge at the distal hub/junction/tube **236.**

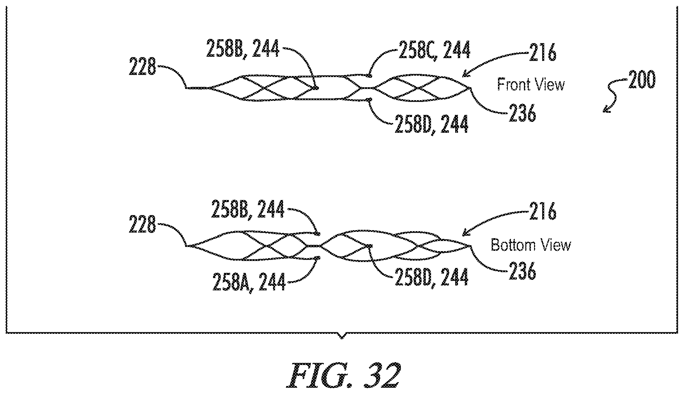

### The alternate exampleof FIG. 32

FIG. 32 shows a distal body **216** in which the proximal strips proximal ends **254** converge and are soldered or welded at the proximal hub/junction **228** and the basket strips **291** located at the distal end **220** of the basket **246** converge and are soldered or welded at the distal hub/junction **236.** To create such an embodiment, the distal body **216** may be prepared from a single tube, as described above, and the proximal and distal tubes may be clipped and the proximal ends **254** of the proximal strips **252** soldered or welded together (and optionally to the pull wire **202)** and the basket strips **291** located at the distal end **220** of the basket **246** may also be welded or soldered or welded together. Optionally, the proximal and distal hubs/junctions **228** and **236** may include x-ray markers **244** as described above.

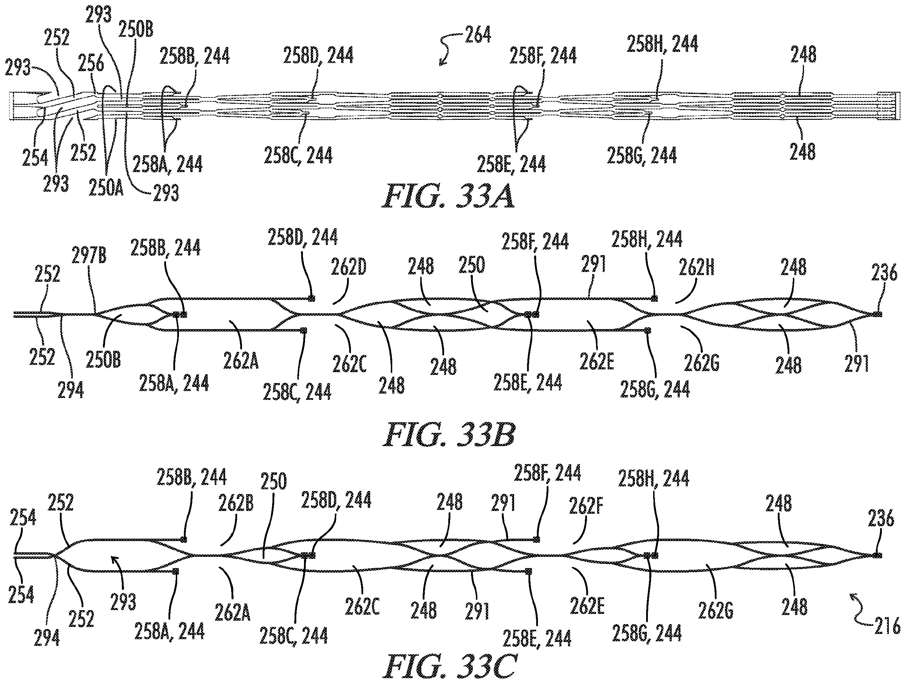

### The alternate examples of FIGs. 33-35

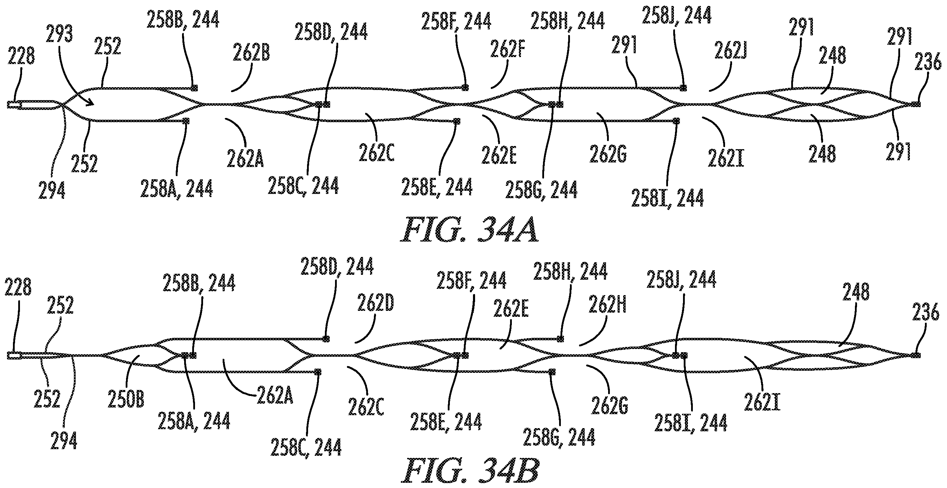

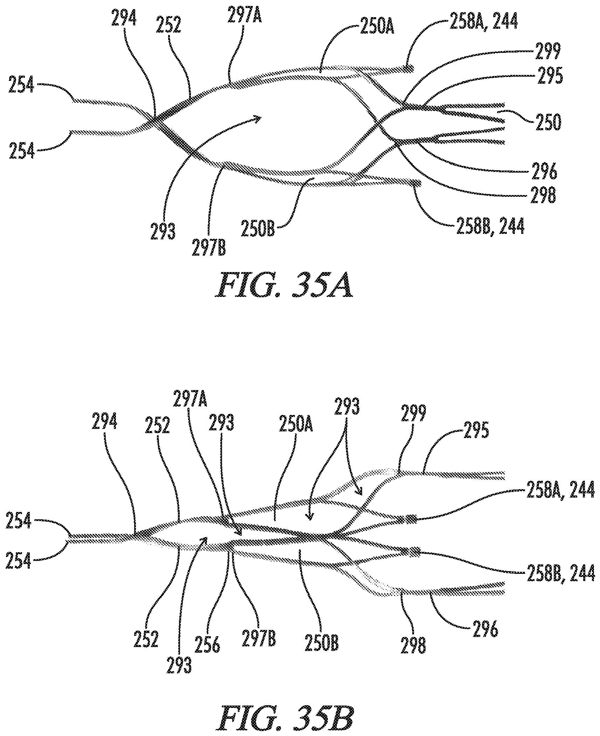

FIGs. 33A-33C and FIGs. 34A-34B (and the close-up views shown in FIGs. 35A and 35B) show an alternate example in which no cells are proximal to the proximal-most cells **250A** and **250B** that have a free distal crown **258A** and **258B.** (FIGs. 33A-35B do not show the pull wire or catheter, both of which are preferably used with the distal body **216** and shown elsewhere, e.g., in FIGs. 11-20, and FIGs. 33B-33C and FIG. 35A-35B do not show the proximal hub **228**). FIGs. 33A-33C has four pair of cells (only a few such cells are labelled with **250** or **250B** and visible) with free distal-pointing crowns **258A-258H,** which create four pairs of enlarged openings **262A-262H.** FIGs. 34A-34B has five pair of cells (again not all labelled) with free distal-pointing crowns **258A-258J,** which create five pairs of enlarged openings **262A-262J.** More particularly, like FIGs. 11-20, in FIGs. 33A-33C and FIGs. 34A-34B, the distal body **216** has a proximal pair of free distal crowns **258A** and **258B** that have x-ray markers **244** and are located approximately the same distance from the proximal junction **228** and between 150 degrees and 180 degrees apart and then a second pair of free distal crowns **258C** and **258D** that have x-ray markers **244,** and are located distal to the proximal pair of free distal crowns **258A** and **258B.** Like FIGs. 11-20, in FIGs. 33A-33C and FIGs. 34A-34B, the second pair of distal crowns **258C** and **258D** are located approximately the same distance from the proximal junction **228,** are located between 150 degrees and 180 degrees apart and are located between about 60 degrees and about 90 degrees relative to the proximal pair of free distal crowns **258A** and **258B.**

The examples of FIGs. 33A-33C, FIGs. 34A-34B and FIGs. 35A-35B have several additional features. For example, in FIGs. 33A-33C, FIGs. 34A-34B, and FIGs. 35A-35B, the proximal pair of cells **250A** and **250B** having free distal crowns **258A** and **258B** each have a proximal crown **297A** and **297B** attached to a proximal strip **252.** In FIGs. 33A-33C and FIGs. 34A-34B, distal to the second pair of free distal crowns **258C** and **258D,** the distal body **216** has a third pair of free distal crowns **258E** and **258F** that have x-ray markers **244,** are located approximately the same distance from the proximal junction **228** and are located between 150 degrees and 180 degrees apart and are located between about 60 degrees and about 90 degrees relative to the second pair of free distal crowns **258C** and **258D.** In FIGs. 33A-33C and FIGs. 34A-34B, distal to the third pair of free distal crowns **258E** and **258F,** the distal body **216** also has a fourth pair of free distal crowns **258G** and **258H** that have x-ray markers **244,** are located approximately the same distance from the proximal junction **228** and are located between 150 degrees and 180 degrees apart and are located between about 60 degrees and about 90 degrees relative to the third pair of free distal crowns **258E** and **258F.** In FIGs. 34A-34B, distal to the fourth pair of free distal crowns **258G** and **258H,** the distal body **216** also has a fifth pair of free distal crowns **258I** and **258J** that have x-ray markers **244,** are located approximately the same distance from the proximal junction **228** and are located between 150 degrees and 180 degrees apart and are located between about 60 degrees and about 90 degrees relative to the fourth pair of free distal crowns **258G** and **258H.** An advantage having eight unattached distal-pointing crowns **258A-258H** that are sequentially offset (as in FIGs. 33A-33C) and ten unattached distal-pointing crowns **258A-258J** that are sequentially offset (as in FIGs. 34A-34B) is that each free distal crown **258A-258J** creates an enlarged cell **262A-262J.** Thus, in FIGs. 33A-33C and FIGs. 34A-34B, eight and ten, respectively, sequentially offset enlarged cells **262A-262H** are formed, creating multiple clot entry points. In FIG. 34A-34B, with the exception of the enlarged cells **262A-262H,** all of the cells located between the cells **250A** and **250B** (**250A** is hidden in FIGs. 34A-34B but shown in FIGs. 35A-35B) having the proximal pair of free distal crowns **258A** and **258B** and the cells having the distal-most pair of free distal crowns **258I** and **258J** have free distal crowns. Indeed, with the exception of the enlarged cells **262A-262H,** all of the cells located between proximal-most free-distal crowns **258A** and **258B** and distal-most free crowns **258I** and **258J** have free distal crowns **258C-258H.** By contrast, the distal body **216** of FIGs. 33A-33C has an intermediate group of cells **248** (i.e., a stent-like structure) with non-free distal crowns (distal crowns that are attached to a basket strip **291**) between the second pair of enlarged openings **262C** and **262D** and the third pair of enlarged openings **262E** and **262F.** In FIG. 34A-34B, all of the cells **248** having distal crowns that are attached to a basket strip **291** are located at the distal end of the distal body **216** (i.e., distal to the distal-most enlarged openings **262G** and **262H**) to create a substantially closed distal end of the basket **246** to capture the clot.

In addition, in FIGs. 33A-33C, 34A-34B, FIGs. 35A-35B, the distal body **216** has two proximal three-dimensional openings **293** that are located 180 degrees apart, as best seen in FIG. 33C and FIG. 34A and the close-up views of FIGs. 35A and 35B. (The proximal three-dimensional openings **293** are aligned and create a continuous void). One such three-dimensional opening **293** has a proximal end at the intersection point **294** of the proximal strips **252** and a distal end at a distal crown **299** attached to a first strut **295** located (lengthwise) approximately the same distance from the proximal hub/junction **228** as the free distal crowns **258A** and **258B** of the first pair of free distal crowns. The other such three-dimensional opening **293** is on the other side of the distal body **216** and has a proximal end at the intersection point **294** of the proximal strips **252** and a distal end at another distal crown **298** attached to a second strut **296** that is also located (lengthwise) approximately the same distance from the proximal hub/junction **228** as the free distal crowns **258A** and **258B** of the first pair of free distal crowns. The second strut **296** is located 180 degrees from the first strut **295.** As shown in FIG. 35A, the intersection point **294** of the proximal strips **252** may be located approximately in the widthwise and heightwise center of the distal body **216** and the proximal crowns **297A** and 297B of the proximal-most cells **250A** and **250B** having the free distal crowns **258A** and **258B** may be located approximately 150 degrees to 180 degrees apart (e.g., at the 12 o'clock and 6 o'clock positions as shown in FIG. 35A and the 3 o'clock and 9 o'clock positions in FIG. 35B) and at the maximum height/width of the distal body **216.** The proximal crowns **297A** and **297B** of the proximal-most cells **250A** and **250B** having the free distal crowns **258A** and **258B** also are attached to the proximal strips **252** as shown in FIGs. 35A and 35B. Similarly, the bridge memory metal strips (distal struts) **295** and **296** may be located approximately 150 degrees to 180 degrees apart (e.g., at the 3 o'clock and 9 o'clock positions as shown in FIG. 35A and the 12 o'clock and 6 o'clock positions in FIG. 35B), at the maximum height/width of the distal body **216,** and approximately 60 degrees to 90 degrees relative to the free distal crowns **258A** and **258B.**

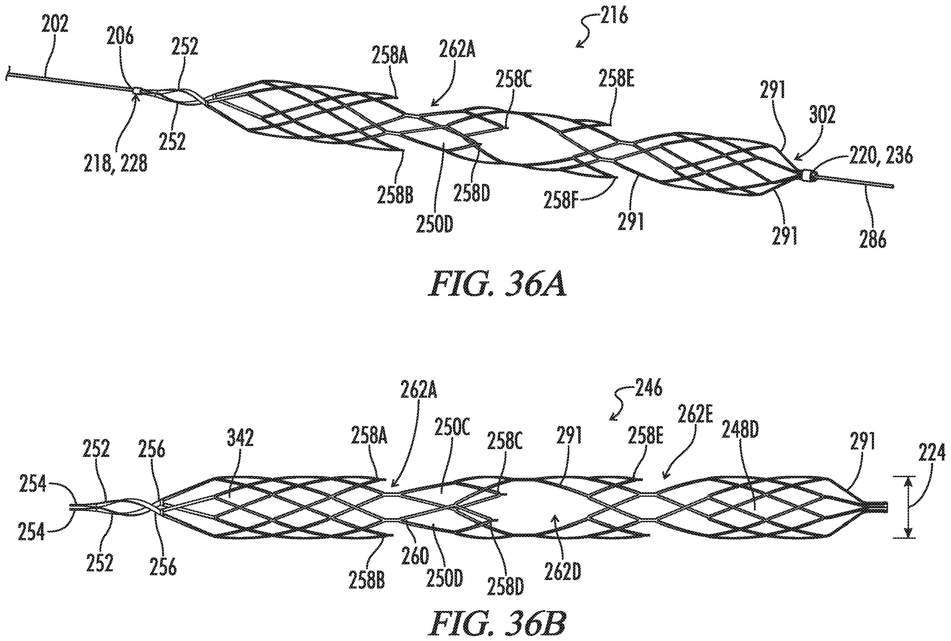

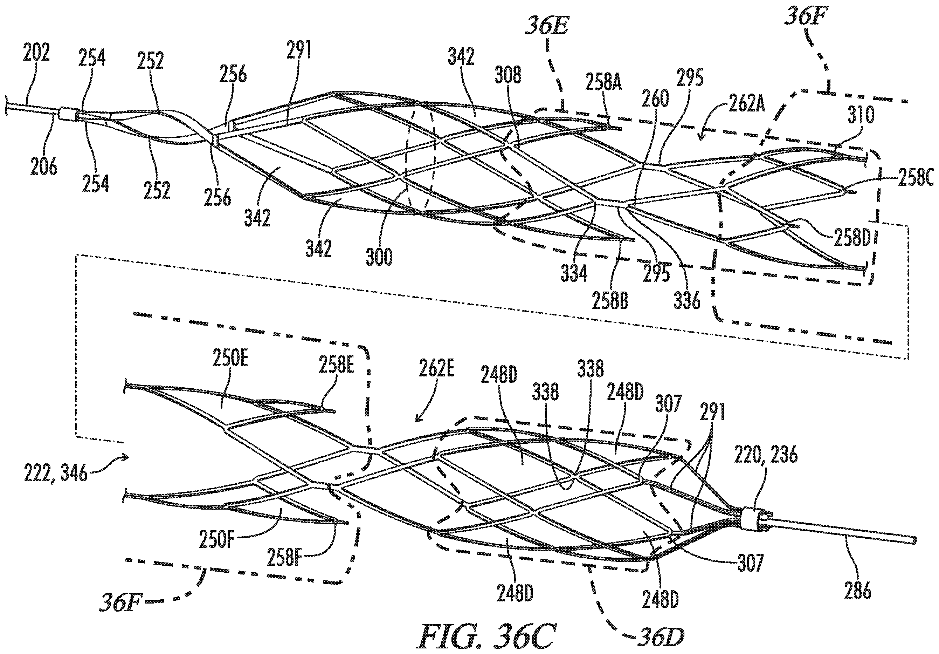

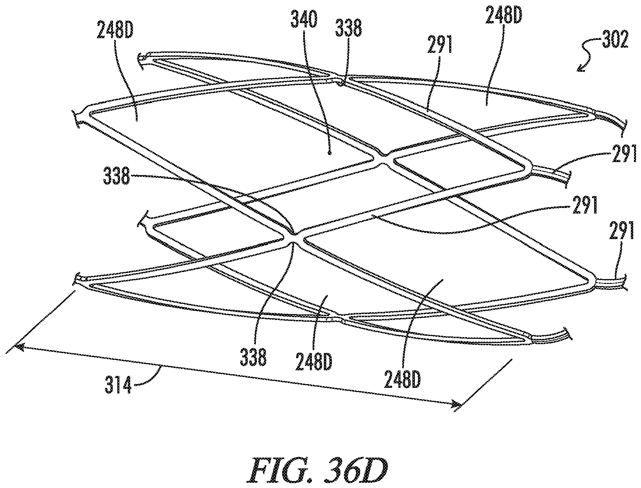

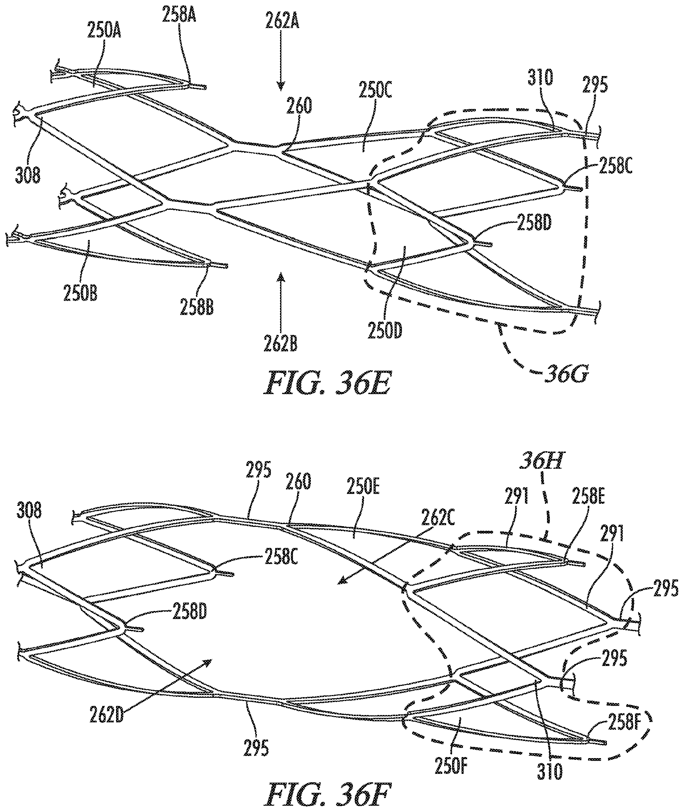

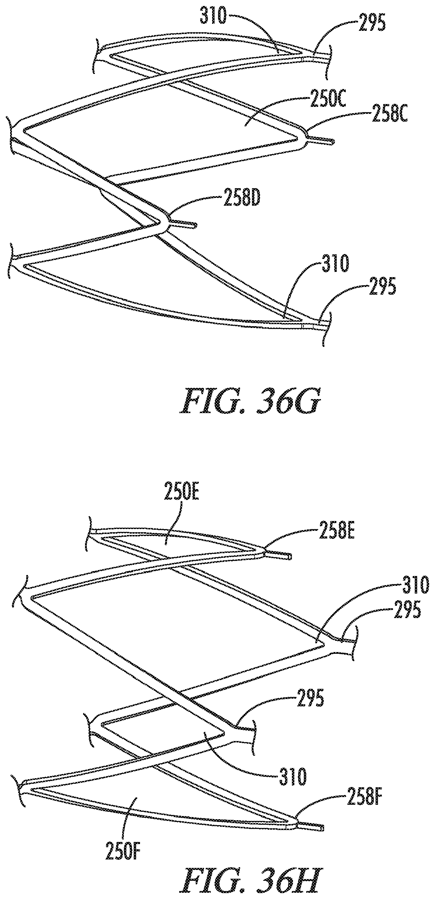

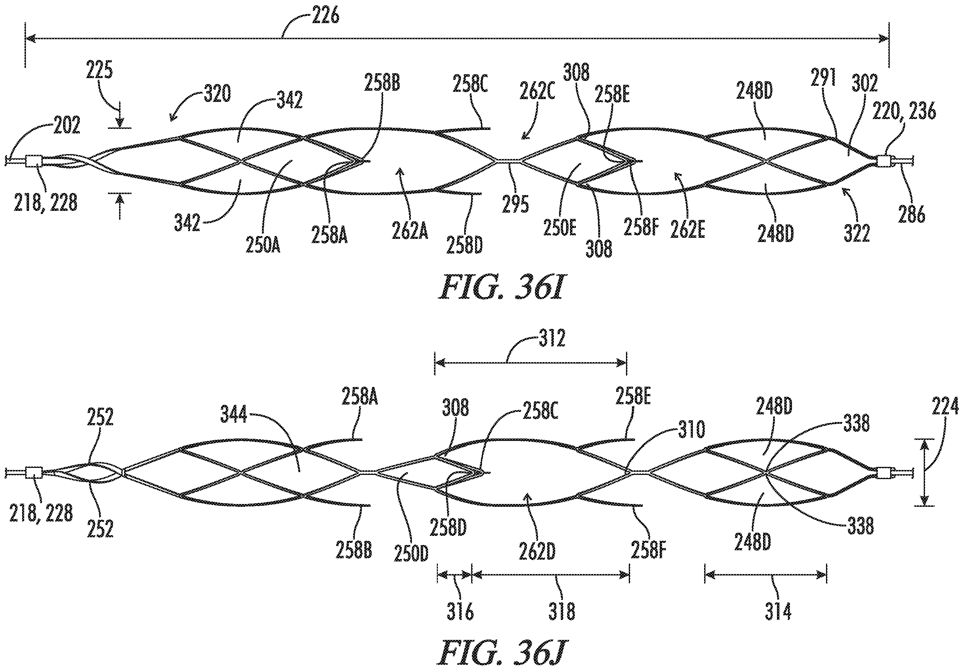

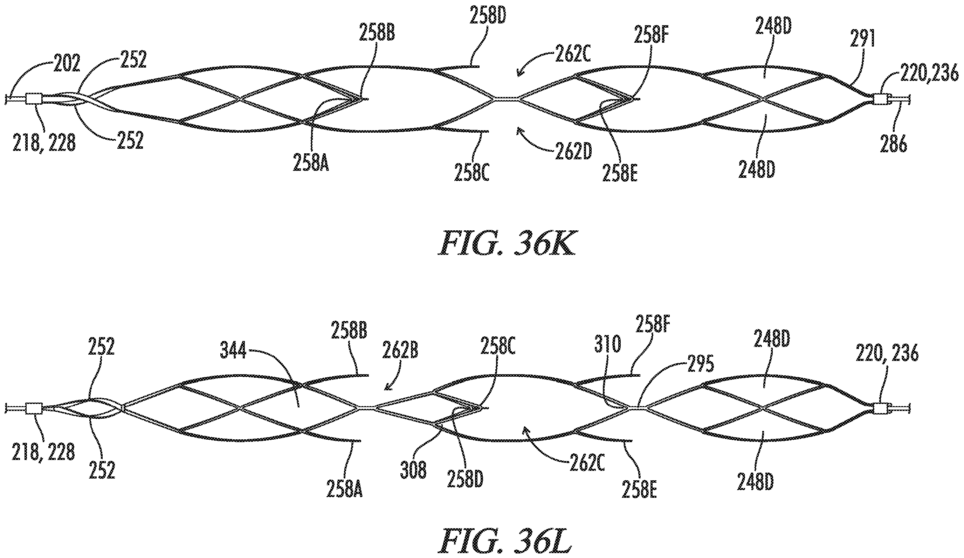

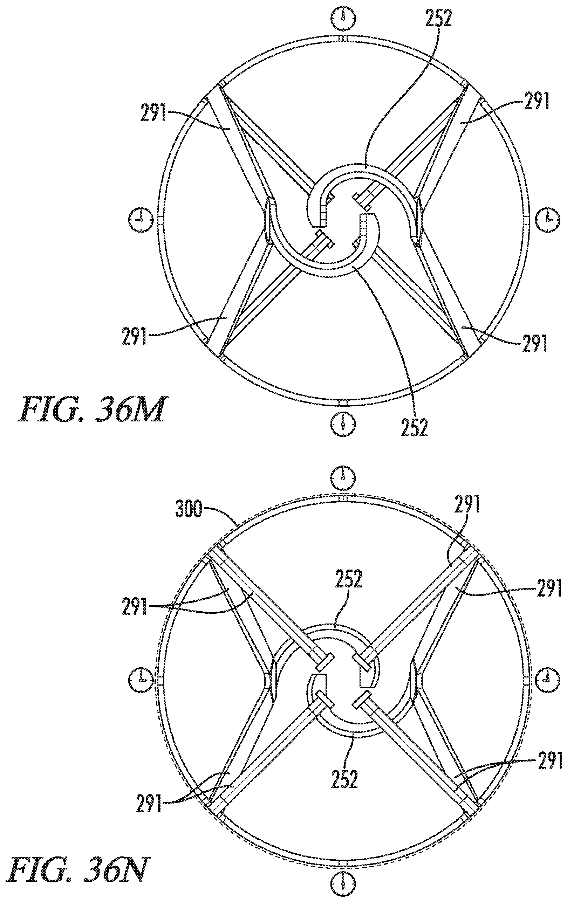

### The Examples of FIGs. 36-37

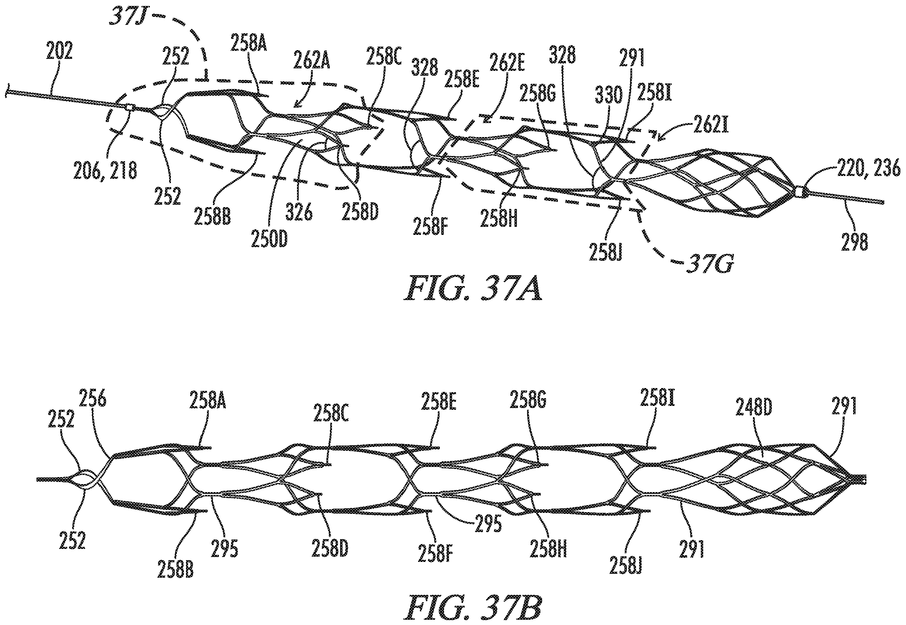

FIGs. 36A-36N and 37A-37K (also referred to herein as FIGs. 36-37 for brevity) show alternate examples of distal bodies **216** that are similar to the examples of FIGs. 11-35. The distal bodies **216** may be made by any method known in the art including but not limited to those described in U.S. Patent No. 9,566,412.

FIGs. 36-37 are CAD drawings drawn to scale. However, it will be appreciated that other dimensions are possible.

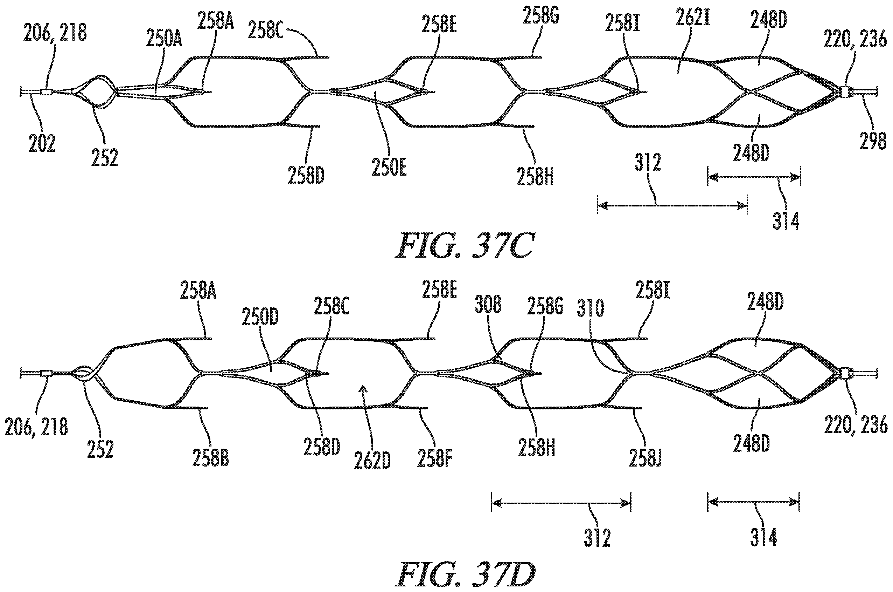

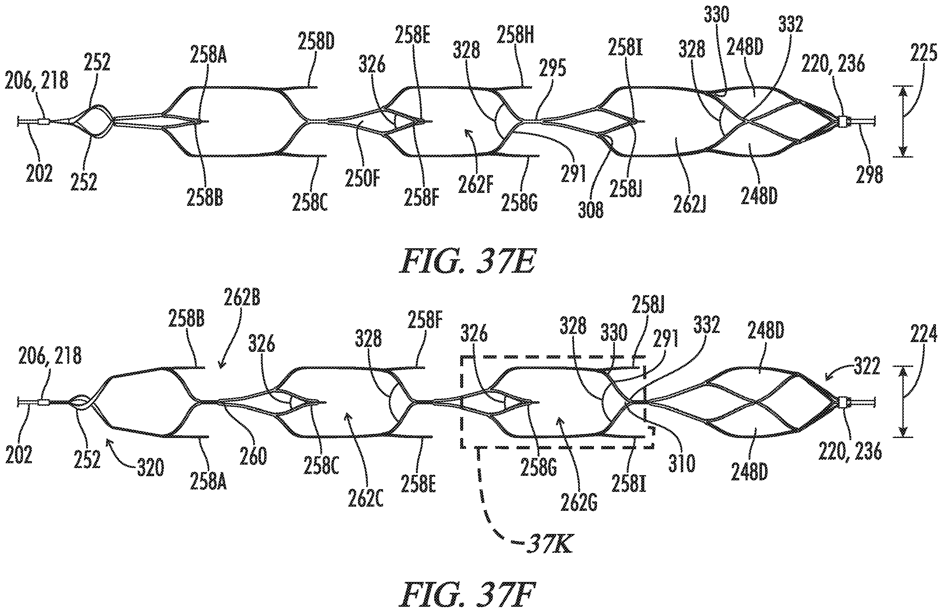

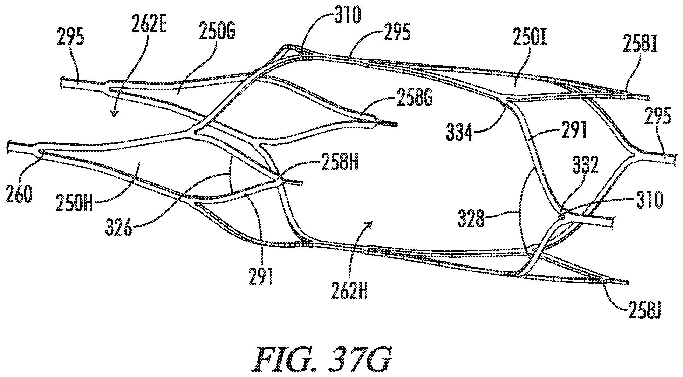

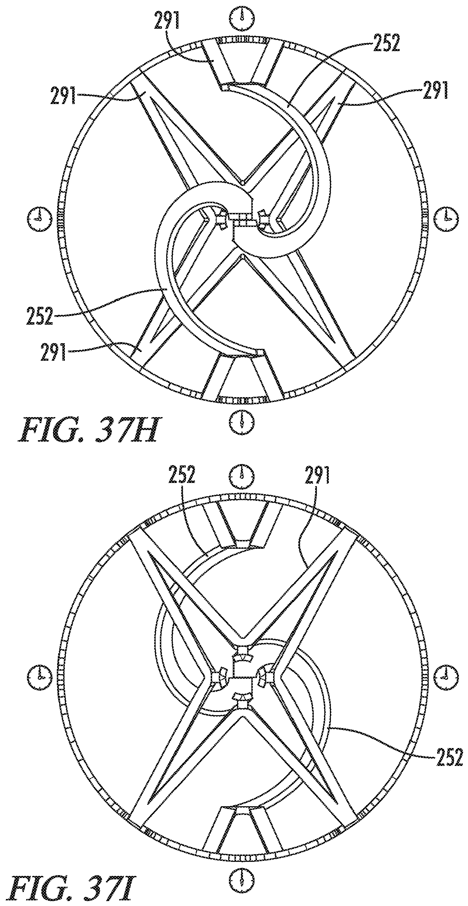

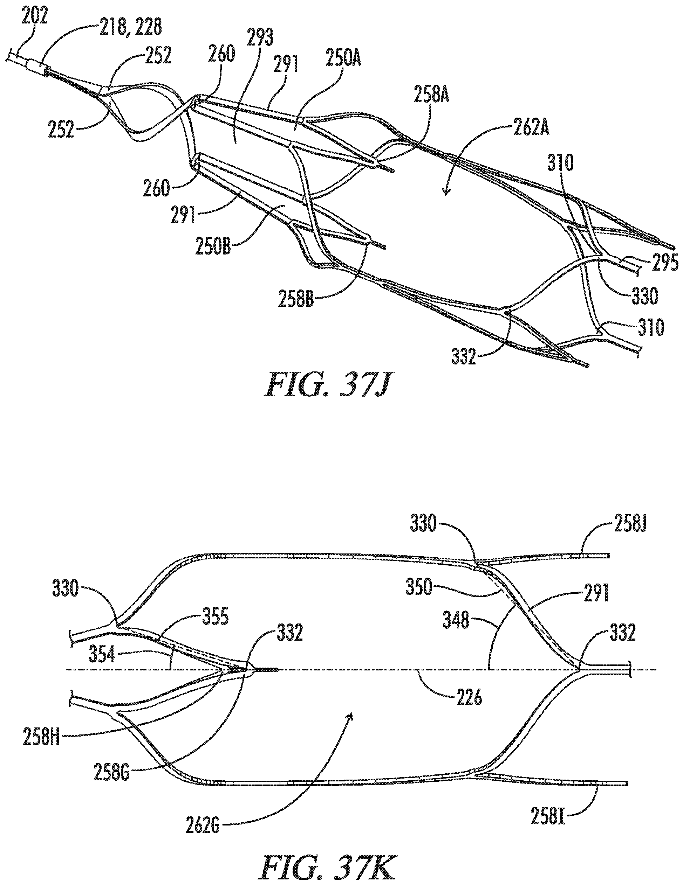

More particularly, FIGs. 36-37 provide a system for removing objects from an interior lumen of an animal. The system may include, as previously described, a pull wire **202** having a proximal end (not shown in FIGs. 36-37) and a distal end **206.** The system of FIGs. 36-37 also includes a distal body **216** that may be attached to the pull wire **202,** preferably the pull wire distal end **206.** As with the prior examples of FIGs. 11-35, the distal body **216** may include an interior **222,** a perimeter **300,** a proximal end **218,** a distal end **220,** a distal body length **226** extending from the proximal end **218** to the distal end **220,** a proximal junction/hub/tube **228** that may be attached to the pull wire **202** and may form the proximal end **218** of the distal body **216,** a plurality of proximal strips **252,** a basket **246** comprised of a plurality of cells formed by a plurality of basket strips **291,** and a distal junction/hub/tube **236** forming a distal end **302** of the basket **246.** As with the prior examples of FIGs. 11-35, the basket **246** may include a basket interior **346,** each proximal strip **252** may have a distal end **256** attached to a cell and a proximal end **254,** the proximal ends **254** of the proximal strips **252** may converge at the proximal junction **228.** As with the prior examples of FIGs. 11-35, the distal body **216** may have a relaxed state wherein the distal body **216** has a first height **224** and a first width **225,** and a collapsed state (not shown in FIGs. 36-37) wherein the distal body **216** has a second height and a second width, the second height less than the first height **224,** the second width less than the first width **225.** As with prior examples, in the exampleof FIG. 36 and the embodiment of FIG. 37, the proximal strips **252** and the basket strips **291** are preferably comprised of a memory metal.

Like the embodiments of FIGs. 33-35, in the example of FIG. 36 and the embodiment FIG. 37, in the relaxed state, the basket **246** may include a series of at least three pair of cells **250A-250F** located on the distal body perimeter **300** having a proximal crown **260** pointing generally in the proximal direction and attached to a memory metal strip (either a proximal strip **252** or basket strip **291**) and a free distal crown **258A-258F** pointing generally in the distal direction. Optionally, as shown in FIGs. 36-37, in the series, the proximal-most free distal crowns **258A, 258B** are located at the 12 and 6 o'clock positions and located about the same distance from the proximal junction **228,** the next proximal-most free distal crowns **258C, 258D** are located at the 3 and 9 o'clock positions and located about the same distance from the proximal junction **228,** and the succeeding proximal-most free distal crowns **258E, 258F** are located at the 12 and 6 o'clock positions (i.e., substantially aligned with proximal-most free distal crowns **258A, 258B)** and located about the same distance from the proximal junction **228.** Optionally, each free distal crown **250A-250F** forms part of a different enlarged cell **262A-262F** that is configured to allow a thrombus to enter the basket interior **346.** In other words, like the prior examples, the enlarged cells **262A-262F** are designed to capture a clot/thrombus. In the proximal and distal end views of FIGs. 36M, 36N, 37H, and 37I, miniature clocks with clock hands are used to illustrate the 12, 3, 6 and 9 o'clock positions.

For purposes of FIGs. 36-37, when it is said that a component, such as a free distal crown **258A-258J,** is located "about the same distance from the proximal junction" **228,** if one component (e.g., one free distal crown **258A** of the pair of proximal-most free distal crowns **258A/258B**) is located X distance from the proximal junction **228,** the other component (e.g., the other free distal crown **258B** of the pair of proximal-most free distal crowns **258A/258B**) is located X distance plus or minus (+/-) 5 millimeters (mm) from the proximal junction **228.** In a preferred embodiment, the other component is located X distance plus or minus (+/-) 3 mm from the proximal junction **228,** more preferably X distance plus or minus (+/-) 0.5 mm from the proximal junction **228.** (The same relationship will hold true for the other pairs of free distal crowns **258C-258F** in the series - e.g., if one free distal crown **258C** of the next proximal-most pair of free distal crowns **258C/258D** is located Y distance from the proximal junction **228,** the other free distal crown **258D** of the next proximal-most pair of free distal crowns **258C/258D** is located Y distance plus or minus (+/-) 5 mm from the proximal junction **228.** Again, more preferably the other component is located Y distance plus or minus (+/-) 3 mm from the proximal junction **228,** more preferably Y distance plus or minus (+/-) 0.5 mm from the proximal junction **228.** The same relationship holds true from **258E** and **258F.**

In FIG. 37, like the example of FIG. 34, the series includes at least five pair of cells **250A-250J** located on the distal body perimeter **300** having a proximal crown **260** pointing generally in the proximal direction and attached to a memory metal strip (either a proximal strip **252** or basket strip **291**) and a free distal crown **258A-258J** pointing generally in the distal direction, and in the series, after the succeeding free distal crowns **258E, 258F,** the next proximal-most free distal crowns **258G, 258H** are located at the 3 and 9 o'clock positions and located about the same distance from the proximal junction **228** and form enlarged cells **262G, 262H,** and the distal-most free distal crowns **258I, 258J** are located at the 12 and 6 o'clock positions and located about the same distance from the proximal junction **228** and form the most distally-located enlarged cells **262I, 262J.** Again, about the same distance from the proximal hub **228,** means the same distance +/- 5 mm from the proximal junction **228.** In a preferred embodiment, **258G/258H** are located the same distance +/- 3 mm from the proximal junction **228,** more preferably the same distance +/- 0.5 mm from the proximal junction. The same relationship holds true from **258I** and **258J.**

As with prior examples, FIGs. 36-37 illustrate that in the relaxed state, the basket **246** may comprise a plurality of distal cells **248D** distal to the distal-most free distal crown (i.e. **258E, 258F** in FIG. 36 and **258I**, **258J** in FIG. 37) that have a proximal crown attached to another cell of the basket **246** and pointing generally in the proximal direction and a distal crown pointing generally in the distal direction and attached to the distal junction **236.** In other words, the distal cells **248D** are designed to retain a clot/thrombus in the basket interior **346.**

As with prior examples, FIGs. 36-37 illustrate that, in the relaxed state, each enlarged cell **262A-262J** may have a proximal end **308** that may be comprised of two proximal crowns pointing generally in the proximal direction, a distal end **310** that may comprise a distal crown pointing generally in the distal direction, and a length **312** extending from the proximal end **308** to the distal end **310** of the respective enlarged cell **262A-262J.**

As with prior examples, FIGs. 36-37 illustrate that in the relaxed state, each distal cell **248D** may have a length **314** extending from the proximal crown to the distal crown **307** of the respective distal cell **248D.**

As with prior examples, FIGs. 36-37, and particularly FIG. 36J, illustrate that in the relaxed state, each of the enlarged cells **262A-262J** may be longer than each of the distal cells **248D.**

As with prior examples, FIGs. 36-37, and particularly FIG. 36J, illustrate that in the relaxed state, for each of the enlarged cells **262A-262J,** the distance **316** from the proximal end **308** of the enlarged cell **262A-262J** to the free distal crown **258A-258J** of the enlarged cell **262A-262J** may be less than the distance **318** from the free distal crown **258A-258J** of the enlarged cell **262A-262J** to the distal end **310** of the enlarged cell **262A-262J.** In other words, the free distal crowns **258A-258J** do not protrude far enough into the enlarged cells **262A-262J** to prevent a clot from entering the basket interior **346** through the enlarged cells **262A-262J.**

As with prior examples, FIGs. 36-37 illustrate that the distal body **216** may further comprise a lead wire **286** extending distally from the distal junction **236.** As with prior examples but not shown in FIGs. 36-37, the system may further comprise a catheter having an interior, a proximal end leading to the interior and a distal end leading to the interior, the catheter comprised of a biocompatible material and configured to envelop the distal body **216** when the distal body **216** is in the collapsed state. Deployment and use of the system may be as shown in FIGs. 15-29.

As with prior examples, FIGs. 36-37 illustrate that in the relaxed state, the basket **246** preferably does not have any free crowns that point generally in the proximal direction, as free proximal crowns could damage the vessel as mentioned previously.

As with prior examples, FIGs. 36-37 illustrate that the distal body **216,** in the relaxed state, may comprise a distal tapered region **322** in which the distal body height **224** and width **225** decrease as the basket **246** approaches the distal junction **236.**

As with prior examples, FIGs. 36-37 illustrate that the distal body **216,** in the relaxed state may comprise a proximal tapered region **320** in which the distal body height **224** and width **225** decrease as the proximal strips **252** approach the proximal junction **228.** It will be appreciated that the tapering of the proximal region **320** may take on a variety of shapes as shown in FIGs. 36 and 37.

As with prior examples, FIGs. 36-37 illustrate that in the relaxed state, each of the enlarged cells **262A-262J** may be longer than each of the pair of cells **250A-250J.**

As with prior examples, FIGs. 36-37 illustrate that in the relaxed state, each of the enlarged cells **262A-262J** may extend from the 6 o'clock position to the 12 o'clock position or the 3 o'clock position to the 9 o'clock position, as best seen in the close-up views of FIGs. 36C, 36E and 36F and 37G but also shown in FIGs. 36A-36B, 36I-36L, 37A-37F.

As with the prior examples of FIGs. 11-19 and 21-32, FIG. 36 illustrates that in the relaxed state the basket **246** may further comprise a plurality of proximal cells **342** proximal to the proximal-most free distal crowns **258A, 258B,** the plurality of proximal cells **342** having a proximal crown attached to the proximal junction **228** and pointing generally in the proximal direction and a distal crown pointing generally in the distal direction and attached to another cell of the basket **246.** However, such proximal cells **342** are optional and not shown in FIG. 37, due to the length of the basket **246** which includes ten enlarged cells **262A-262J.** If the proximal cells **342** are not included, optionally, as with the prior examples of FIGs. 20 and 33-35, FIG. 37 illustrates that in the relaxed state the proximal crowns of the cells **250A, 250B** comprising the proximal-most free distal crowns **258A, 258B** may be attached to the distal ends **256** of the proximal strips **252.** Without being bound by any particular theory, a purpose of the proximal cells **342** may be to allow the clot to rest on the proximal cells **342** prior to entering through the enlarged cells **262A-262J.**

In preliminary clot capture animal studies using a swine thrombectomy model, it has been observed the distal body **216** of FIG. 37 is particularly adept at capturing a clot. (Preliminary clot capture animal studies were performed using conditions similar to that described in Ulm et al., Preclinical Evaluation of the NeVaTM Stent Retriever: Safety and Efficacy in the Swine Thrombectomy Model, Interv Neurol. 2018 Apr;7(5):205-217). Without being bound by any particular theory, it is believed that the clot capture ability of the distal body **216** of FIG. 37 is due to the minimal interference at the enlarged cells **262A-262J** (i.e., that the basket strips **291** are flared apart at the distal crowns located at the distal ends **310** of the enlarged cells **262A-262J).**

Thus, in accordance with the present invention and as shown in FIG. 37, in the relaxed state, for each enlarged cell **262A-262J**, two basket strips **291** meet to form the distal crown located at the distal end **310** of the enlarged cell **262A-262J,** and the two basket strips **291** form a maximum angle **328** greater than 90 degrees, more prefereably greater than 95 degrees, more preferably greater than 100 degrees at the respective distal crown located at the distal end **310** of the enlarged cell **262A-262J.** Preferably, the angle **328** is less than 150 degrees. It is believed that the flared nature of the basket strips **291** meeting at the distal crown at the distal end **310** of the enlarged cells **250A-250J** assists in allowing the clot to enter the basket interior **346.** By comparison, the basket strips **291** meeting to form the free distal crowns **258A-258J** may have a much smaller angle **326** at the respective free distal crown **258A-258J,** given that cells **250A-250J** are not intended to be clot capture cells. For example, the angle **326** at the respective free distal crown **258A-258J** may be less than 50 degrees, more preferably less than 45 degrees, more preferably less than 40 degrees. In other words, for some, most or all enlarged cells **262A-262J,** the angle **328** at the distal crown at the distal end **310** may be at least 2 times (more preferably at least 2.5 times, more preferably at least 3 times) as large as the angle **326** at the free distal crown **258A-258J.** Preferably, angle **328** is no more than 5 times as large as angle **326.** Due to the symmetry of the distal body **216,** the angle **326** at free distal crown **258A** may be substantially the same as the angle **326** at free distal crown **258B,** the angle **326** at free distal crown **258C** may be substantially the same as the angle **326** at free distal crown **258D,** the angle **326** at free distal crown **258E** may be substantially the same as the angle **326** at free distal crown **258F,** the angle **326** at free distal crown **258G** may be substantially the same as the angle **326** at free distal crown **258H,** the angle **326** at free distal crown **258I** may be substantially the same as the angle **326** at free distal crown **258J.** Similarly, the angle **328** at the distal crown at the distal end **310** of enlarged cell **262A** may be substantially the same as the angle **328** at the distal crown of enlarged cell **262B,** the angle **328** at the distal crown at the distal end **310** of enlarged cell **262C** may be substantially the same as the angle **328** at the distal crown of enlarged cell **262D,** the angle **328** at the distal crown at the distal end **310** of enlarged cell **262E** may be substantially the same as the angle **328** at the distal crown of enlarged cell **262F,** the angle **328** at the distal crown at the distal end **310** of enlarged cell **262G** may be substantially the same as the angle **328** at the distal crown of enlarged cell **262H,** and the angle **328** at the distal crown at the distal end **310** of enlarged cell **262I** may be substantially the same as the angle **328** at the distal crown of enlarged cell **262J.**

Because the basket strips **291** may be non-linear, the angles **326** and **328** may be determined as if the basket strips **291** were straight from their proximal ends **330** to their distal ends **332.** (*See* dashed lines **350** and **355** in FIG. 37K). In other words, the angles **326** and **328** may be the average (mean) angle between the basket strips **291** along the basket strip lengths. In such measurements, the angles **326** and **328** are measured by drawing an arc between a point on each basket strip **291** at the same position along the distal body length **226.** Alternatively, the angles **326** and **328** may be the maximum angle between the basket strips **291** along the basket strip lengths, drawing an arc between a point on each of the two basket strips **291** at the same position along the distal body length **226.** *See* FIG. 37A, 37E, 37F, 37G for showing how arcs are drawn and maximum angles **326** and **328** are measured.

As an example, the maximum angles **328** between the basket strips **291** forming the distal crowns at the distal ends **310** of the enlarged cells **262C, 262F,** and **262G,** as illustrated in FIGs. 37E-37G, are approximately 100 degrees, whereas the maximum angles **326** between the basket strips **291** forming free distal crowns **258C, 258F,** and **258G,** as illustrated in FIGs. 37E-37G, by comparison are about 40 degrees. (It should be noted that the maximum angle **328** between the basket strips **291** forming the distal crown at the distal end **310** of distal-most enlarged cell **262J,** as illustrated in FIG. 37E, is approximately 80 degrees, showing that there may be variability in the angles **326, 328**).

Relatedly, optionally, in the relaxed state, each of the basket strips **291** meeting at the distal crown at the distal end **310** of some or all of the enlarged cells **250A-250J** extends proximally from the respective distal crown at the distal end **310** at an angle **348** of at least 32.5 degrees, more preferably at least 35 degrees, more preferably at least 37.5 degrees, more preferably at least 40 degrees, more preferably at least 42.5 degrees, more preferably at least 45 degrees, more preferably at least 47.5 degrees, more preferably at least 50 degrees relative to a line bisecting the respective distal crown that is parallel to the distal body length **226** as best seen in FIG 37K. The angle **348** may be determined by measuring the basket strip **291** as if it were straight from the proximal end **330** to the distal end **332.** Thus, for example, each basket strip length, as measured from drawing a straight line from the proximal end **330** to the distal end **332** of the basket strip **291** may be at least 32.5 degrees, more preferably at least 35 degrees, more preferably at least 37.5 degrees, more preferably at least 40 degrees, more preferably at least 42.5 degrees, more preferably at least 45 degrees, more preferably at least 47.5 degrees, more preferably at least 50 degrees relative to a line bisecting the respective distal crown that is parallel to the distal body length **226** (as well as an adjacent bridge memory metal strip **336,** which as explained herein may be substantially parallel to the distal body length **226**). *See* FIG. 36K (dotted line **350** representing basket strip length, dotted line **226** drawn through the respective distal crown that is parallel to the distal body length **226,** and angle **348** between the lines **350** and **226** measuring 45 degrees). Relatedly, for some, most or all the free distal crowns **258A-258J,** the basket strip lengths, as measured from drawing a straight line from the proximal end **330** to the distal end **332** of the basket strip **291** may be less than 25 degrees, more preferably less than 22.5 degrees, more preferably less than 20 degrees relative to a line that bisects the free distal crowns **258A-258J** and is parallel to the distal body length **226.** *See* FIG. 36K (dotted line **355** representing basket strip length, dotted line **226** drawn through distal crown **258H** that is parallel to the distal body length **226,** and angle **354** between the lines **355** and **226** measuring 18 degrees at free distal crown **258H**). Thus, for some, most or each enlarged cell **262A-262J,** angle **348** at free distal crowns **258A-258J** may be twice, more preferably 2.5 times, more preferably 3 times the size of angle **354.**

Relatedly, in FIG. 37E, which shows a top plan view, the distal ends **332** of the basket strips **291** forming the distal crown of enlarged cell **262J** are located approximately in the center of the distal body width **225** and the proximal ends **330** of the basket strips **291** are located approximately at the front and rear of the distal body **216.** The proximal end **330** and distal ends **332** of the basket strips **291** meeting to form the distal crown located at the distal end **310** of enlarged cell **262H** are also shown in the perspective view of FIG. 37G. Optionally, the lengths of each basket strip **291** meeting at the distal crown at the distal end **310** of some or all of the enlarged cells **250A-250J,** as measured from their proximal ends **330** to their distal ends **332,** may be approximately equal to ½ of the distal body width **225** and height **224.**

Relatedly, optionally, as shown in the embodiment of FIG. 37 and best seen in FIG. 37G and 37K, in the relaxed state, for at least some enlarged cells **262A-262J** (preferably for each enlarged cell **262A-262J**), in the relaxed state, the two basket strips **291** meeting to form the distal crown located at the distal end **310** of the enlarged cell **262A-262J** may have distal ends **332** meeting at the respective distal crown and located approximately in the center of the distal body height **224** or width **225** and a proximal end **330** attached to another cell of the basket **246** and located approximately at the top, bottom or front side, or rear side of the distal body **216.** In other words, in FIGs. 37F and 37K, which show front and rear elevation views, the distal ends **332** of the basket strips **291** forming the distal crown of enlarged cells **262G** and **262H** are located approximately in the center of the distal body height **224** and the proximal ends **330** of the basket strips **291** are located approximately at the top and bottom of the distal body **216.**

As with the prior examples of FIGs. 34A-34B, in the distal body **216** of FIG. 37, as seen in FIGs. 37E-37F for example, in the relaxed state, due to the space occupied by the ten enlarged cells **250A-250J,** from at least the proximal crowns **260** of the cells **250C, 250D** comprising the next proximal-most free distal crowns **258C, 258D** to the proximal ends **308** of the enlarged cells **262I, 262J** formed by the distal-most pair of free distal crowns **258I, 258J,** the basket **246** has no cells other than the enlarged cells **262A-262J** and the cells **250C-250J** comprising the free distal crowns **258C-258J.**

In the distal body **216** of FIG. 36, in the relaxed state, due to the space occupied by the six enlarged cells **250A-250F,** as seen in FIGs. 37E-37F for example, from at least the proximal crowns **260** of the cells **250C, 250D** comprising the next proximal-most free distal crowns **258B, 258C** to the proximal ends **308** of the enlarged cells **262E, 262F** formed by the succeeding pair of free distal crowns **258E, 258F** the basket **246** has no cells other than the enlarged cells **250A-250F** and the cells **262C-262F** comprising the free distal crowns **258C-258F.**

The distal bodies **216** may be substantially symmetrical from front to rear and bottom to top, as shown in FIGS. 36I-36L and 37C-37F for example.

As with prior examples, FIGs. 36-37 illustrate that in the relaxed state, the distal crowns located at the distal end **310** of the enlarged cells **250A-250J** may be attached to another cell of basket **246** (i.e., are not free floating).

Optionally, as with prior examples, in the example of FIG. 36, as best seen in FIGs. 36J and 36L, in the relaxed state, the basket **246** further comprises an additional pair of cells **344** located about the same distance from the proximal junction **228** as the cells **250A, 250B** comprising the proximal-most pair of free distal crowns **258A, 258B** and located at the 9 and 3 o'clock positions, each cell of the additional pair of cells **344** having a proximal crown attached to a memory metal strip (either a basket strip **291** or a proximal strip **252**) and a distal crown attached to another cell of the basket **246** (i.e., a non-free floating distal crown). Again, about the same distance means the same distance from the proximal hub **228** +/- 5 mm. (In preferred examples, the additional pair of cells **344** are located the same distance +/- 3 mm, more preferably the same distance +/- 0.5 mm, from the proximal hub **228**). As best seen in FIGs. 36J and 36L, the additional pair of cells **344** may adjoin the enlarged cells **262A, 262B** formed by the proximal-most free distal crowns **258A, 258B.** Optionally, from at least the distal crowns of the additional pair of cells **344** to the proximal ends of the enlarged cells **262E, 262F** formed by the succeeding free distal crowns **258E, 258F,** the basket **246** has no cells other than the enlarged cells **262A-262F,** the cells **250A-250F** comprising the free distal crowns **258A-258F** and the additional pair of cells **344.**

As with prior examples, FIGs. 36-37 illustrate that in the relaxed state, the basket **246** may comprise a series of struts/bridge memory metal strips **295** having a proximal end **334** attached to a distal crown of a cell and a distal end **336** attached to a proximal crown of a distally-located cell. The proximal-most pair of bridge memory metal strips **295** may be located at the 3 and 9 o'clock positions, the next proximal-most pair of bridge memory metal strips **295** may be located at the 12 and 6 o'clock positions, the succeeding proximal-most pair of bridge memory metal strips may be located at the 3 and 9 o'clock positions, so that the bridge memory strips **295,** like the free distal crowns **258A-258J,** alternate along the distal body length **226.** Each of the pair of bridge memory metal strips **295** may form part of at least one enlarged cell **262A-262J.** Optionally, the bridge memory metal strips **295** are the sole distally-extending basket strips **291** attached to the respective proximal crowns and the sole proximally-extending basket strips **291** attached to the respective distal crowns.

As with prior examples, FIGs. 36-37 illustrate that in the relaxed state, each of the pair of bridge memory metal strips **295** may form part of two enlarged cells **262A-262J.** As with prior examples, FIGs. 36 and 37 illustrate that in the relaxed state the bridge memory metal strips **295** may be substantially parallel to the distal body length **226.**

As with prior examples, FIGs. 36-37 illustrate that in the relaxed state, the plurality of distal cells **248D** may be comprised of four distal cells **248D** located about the same distance from the proximal junction **228,** each cell having a center **340,** and the centers **340** of the cells **248D** may be spaced at approximately 90 degree intervals about the distal body perimeter **300,** and each of said four distal cells **248D** may adjoin two of the other of said distal four cells **248D,** as best seen in FIGs. 36C and 36D. Again, about the same distance means the same distance from the proximal hub **228** +/- 5 mm. (In preferred embodiments, the four distal cells **248D** are located the same distance +/- 3 mm, more preferably the same distance +/- 0.5 mm, from the proximal hub **228**). Optionally, in the relaxed state, as best seen in FIGs. 36C and 36D, each of the four distal cells **248D** comprises two lateral crowns **338** pointing generally in a direction perpendicular to the distal body length **226** and each lateral crown **338** of one of said four cells **248D** adjoins a lateral crown **338** of an adjacent of said four cells **248D.**

As with prior examples, FIGs. 36-37 illustrate that the distal body **216** preferably has no more than four cells at any location along the distal body length **226.**

As with prior examples, FIGs. 36-37 illustrate that each of the enlarged cells **260A-260J** may be approximately the same size.

As with prior examples, FIGs. 36-37 illustrate that in the relaxed state, the basket interior **346** may be substantially hollow.

As with prior examples, FIGs. 36-37 illustrate that in the relaxed state, the enlarged cells **262A, 262B** formed by the proximal-most free distal crowns **258A, 258B** may be adjoining, the enlarged cells **262C, 262D** formed by the next proximal-most free distal crowns **258C, 258D** may be adjoining and the enlarged cells **262E, 262F** formed by the succeeding free distal crowns **258E, 258F** may be adjoining. Optionally, as shown in the illustrations of FIGs. 36-37, in the relaxed state each of the enlarged cells **262C, 262D** formed by the next proximal-most free distal crowns **258C, 258D** adjoins an enlarged cell **262A, 262B** formed by a proximal-most free distal crown **258A, 258B** and an enlarged cell **262E, 262F** formed by a succeeding free distal crown **258E, 258F.**

As with prior examples, FIGs. 36-37 illustrate that in the relaxed state, for at least some enlarged cells **262A-262J** (preferably for each enlarged cell, as shown in the illustrations), the free distal crown **258A-258J** may be aligned with the distal crown **307** located at the distal end **310** of the enlarged cell **262A-262J.**

As with prior examples, FIGs. 36-37 illustrate that in the relaxed state, the distal cells **248D** may be substantially the same size and attached to the distal junction **236** by a basket strip **291** having a proximal end attached to a distal crown of a distal cell **248D** and a distal end attached to the distal junction **236.**

As with prior examples, FIGs. 36-37 illustrate that in the relaxed state, the cells 250A-250J comprising the free distal crowns **258A-258J** may be substantially the same size.

As with prior examples, the system of FIGs. 36-37 may be used in a method of removing a blood clot from a blood vessel of an animal the method comprising the steps of: a) providing the system; b) positioning the system in the blood vessel; c) allowing the height **224** and width **225** of the distal body **216** to increase; d) moving the blood clot into the basket interior **346;** and e) moving the distal body **216** proximally out of the blood vessel.

As with prior examples, in FIGs. 36-37, not all parts are labelled in every drawing for clarity.

The exampleof FIG. 36 and the embodiment of FIG. 37 may include any feature described or shown with the prior examples. For example, as illustrated in FIGs. 36-37, the proximal junction **228** and distal junction **236** may be located approximately in the center of the distal body width **225** and distal body height **224** in the relaxed state. Additionally, as illustrated in FIGs. 36-37, each pair of enlarged cells **262A/262B; 262C/262D; 262E/262F; 262G/262H; 262I/262J** may be substantially aligned such that each pair of enlarged cells **262A/262B; 262C/262D; 262E/262F; 262G/262H; 262I/262J** and the substantially hollow interior **346** create a void extending from one side of the basket **246** through the substantially hollow interior **346** to the opposite side of the basket **246.** In addition, FIGs. 36-37 illustrate the previously mentioned and shown twisting proximal strips **252.** For example, the proximal end **254** of a first proximal strip **252** may be located at least about 65 degrees relative to the distal end **256** of the first proximal strip **252,** the proximal end **254** of a second proximal strip **252** may be located least about 65 degrees relative to the distal end **256** of the second proximal strip **252,** and the first and second proximal strips **252** may intersect adjacent and distal to the proximal junction **228** and the intersection may be located approximately in the center of the first height **224** and first width **225** in the relaxed state. In addition, FIG. 37 illustrate that the distal body **216** may have a void located between the proximal junction **228** and the cells **250A, 250B** comprising the proximal-most free distal crowns **258A, 258B.** In addition, FIGs. 36-37 illustrate that the basket **246** has a non-uniform outward radial force from the proximal strips **252** to the basket distal end **302.** As with prior examples, in the example of FIG. 36 and the embodiment of FIG. 37, the free distal crowns of each pair of free distal crowns **258A/258B; 258C/258D; 258E/258F; 258G/258H; 258I/258J** may be configured to contact each other when an exterior, external compressive force is exerted on the free distal crowns **258A-258J** when the distal body **216** is in the relaxed state. As with prior examples, in the exampleof FIG. 36 and the embodiment of FIG. 37, the proximal junction **228** and distal junction **236** may be in the form of tubes. In addition, the distal body **216** may include the three-dimensional openings **293** mentioned previously. As with the prior embodiments, in the example of FIG. 36 and the embodiment of FIG. 37, some or all of the free distal crowns **258A-258J,** as well as proximal and distal junctions **228** and **236,** may include x-ray markers, as previously described.

Having now described the invention, those skilled in the art will understand how to make changes and modifications to the disclosed embodiments to meet their specific requirements or conditions. Changes and modifications may be made without departing from the scope of the invention, as defined and limited solely by the following claims. In particular, although the system has been exemplified for use in retrieving blood clots, the system may be used to retrieve other objects from animal lumens. In addition, the steps of any method described herein may be performed in any suitable order and steps may be performed simultaneously if needed.

Terms of degree such as "substantially", "about" and "approximately" as used herein mean a reasonable amount of deviation of the modified term such that the end result is not significantly changed. For example, these terms can be construed as including a deviation of at least ± 5% of the modified term if this deviation would not negate the meaning of the word it modifies.

## Claims

1. A system for removing objects from an interior lumen of an animal, the system comprising:
a pull wire (202) having a proximal end and a distal end (206);
a distal body (216) attached to the pull wire, the distal body comprising an interior (222), a perimeter (300), a proximal end (218), a distal end (220), a distal body length (226) extending from the proximal end to the distal end, a proximal junction (228) forming the proximal end of the distal body, a plurality of proximal strips (252), a basket (246) comprised of a plurality of cells formed by a plurality of basket strips (291), and a distal junction (236) forming a distal end (302) of the basket, the basket comprising a basket interior (346), each proximal strip having a distal end (256) attached to a cell and a proximal end (254), the proximal ends of the proximal strips converging at the proximal junction, the distal body having a relaxed state wherein the distal body has a first height (224) and a first width (225), and a collapsed state wherein the distal body has a second height and a second width, the second height less than the first height, the second width less than the first width,
wherein, in the relaxed state, the basket comprises a series of at least three pair of cells (250A-250F) located on the distal body perimeter having a proximal crown (260) pointing generally in the proximal direction and attached to a memory metal strip and a free distal crown (258A-258F) pointing generally in the distal direction, wherein in the series, the proximal-most free distal crowns (258A, 258B) are located at the 12 and 6 o'clock positions and located about the same distance from the proximal junction (228), the next proximal - most free distal crowns (258C, 258D) are located at the 3 and 9 o'clock positions and located about the same distance from the proximal junction, and the succeeding proximal-most free distal crowns (258E, 258F) are located at the 12 and 6 o'clock positions and located about the same distance from the proximal junction, wherein each free distal crown forms part of a different enlarged cell (262A-262J) configured to allow a thrombus to enter the basket interior, wherein, in the relaxed state, the basket comprises a plurality of distal cells (248D) distal to the distal-most free distal crowns of the series, the plurality of distal cells having a proximal crown attached to another cell of the basket and pointing generally in the proximal direction and a distal crown pointing generally in the distal direction and attached to the distal junction (236),
wherein, in the relaxed state, each enlarged cell has a proximal end (308), a distal end (310) comprising a distal crown pointing generally in the distal direction, and a length (312) extending from the proximal end to the distal end of the respective enlarged cell, wherein, in the relaxed state, each distal cell has a length (314) extending from the proximal crown to the distal crown of the respective distal cell,
wherein, in the relaxed state, each of the enlarged cells is longer than each of the distal cells,
wherein, in the relaxed state, for each of the enlarged cells, the distance from the proximal end of the enlarged cell to the free distal crown of the enlarged cell is less than the distance from the free distal crown of the enlarged cell to the distal end of the enlarged cell, wherein, in the relaxed state, the basket does not have any free crowns that point generally in the proximal direction,
wherein the distal body, in the relaxed state, comprises a distal tapered region (322) in which the distal body height and width decrease as the basket approaches the distal junction,
wherein, in the relaxed state, from at least the proximal crowns of the cells comprising the next proximal-most free distal crowns to the proximal ends of the enlarged cells formed by the succeeding proximal-most free distal crowns, the basket has no cells other than the enlarged cells and the cells comprising the free distal crowns, and
further wherein, in the relaxed state, for each of the enlarged cells, two basket strips meet to form the distal crown located at the distal end of the enlarged cell, wherein each basket strip has a basket strip proximal end, a basket strip distal end, and a basket strip length extending from the proximal end to the distal end, and further wherein, for at least some of the enlarged cells, the maximum angle (328) between the two basket strips at the same location along the distal body length is at least 90 degrees.

2. The system of claim 1 wherein, in the relaxed state, each of the enlarged cells (262A-262J) is longer than each of the pair of cells (250A-250F).

3. The system of claim 1 wherein, in the relaxed state, each of the enlarged cells (262A-262J) extends from the 6 o'clock position to the 12 o'clock position or the 3 o'clock position to the 9 o'clock position.

4. The system of claim 1, wherein, in the relaxed state, the basket (246) further comprises a plurality of proximal cells (342) proximal to the proximal-most free distal crowns (258A, 258B) of the series, the plurality of proximal cells having a proximal crown attached to the proximal junction (228) and pointing generally in the proximal direction and a distal crown pointing generally in the distal direction and attached to another cell of the basket.

5. The system of claim 1 wherein the proximal crowns of the cells (250A, 250B) comprising the proximal- most free distal crowns (258A, 258B) are attached to the distal ends (256) of the proximal strips (252).

6. The system of claim 1 wherein, in the relaxed state, for said at least some of the enlarged cells (262A-262J), two basket strips (291) meet to form the free distal crowns (258A-258F) of the enlarged cell, wherein each basket strip has a basket strip proximal end, a basket strip distal end, and a basket strip length extending from the proximal end to the distal end, and further wherein the maximum angle (326) between the two basket strips at the same location along the distal body length is no more than 40 degrees.

7. The system of claim 1 wherein, in the relaxed state, for each of the enlarged cells (262A-262J), two basket strips (291) meet to form the distal crown located at the distal end (310) of the enlarged cell, wherein each basket strip has a basket strip proximal end, a basket strip distal end, and a basket strip length extending from the proximal end to the distal end, and further wherein, for at least some of the enlarged cells, the average angle between the two basket strips is at least 90 degrees.

8. The system of claim 1 wherein, in the relaxed state, for each of the enlarged cells (262A-262J), two basket strips (291) meet to form the distal crown located at the distal end (310) of the enlarged cell, the two basket strips having a distal end (332) meeting at the respective distal crown, a proximal end (330) attached to another cell of the basket, and a basket strip length extending from the proximal end to the distal end, and further wherein, for at least some of the enlarged cells, each of the basket strip lengths are approximately equal to ½ of the distal body height (224) and width (225).

9. The system of claim 1 wherein, in the relaxed state, from at least the proximal-most free distal crowns (258A, 258B) to the proximal ends of the enlarged cells (262E, 262F) formed by the succeeding proximal-most free distal crowns (258E, 258F), the basket has no cells other than the enlarged cells (262A-262F) and the cells (250A-250F) comprising the free distal crowns (258A-258F).

10. The system of claim 1 wherein the basket (246) comprises a series of at least five pairs of cells (250A-250F) located on the distal body perimeter (300) having a proximal crown (260) pointing generally in the proximal direction and attached to a memory metal strip and a free distal crown pointing generally in the distal direction, wherein in the series, the next proximal-most free distal crowns (258C,258D) after the succeeding proximal -most free distal crowns (258E, 258F) are located at the 3 and 9 o'clock positions and located about the same distance from the proximal junction, and the distal-most free distal crowns are located at the 12 and 6 o'clock positions and located about the same distance from the proximal junction.

11. The system of claim 1 wherein, in the relaxed state, for at least some of the enlarged cells (262A-262J), the maximum angle (328) between the two basket strips (291) at the same location along the distal body length is at least 100 degrees.

12. The system of claim 1 wherein, in the relaxed state, the distal crowns of the enlarged cells (262A-262J) are attached to another cell of the basket (246).

13. The system of claim 1, wherein in the relaxed state, the basket (246) further comprises an additional pair of cells (344) located about the same distance from the proximal junction (228) as the cells (250A, 250B) comprising the proximal -most pair of free distal crowns (258A, 258B) and located at the 9 and 3 o'clock positions, each cell of the additional pair of cells having a proximal crown attached to a memory metal strip and a distal crown attached to another cell of the basket, wherein the additional pair of cells adjoin the enlarged cells (262A, 262B) formed by the proximal -most free distal crowns (258A, 258B), and further wherein from at least the distal crowns of the additional pair of cells to the proximal ends of the enlarged cells (262E, 262F) formed by the succeeding free distal crowns (258E, 258F), the basket has no cells other than the enlarged cells (262A-262F), the cells (250A-250F) comprising the free distal crowns (258A-258F) and the additional pair of cells.

14. The system of claim 1 wherein, in the relaxed state, the basket (246) comprises a series of bridge memory metal strips (295) having a proximal end (334) attached to a distal crown of a cell and a distal end (336) attached to a proximal crown of a distally-located cell, wherein a proximal pair of bridge memory metal strips are located at the 3 and 9 o'clock positions and form part of two enlarged cells (262A-262J), wherein a distal pair of bridge memory metal strips distal to the proximal pair of bridge memory metal strips are located at the 12 and 6 o'clock positions and form part of two enlarged cells, and further wherein the bridge memory metal strips are the sole distally-extending basket strips (291) attached to the respective proximal crowns and the sole proximally-extending basket strips attached to the respective distal crowns.

15. The system of claim 14 wherein, in the relaxed state, the bridge memory metal strips (295) are substantially parallel to the distal body length.

## Patentansprüche

1. System zum Entfernen von Objekten aus einem inneren Lumen eines Tieres, wobei das System umfasst:
einen Zugdraht (202), der ein proximales Ende und ein distales Ende (206) aufweist;
einen an dem Zugdraht befestigten distalen Körper (216), wobei der distale Körper ein Inneres (222), einen Umfang (300), ein proximales Ende (218), ein distales Ende (220), eine distale Körperlänge (226), die sich vom proximalen Ende zum distalen Ende erstreckt, eine proximale Anschlussstelle (228), die das proximale Ende des distalen Körpers bildet, eine Vielzahl von proximalen Streifen (252), einen Korb (246), der aus einer Vielzahl von Zellen besteht, die aus einer Vielzahl von Korbstreifen (291) gebildet sind, und eine distale Anschlussstelle (236), die ein distales Ende (302) des Korbs bildet, umfasst,
wobei der Korb ein Korbinneres (346) umfasst, jeder proximale Streifen ein an einer Zelle befestigtes distales Ende (256) und ein proximales Ende (254) aufweist, die proximalen Enden der proximalen Streifen an der proximalen Anschlussstelle zusammenlaufen, der distale Körper einen entspannten Zustand aufweist, in dem der distale Körper eine erste Höhe (224) und eine erste Breite (225) aufweist, und einen eingeklappten Zustand, in dem der distale Körper eine zweite Höhe und eine zweite Breite aufweist, wobei die zweite Höhe geringer als die erste Höhe ist, wobei die zweite Breite geringer als die erste Breite ist,
wobei der Korb im entspannten Zustand eine Reihe von mindestens drei Zellpaaren (250A-250F) umfasst, die sich am distalen Körperumfang befinden, der eine proximale Krone (260) aufweist, die im Allgemeinen in die proximale Richtung zeigt und an einem Formgedächtnismetallstreifen befestigt ist, und eine freie distale Krone (258A-258F), die im Allgemeinen in die distale Richtung zeigt, wobei sich die proximalsten freien distalen Kronen (258A, 258B) in den Reihen in den 12 und 6 Uhr-Positionen befinden und sich etwa im selben Abstand von der proximalen Anschlussstelle (228) befinden, sich die nächsten proximalsten freien distalen Kronen (258C, 258D) in den 3 und 9 Uhr-Positionen befinden und sich etwa im selben Abstand von der proximalen Anschlussstelle befinden, und sich die nachfolgenden proximalsten freien distalen Kronen (258E, 258F) in den 12 und 6 Uhr-Positionen befinden und sich etwa im selben Abstand von der proximalen Anschlussstelle befinden, wobei jede freie distale Krone einen Teil einer andersartigen vergrößerten Zelle (262A-262J) bildet, die konfiguriert ist, um zu erlauben, dass ein Thrombus ins Korbinnere eintritt, wobei der Korb im entspannten Zustand eine Vielzahl von distalen Zellen (248D), distal zu den distalsten freien distalen Kronen der Reihe umfasst, wobei die Vielzahl von distalen Zellen eine proximale Krone, die an einer anderen Zelle des Korbes befestigt ist und im Allgemeinen in die proximale Richtung zeigt, und eine distale Krone, die im Allgemeinen in die distale Richtung zeigt und an der distalen Anschlussstelle (236) befestigt ist, aufweist,
wobei jede vergrößerte Zelle im entspannten Zustand ein proximales Ende (308), ein distales Ende (310), das eine distale Krone umfasst, die im Allgemeinen in die distale Richtung zeigt, und eine Länge (312), die sich vom proximalen Ende zum distalen Ende der jeweiligen vergrößerten Zelle erstreckt, aufweist, wobei jede distale Zelle im entspannten Zustand eine Länge (314) aufweist, die sich von der proximalen Krone zur distalen Krone der jeweiligen distalen Zelle erstreckt,
wobei jede von den vergrößerten Zellen im entspannten Zustand länger als jede von den distalen Zellen ist,
wobei im entspannten Zustand der Abstand für jede der vergrößerten Zellen vom proximalen Ende der vergrößerten Zelle zur freien distalen Krone der vergrößerten Zelle geringer ist als der Abstand von der freien distalen Krone der vergrößerten Zelle zum distalen Ende der vergrößerten Zelle, wobei der Korb im entspannten Zustand keine freien Kronen aufweist, die im Allgemeinen in die proximale Richtung zeigen,
wobei der distale Körper im entspannten Zustand einen distalen sich verjüngenden Bereich (322) umfasst, in dem die Höhe und Breite des distalen Körpers abnehmen, wenn sich der Korb der distalen Anschlussstelle nähert,
wobei der Korb im entspannten Zustand von mindestens den proximalen Kronen der Zellen, welche die nächsten proximalsten freien distalen Kronen umfassen, zu den proximalen Enden der vergrößerten Zellen, die von den nachfolgenden proximalsten freien distalen Kronen gebildet werden, keine anderen Zellen als die vergrößerten Zellen und die Zellen, die die freien distalen Kronen umfassen, aufweist, und
wobei sich im entspannten Zustand weiter zwei Korbstreifen für jede der vergrößerten Zellen treffen, um die distale Krone zu bilden, die sich am distalen Ende der vergrößerten Zelle befindet, wobei jeder Korbstreifen ein proximales Ende eines Korbstreifens, ein distales Ende eines Korbstreifens und eine Korbstreifenlänge, die sich vom proximalen Ende zum distalen Ende erstreckt, aufweist, und wobei weiter für mindestens einige der vergrößerten Zellen der maximale Winkel (328) zwischen den beiden Korbstreifen an derselben Stelle entlang der distalen Körperlänge mindestens 90 Grad beträgt.

2. System nach Anspruch 1, wobei jede von den vergrößerten Zellen (262A-262J) im entspannten Zustand länger als jede des Zellenpaars (250A-250F) ist.

3. System nach Anspruch 1, wobei sich jede von den vergrößerten Zellen (262A-262J) im entspannten Zustand von der 6 Uhr-Position in die 12 Uhr-Position oder der 3 Uhr-Position in die 9 Uhr-Position erstreckt.

4. System nach Anspruch 1, wobei der Korb (246) im entspannten Zustand weiter eine Vielzahl von proximalen Zellen (342), proximal zu den proximalsten freien distalen Kronen (258A, 258B) der Reihe umfasst, wobei die Vielzahl von proximalen Zellen eine proximale Krone, die an der proximalen Anschlussstelle (228) befestigt ist und im Allgemeinen in die proximale Richtung zeigt, und eine distale Krone, die im Allgemeinen in die distale Richtung zeigt und an einer anderen Zelle des Korbes befestigt ist, aufweist.

5. System nach Anspruch 1, wobei die proximalen Kronen der Zellen (250A, 250B), welche die proximalsten freien distalen Kronen (258A, 258B) umfassen, an den distalen Enden (256) der proximalen Streifen (252) befestigt sind.

6. System nach Anspruch 1, wobei sich im entspannten Zustand zwei Korbstreifen (291) für die mindestens einigen von den vergrößerten Zellen (262A-262J) treffen, um freie distale Kronen (258A-258F) der vergrößerten Zelle zu bilden, wobei jeder Korbstreifen ein proximales Ende eines Korbstreifens, ein distales Ende eines Korbstreifens und eine Korbstreifenlänge, die sich vom proximalen Ende zum distalen Ende erstreckt, aufweist, und wobei weiter der maximale Winkel (326) zwischen den beiden Korbstreifen an derselben Stelle entlang der distalen Körperlänge nicht mehr als 40 Grad beträgt.

7. System nach Anspruch 1, wobei sich im entspannten Zustand zwei Korbstreifen (291) für jede der vergrößerten Zellen (262A-262J) treffen, um die distale Krone zu bilden, die sich am distalen Ende (310) der vergrößerten Zelle befindet, wobei jeder Korbstreifen ein proximales Ende eines Korbstreifens, ein distales Ende eines Korbstreifens, und eine Korbstreifenlänge, die sich vom proximalen Ende zum distalen Ende erstreckt, aufweist, und wobei weiter für mindestens einige der vergrößerten Zellen der mittlere Winkel zwischen den beiden Korbstreifen mindestens 90 Grad beträgt.

8. System nach Anspruch 1, wobei sich im entspannten Zustand zwei Korbstreifen (291) für jede der vergrößerten Zellen (262A-262J) treffen, um die distale Krone zu bilden, die sich am distalen Ende (310) der vergrößerten Zelle befindet, wobei die beiden Korbstreifen ein distales Ende (332), die sich an der jeweiligen distalen Krone treffen, ein proximales Ende (330), das an einer anderen Zelle des Korbes befestigt ist, und eine Korbstreifenlänge, die sich vom proximalen Ende zum distalen Ende erstreckt, aufweisen, und wobei weiter für mindestens einige der vergrößerten Zellen jede von den Korbstreifenlängen annähernd gleich 14 von der Höhe (224) und Breite (225) des distalen Körpers ist.

9. System nach Anspruch 1, wobei der Korb im entspannten Zustand von mindestens den proximalsten freien distalen Kronen (258A, 258B) zu den proximalen Enden der vergrößerten Zellen (262E, 262F), die von den nachfolgenden proximalsten freien distalen Kronen (258E, 258F) gebildet werden, keine anderen Zellen als die vergrößerten Zellen (262A-262F), und die Zellen (250A-250F), die die freien distalen Kronen (258A-258F) umfassen, aufweist.

10. System nach Anspruch 1, wobei der Korb (246) eine Reihe von mindestens fünf Zellpaaren (250A-250F) umfasst, die sich am distalen Körperumfang (300) befinden, der eine proximale Krone (260), die im Allgemeinen in die proximale Richtung zeigt, und an einem Formgedächtnismetallstreifen befestigt ist, und eine freie distale Krone, die im Allgemeinen in die distale Richtung zeigt, aufweist, wobei sich in der Reihe die nächsten proximalsten freien distalen Kronen (258C, 258D) nach den nachfolgenden proximalsten freien distalen Kronen (258E, 258F) in den 3 und 9 Uhr-Positionen befinden und sich etwa im selben Abstand von der proximalen Anschlussstelle befinden, und sich die distalsten freien distalen Kronen in den 12 und 6 Uhr-Positionen befinden und sich etwa im selben Abstand von der proximalen Anschlussstelle befinden.

11. System nach Anspruch 1, wobei im entspannten Zustand für mindestens einige von den vergrößerten Zellen (262A-262J) der maximale Winkel (328) zwischen den beiden Korbstreifen (291) an derselben Stelle entlang der distalen Körperlänge mindestens 100 Grad beträgt.

12. System nach Anspruch 1, wobei im entspannten Zustand die distalen Kronen der vergrößerten Zellen (262A-262J) an einer anderen Zelle des Korbes (246) befestigt sind.

13. System nach Anspruch 1, wobei der Korb (246) im entspannten Zustand weiter ein zusätzliches Zellpaar (344) umfasst, das sich etwa im selben Abstand von der proximalen Anschlussstelle (228) wie die Zellen (250A, 250B) befindet, die das proximalste Paar von freien distalen Kronen (258A, 258B) umfassen und sich in den 9 und 3 Uhr-Positionen befinden, wobei jede Zelle des zusätzlichen Zellenpaares eine proximale Krone, die an einem Formgedächtnismetallstreifen befestigt ist, und eine distale Krone, die an einer anderen Zelle des Korbes befestigt ist, aufweist, wobei das zusätzliche Zellpaar an den vergrößerten Zellen (262A, 262B), die durch die proximalsten freien distalen Kronen (258A, 258B) gebildet werden, anliegt, und wobei der Korb weiter von mindestens den distalen Kronen des zusätzlichen Zellpaares zu den proximalen Enden der vergrößerten Zellen (262E, 262F), die durch die nachfolgenden freien distalen Kronen (258E, 258F) gebildet werden, keine anderen Zellen als die vergrößerten Zellen (262A-262F), die Zellen (250A-250F), welche die freien distalen Kronen (258A-258F) umfassen, und das zusätzliche Zellpaar aufweist.

14. System nach Anspruch 1, wobei der Korb (246) im entspannten Zustand eine Reihe von Brücken-Formgedächtnismetallstreifen (295) umfasst, die ein proximales Ende (334), das an einer distalen Krone einer Zelle befestigt ist, und ein distales Ende (336), das an einer proximalen Krone einer distal befindlichen Zelle befestigt ist, aufweisen, wobei sich ein proximales Paar von Brücken-Formgedächtnismetallstreifen in den 3 und 9 Uhr-Positionen befindet und einen Teil von zwei vergrößerten Zellen (262A-262J) bildet, wobei sich ein distales Paar von Brücken-Formgedächtnismetallstreifen distal zum proximalen Paar von Brücken-Formgedächtnismetallstreifen in den 12 und 6 Uhr-Positionen befindet und einen Teil von zwei vergrößerten Zellen bildet, und wobei die Brücken-Formgedächtnismetallstreifen weiter die einzigen sich distal erstreckenden Korbstreifen (291), die an den jeweiligen proximalen Kronen befestigt sind, und die einzigen sich proximal erstreckenden Korbstreifen, die an den jeweiligen distalen Kronen befestigt sind, sind.

15. System nach Anspruch 14, wobei die Brücken-Formgedächtnismetallstreifen (295) im entspannten Zustand im Wesentlichen parallel zur distalen Körperlänge verlaufen.

## Revendications

1. Système pour retirer des objets d'une lumière intérieure d'un animal, le système comprenant :
un fil de traction (202) présentant une extrémité proximale et une extrémité distale (206) ;
un corps distal (216) attaché au fil de traction, le corps distal comprenant un intérieur (222), un périmètre (300), une extrémité proximale (218), une extrémité distale (220), une longueur de corps distal (226) s'étendant depuis l'extrémité proximale jusqu'à l'extrémité distale, une jonction proximale (228) formant l'extrémité proximale du corps distal, une pluralité de bandes proximales (252), un panier (246) composé d'une pluralité de cellules formées par une pluralité de bandes de panier (291), et une jonction distale unique (236) formant une extrémité distale (302) du panier, le panier comprenant un intérieur de panier (346), chaque bande proximale présentant une extrémité distale (256) attachée à une cellule et une extrémité proximale (254), les extrémités proximales des bandes proximales convergeant au niveau de la jonction proximale, le corps distal présentant un état relâché dans lequel le corps distal présente une première hauteur (224) et une première largeur (225), et un état affaissé dans lequel le corps distal présente une seconde hauteur et une seconde largeur, la seconde hauteur étant inférieure à la première hauteur, la seconde largeur étant inférieure à la première largeur,
dans lequel, dans l'état relâché, le panier comprend une série d'au moins trois paires de cellules (250A-250F) situées sur le périmètre de corps distal présentant une couronne proximale (260) pointant généralement dans la direction proximale et attachée à une bande de métal à mémoire et une couronne distale libre (258A-258F) pointant généralement dans la direction distale, dans lequel, dans la série, les couronnes distales libres les plus proximales (258A, 258B) sont situées aux positions à 12 heures et à 6 heures et situées à peu près à la même distance de la jonction proximale (228), les couronnes distales libres les plus proximales suivantes (258C, 258D) sont situées aux positions à 3 heures et à 9 heures et situées à peu près à la même distance de la jonction proximale, et les couronnes distales libres les plus proximales suivantes (258E, 258F) sont situées aux positions à 12 heures et à 6 heures et situées à peu près à la même distance de la jonction proximale, dans lequel chaque couronne distale libre fait partie d'une cellule agrandie différente (262A-262J) configurée pour permettre à un caillot d'entrer à l'intérieur du panier, dans lequel, dans l'état relâché, le panier comprend une pluralité de cellules distales (248D) distales des couronnes distales libres les plus distales de la série, la pluralité de cellules distales présentant une couronne proximale attachée à une autre cellule du panier et pointant généralement dans la direction proximale et une couronne distale pointant généralement dans la direction distale et attachée à la jonction distale (236),
dans lequel, dans l'état relâché, chaque cellule agrandie présente une extrémité proximale (308), une extrémité distale (310) comprenant une couronne distale pointant généralement dans la direction distale, et une longueur (312) s'étendant depuis l'extrémité proximale jusqu'à l'extrémité distale de la cellule agrandie respective, dans lequel, dans l'état relâché, chaque cellule distale présente une longueur (314) s'étendant depuis la couronne proximale jusqu'à la couronne distale de la cellule distale respective,
dans lequel, dans l'état relâché, chacune des cellules agrandies est plus longue que chacune des cellules distales,
dans lequel, dans l'état relâché, pour chacune des cellules agrandies, la distance depuis l'extrémité proximale de la cellule agrandie jusqu'à la couronne distale libre de la cellule agrandie est inférieure à la distance depuis la couronne distale libre de la cellule agrandie jusqu'à l'extrémité distale de la cellule agrandie, dans lequel, dans l'état relâché, le panier ne présente pas de couronnes libres qui pointent généralement dans la direction proximale,
dans lequel le corps distal, dans l'état relâché, comprend une région effilée distale (322) dans laquelle la hauteur et la largeur du corps distal diminuent au fur et à mesure que le panier s'approche de la jonction distale,
dans lequel, dans l'état relâché, depuis au moins les couronnes proximales des cellules comprenant les couronnes distales libres les plus proximales suivantes jusqu'aux extrémités proximales des cellules agrandies formées par les couronnes distales libres les plus proximales suivantes, le panier ne présente pas de cellules autres que les cellules agrandies et les cellules comprenant les couronnes distales libres, et
en outre dans lequel, dans l'état relâché, pour chacune des cellules agrandies, deux bandes de panier se rejoignent pour former la couronne distale située au niveau de l'extrémité distale de la cellule agrandie, dans lequel chaque bande de panier présente une extrémité proximale de bande de panier, une extrémité distale de bande de panier et une longueur de bande de panier s'étendant depuis l'extrémité proximale jusqu'à l'extrémité distale et, en outre, dans lequel, pour au moins certaines des cellules agrandies, l'angle maximal (328) entre les deux bandes de panier au même emplacement le long de la longueur de corps distal est au moins 90 degrés.

2. Système selon la revendication 1, dans lequel, dans l'état relâché, chacune des cellules agrandies (262A-262J) est plus longue que chacune des paires de cellules (250A-250F).

3. Système selon la revendication 1, dans lequel, dans l'état relâché, chacune des cellules agrandies (262A-262J) s'étend depuis la position à 6 heures jusqu'à la position à 12 heures ou depuis la position à 3 heures jusqu'à la position à 9 heures.

4. Système selon la revendication 1, dans lequel, dans l'état relâché, le panier (246) comprend en outre une pluralité de cellules proximales (342) proximales des couronnes distales libres les plus proximales (258A, 258B) de la série, la pluralité de cellules proximales présentant une couronne proximale attachée à la jonction proximale (228) et pointant généralement dans la direction proximale et une couronne distale pointant généralement dans la direction distale et attachée à une autre cellule du panier.

5. Système selon la revendication 1, dans lequel les couronnes proximales des cellules (250A, 250B) comprenant les couronnes distales libres les plus proximales (258A, 258B) sont attachées aux extrémités distales (256) des bandes proximales (252).

6. Système selon la revendication 1, dans lequel, dans l'état relâché, pour lesdites au moins certaines des cellules agrandies (262A-262J), deux bandes de panier (291) se rejoignent pour former les couronnes distales libres (258A-258F) de la cellule agrandie, dans lequel chaque bande de panier présente une extrémité proximale de bande de panier, une extrémité distale de bande de panier et une longueur de bande de panier s'étendant depuis l'extrémité proximale jusqu'à l'extrémité distale et, en outre, dans lequel l'angle maximal (326) entre les deux bandes de panier au même emplacement le long de la longueur de corps distal est inférieur ou égal à 40 degrés.

7. Système selon la revendication 1, dans lequel, dans l'état relâché, pour chacune des cellules agrandies (262A-262J), deux bandes de panier (291) se rejoignent pour former la couronne distale située au niveau de l'extrémité distale (310) de la cellule agrandie, dans lequel chaque bande de panier présente une extrémité proximale de bande de panier, une extrémité distale de bande de panier,et une longueur de bande de panier s'étendant depuis l'extrémité proximale jusqu'à l'extrémité distale et, en outre, dans lequel, pour au moins certaines des cellules agrandies, l'angle maximal entre les deux bandes de panier est au moins 90 degrés.

8. Système selon la revendication 1, dans lequel, dans l'état relâché, pour chacune des cellules agrandies (262A-262J), deux bandes de panier (291) se rejoignent pour former la couronne distale située à l'extrémité distale (310) de la cellule agrandie, les deux bandes de panier présentant une extrémité distale (332) rejoignant la couronne distale respective, une extrémité proximale (330) attachée à une autre cellule du panier et une longueur de bande de panier s'étendant depuis l'extrémité proximale jusqu'à l'extrémité distale et, en outre, dans lequel, pour au moins certaines des cellules agrandies, chacune des longueurs de bandes de panier sont approximativement égales à 14 de la hauteur de corps distal (224) et de la largeur de corps distal (225).

9. Système selon la revendication 1, dans lequel, dans l'état relâché, depuis au moins les couronnes distales libres les plus proximales (258A, 258B) jusqu'aux extrémités proximales des cellules agrandies (262E, 262F) formées par les couronnes distales libres les plus proximales suivantes (258E, 258F), le panier ne présente pas de cellules autres que les cellules agrandies (262A-262F) et les cellules (250A-250F) comprenant les couronnes distales libres (258A-258F).

10. Système selon la revendication 1, dans lequel le panier (246) comprend une série d'au moins cinq paires de cellules (250A-250F) situées sur le périmètre de corps distal (300) présentant une couronne proximale (260) pointant généralement dans la direction proximale et attachée à une bande de métal à mémoire et une couronne distale libre pointant généralement dans la direction distale, dans lequel, dans la série, les couronnes distales libres les plus proximales suivantes (258C, 258D) après les couronnes distales libres les plus proximales suivantes (258E, 258F) sont situées aux positions à 3 heures et à 9 heures et situées à peu près à la même distance de la jonction proximale, et les couronnes distales libres les plus distales sont situées aux positions à 12 heures et à 6 heures et situées à peu près à la même distance de la jonction proximale.

11. Système selon la revendication 1, dans lequel, dans l'état relâché, pour au moins certaines des cellules agrandies (262A-262J), l'angle maximal (328) entre les deux bandes de panier (291) au même emplacement le long de la longueur de corps distal est au moins 100 degrés.

12. Système selon la revendication 1, dans lequel, dans l'état relâché, les couronnes distales des cellules agrandies (262A-262J) sont attachées à une autre cellule du panier (246).

13. Système selon la revendication 1, dans lequel, dans l'état relâché, le panier (246) comprend en outre une paire supplémentaire de cellules (344) situées à peu près à la même distance de la jonction proximale (228) que les cellules (250A, 250B) comprenant la paire la plus proximale des couronnes distales libres (258A, 258B) et situées aux positions à 9 heures et à 3 heures, chaque cellule de la paire supplémentaire de cellules présentant une couronne proximale attachée à une bande de métal à mémoire et une couronne distale attachée à une autre cellule du panier, dans lequel la paire supplémentaire de cellules est adjacente aux cellules agrandies (262A, 262B) formées par les couronnes distales libres les plus proximales (258A, 258B) et, en outre, dans lequel, à partir d'au moins les couronnes distales de la paire supplémentaire de cellules jusqu'aux extrémités proximales des cellules agrandies (262E, 262F) formées par les couronnes distales libres suivantes (258E, 258F), le panier ne présente pas de cellules autres que les cellules agrandies (262A-262F), les cellules (250A-250F) comprenant les couronnes distales libres (258A-258F) et la paire supplémentaire de cellules.

14. Système selon la revendication 1, dans lequel, dans l'état relâché, le panier (246) comprend une série de bandes de métal à mémoire en pont (295) présentant une extrémité proximale (334) attachée à une couronne distale d'une cellule et une extrémité distale (336) attachée à une couronne proximale d'une cellule située de manière distale, dans lequel une paire proximale de bandes de métal à mémoire en pont sont situées aux positions à 3 heures et à 9 heures et font partie de deux cellules agrandies (262A-262J), dans lequel une paire distale de bandes de métal à mémoire en pont distales de la paire proximale de bandes de métal à mémoire en pont sont situées aux positions à 12 heures et à 6 heures et font partie de deux cellules agrandies et, en outre, dans lequel les bandes de métal à mémoire en pont sont les seules bandes de panier s'étendant de manière distale (291) attachées aux couronnes proximales respectives et les seules bandes de panier s'étendant de manière proximale attachées aux couronnes distales respectives.

15. Système selon la revendication 14, dans lequel, dans l'état relâché, les bandes de métal à mémoire en pont (295) sont sensiblement parallèles à la longueur de corps distal.
